(19) Europäisches Patentamt
European Patent Office
Office européen des brevets

(11) EP 3 936 526 A1

(12) **EUROPEAN PATENT APPLICATION**
published in accordance with Art. 153(4) EPC

(43) Date of publication:
**12.01.2022 Bulletin 2022/02**

(21) Application number: **20766028.3**

(22) Date of filing: **05.03.2020**

(51) Int Cl.:
***C07K 19/00*** *(2006.01)* ***C12N 15/62*** *(2006.01)*
***A61K 39/395*** *(2006.01)* ***A61P 35/00*** *(2006.01)*

(86) International application number:
**PCT/CN2020/077907**

(87) International publication number:
**WO 2020/177733 (10.09.2020 Gazette 2020/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.03.2019 CN 201910168433**
**24.05.2019 CN 201910437477**

(71) Applicants:
- **Jiangsu Hengrui Medicine Co., Ltd.
Lianyungang, Jiangsu 222047 (CN)**
- **Shanghai Hengrui Pharmaceutical Co., Ltd.
Shanghai 200245 (CN)**

(72) Inventors:
- **GU, Xiaoling
Shanghai 200245 (CN)**
- **YE, Xin
Shanghai 200245 (CN)**
- **HU, Bing
Shanghai 200245 (CN)**
- **GE, Hu
Shanghai 200245 (CN)**
- **TAO, Weikang
Shanghai 200245 (CN)**

(74) Representative: **Regimbeau
20, rue de Chazelles
75847 Paris Cedex 17 (FR)**

(54) **BIFUNCTIONAL FUSION PROTEIN AND PHARMACEUTICAL USE THEREOF**

(57) Provided are a bifunctional fusion protein and pharmaceutical use thereof. Specifically, provided are a bifunctional fusion protein comprising an SIRPγ peptide variant and an anti-human PD-L1 antibody, an SIRPγ peptide variant, and pharmaceutical use thereof. The bifunctional fusion protein can specifically bind PD-L1 and CD47 to block the binding of PD-L1 or CD47 to a receptor or ligand thereof. In addition, also provided are preparation and application of the bifunctional fusion protein, and treatment of cancers and immune-related diseases.

EP 3 936 526 A1

<110>

<120>

<130> 702015CPCT

<160> 133

<170> SIPOSequenceListing 1.0

<210> 1
<211> 119
<212> PRT
<213> (Artificial Sequence)

<220>
<221> PEPTIDE
<223> SIRP(I)

<220>
<221> PEPTIDE
<222> (31)..(31)
<223> XaaLeuTrp

<220>
<221> PEPTIDE
<222> (51)..(51)
<223> XaaMetValPheIleLeu

<220>
<221> PEPTIDE
<222> (52)..(52)
<223> XaaGlnSerThr

<220>
<221> PEPTIDE
<222> (54)..(54)
<223> XaaGluThrArg

<220>
<221> PEPTIDE
<222> (56)..(56)
<223> XaaHisArg

<220>
<221> PEPTIDE
<222> (70)..(70)
<223> XaaAspGlnGlu

<220>
<221> PEPTIDE
<222> (72)..(72)
<223> XaaIleValMetArgLys

<220>
<221> PEPTIDE
<222> (112)..(112)
<223> XaaMetVal

<400> 1
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Xaa Pro
20 25 30
Val Gly Pro Val Leu Trp Phe Arg Gly Val Gly Pro Gly Arg Glu Leu
35 40 45
Ile Tyr Xaa Xaa Gly Xaa Gly Xaa Phe Pro Arg Val Thr Thr Val Ser
50 55 60
Asp Leu Thr Lys Arg Xaa Asn Xaa Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Xaa
100 105 110
Ala Leu Gly Ala Lys Pro Ser
115

<210> 2
<211> 119
<212> PRT
<213> (Artificial Sequence)

<220>
<221> PEPTIDE
<223> SIRP(II)

<220>
<221> PEPTIDE
<222> (31)..(31)
<223> XaaLeuTrp

<220>
<221> PEPTIDE
<222> (52)..(52)
<223> XaaGlnSerThr

<220>
<221> PEPTIDE
<222> (54)..(54)
<223> XaaGluThrArg

<220>
<221> PEPTIDE
<222> (56)..(56)
<223> XaaHisArg

<220>
<221> PEPTIDE

<222> (70)..(70)
<223> XaaAspGlnGlu

<220>
<221> PEPTIDE
<222> (72)..(72)
<223> XaaIleValMetArgLys

<220>
<221> PEPTIDE
<222> (112)..(112)
<223> XaaMetVal

<400> 2
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Xaa Pro
20 25 30
Val Gly Pro Val Leu Trp Phe Arg Gly Val Gly Pro Gly Arg Glu Leu
35 40 45
Ile Tyr Arg Xaa Gly Xaa Gly Xaa Phe Pro Arg Val Thr Thr Val Ser
50 55 60
Asp Leu Thr Lys Arg Xaa Asn Xaa Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Xaa
100 105 110
Ala Leu Gly Ala Lys Pro Ser
115

<210> 3
<211> 129
<212> PRT
<213> (Artificial Sequence)

<220>
<221> PEPTIDE
<223> CD47-ECD-His

<400> 3
Gln Leu Leu Phe Asn Lys Thr Lys Ser Val Glu Phe Thr Phe Cys Asn
1 5 10 15
Asp Thr Val Val Ile Pro Cys Phe Val Thr Asn Met Glu Ala Gln Asn
20 25 30
Thr Thr Glu Val Tyr Val Lys Trp Lys Phe Lys Gly Arg Asp Ile Tyr
35 40 45
Thr Phe Asp Gly Ala Leu Asn Lys Ser Thr Val Pro Thr Asp Phe Ser
50 55 60
Ser Ala Lys Ile Glu Val Ser Gln Leu Leu Lys Gly Asp Ala Ser Leu
65 70 75 80
Lys Met Asp Lys Ser Asp Ala Val Ser His Thr Gly Asn Tyr Thr Cys
85 90 95
Glu Val Thr Glu Leu Thr Arg Glu Gly Glu Thr Ile Ile Glu Leu Lys
100 105 110
Tyr Arg Val Val Ser Trp Phe Ser Pro Asn Glu His His His His His
115 120 125
His

<210> 4
<211> 355
<212> PRT
<213> (Artificial Sequence)

<220>
<221> PEPTIDE
<223> CD47-ECD-Fc

<400> 4
Gln Leu Leu Phe Asn Lys Thr Lys Ser Val Glu Phe Thr Phe Cys Asn
1 5 10 15
Asp Thr Val Val Ile Pro Cys Phe Val Thr Asn Met Glu Ala Gln Asn
20 25 30
Thr Thr Glu Val Tyr Val Lys Trp Lys Phe Lys Gly Arg Asp Ile Tyr
35 40 45
Thr Phe Asp Gly Ala Leu Asn Lys Ser Thr Val Pro Thr Asp Phe Ser
50 55 60
Ser Ala Lys Ile Glu Val Ser Gln Leu Leu Lys Gly Asp Ala Ser Leu
65 70 75 80
Lys Met Asp Lys Ser Asp Ala Val Ser His Thr Gly Asn Tyr Thr Cys
85 90 95
Glu Val Thr Glu Leu Thr Arg Glu Gly Glu Thr Ile Ile Glu Leu Lys
100 105 110
Tyr Arg Val Val Ser Trp Phe Ser Pro Asn Glu Glu Pro Lys Ser Ser
115 120 125
Asp Lys Thr His Thr Cys Pro Pro Cys Pro Ala Pro Glu Leu Leu Gly
130 135 140
Gly Pro Ser Val Phe Leu Phe Pro Pro Lys Pro Lys Asp Thr Leu Met
145 150 155 160
Ile Ser Arg Thr Pro Glu Val Thr Cys Val Val Val Asp Val Ser His
165 170 175
Glu Asp Pro Glu Val Lys Phe Asn Trp Tyr Val Asp Gly Val Glu Val
180 185 190
His Asn Ala Lys Thr Lys Pro Arg Glu Glu Gln Tyr Asn Ser Thr Tyr
195 200 205
Arg Val Val Ser Val Leu Thr Val Leu His Gln Asp Trp Leu Asn Gly
210 215 220
Lys Glu Tyr Lys Cys Lys Val Ser Asn Lys Ala Leu Pro Ala Pro Ile
225 230 235 240
Glu Lys Thr Ile Ser Lys Ala Lys Gly Gln Pro Arg Glu Pro Gln Val
245 250 255
Tyr Thr Leu Pro Pro Ser Arg Asp Glu Leu Thr Lys Asn Gln Val Ser
260 265 270
Leu Thr Cys Leu Val Lys Gly Phe Tyr Pro Ser Asp Ile Ala Val Glu
275 280 285
Trp Glu Ser Asn Gly Gln Pro Glu Asn Asn Tyr Lys Thr Thr Pro Pro
290 295 300
Val Leu Asp Ser Asp Gly Ser Phe Phe Leu Tyr Ser Lys Leu Thr Val
305 310 315 320
Asp Lys Ser Arg Trp Gln Gln Gly Asn Val Phe Ser Cys Ser Val Met
325 330 335
His Glu Ala Leu His Asn His Tyr Thr Gln Lys Ser Leu Ser Leu Ser
340 345 350
Pro Gly Lys
355

<210> 5
<211> 350
<212> PRT
<213> (Artificial Sequence)

<220>
<221> PEPTIDE
<223> SIRP-Fc

<400> 5
Glu Glu Glu Leu Gln Ile Ile Gln Pro Asp Lys Ser Val Ser Val Ala
1 5 10 15
Ala Gly Glu Ser Ala Ile Leu His Cys Thr Ile Thr Ser Leu Phe Pro
20 25 30
Val Gly Pro Ile Gln Trp Phe Arg Gly Ala Gly Pro Ala Arg Val Leu
35 40 45
Ile Tyr Asn Gln Arg Gln Gly Pro Phe Pro Arg Val Thr Thr Val Ser
50 55 60
Glu Thr Thr Lys Arg Glu Asn Met Asp Phe Ser Ile Ser Ile Ser Asn
65 70 75 80
Ile Thr Pro Ala Asp Ala Gly Thr Tyr Tyr Cys Ile Lys Phe Arg Lys
85 90 95
Gly Ser Pro Asp Thr Glu Phe Lys Ser Gly Ala Gly Thr Glu Leu Ser
100 105 110
Val Arg Ala Lys Pro Ser Glu Pro Lys Ser Ser Asp Lys Thr His Thr
115 120 125
Cys Pro Pro Cys Pro Ala Pro Glu Leu Leu Gly Gly Pro Ser Val Phe
130 135 140
Leu Phe Pro Pro Lys Pro Lys Asp Thr Leu Met Ile Ser Arg Thr Pro
145 150 155 160
Glu Val Thr Cys Val Val Val Asp Val Ser His Glu Asp Pro Glu Val
165 170 175
Lys Phe Asn Trp Tyr Val Asp Gly Val Glu Val His Asn Ala Lys Thr
180 185 190
Lys Pro Arg Glu Glu Gln Tyr Asn Ser Thr Tyr Arg Val Val Ser Val
195 200 205
Leu Thr Val Leu His Gln Asp Trp Leu Asn Gly Lys Glu Tyr Lys Cys
210 215 220
Lys Val Ser Asn Lys Ala Leu Pro Ala Pro Ile Glu Lys Thr Ile Ser
225 230 235 240
Lys Ala Lys Gly Gln Pro Arg Glu Pro Gln Val Tyr Thr Leu Pro Pro
245 250 255
Ser Arg Asp Glu Leu Thr Lys Asn Gln Val Ser Leu Thr Cys Leu Val
260 265 270
Lys Gly Phe Tyr Pro Ser Asp Ile Ala Val Glu Trp Glu Ser Asn Gly
275 280 285
Gln Pro Glu Asn Asn Tyr Lys Thr Thr Pro Pro Val Leu Asp Ser Asp
290 295 300
Gly Ser Phe Phe Leu Tyr Ser Lys Leu Thr Val Asp Lys Ser Arg Trp
305 310 315 320
Gln Gln Gly Asn Val Phe Ser Cys Ser Val Met His Glu Ala Leu His
325 330 335
Asn His Tyr Thr Gln Lys Ser Leu Ser Leu Ser Pro Gly Lys
340 345 350

<210> 6
<211> 120
<212> PRT
<213> (Artificial Sequence)

<220>
<221> DOMAIN
<223> 9-2 H5

<400> 6

Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser
        115                 120

<210> 7
<211> 113
<212> PRT
<213> (Artificial Sequence)

<220>
<221> DOMAIN
<223> 9-2 L11

<400> 7
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Phe Tyr His
            20                  25                  30
Ser Asn Gln Lys His Ser Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Gly Tyr Pro Tyr Thr Phe Gly Gly Gly Thr Lys Val Glu Ile
            100                 105                 110
Lys

<210> 8
<211> 119
<212> PRT
<213> (Artificial Sequence)

<220>
<221> DOMAIN
<223> 24D5 H12

<400> 8
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe

```
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser
        115

<210>  9
<211>  111
<212>  PRT
<213>  (Artificial Sequence)

<220>
<221>  DOMAIN
<223>  24D5 L61


<400>  9
Asp Ile Val Leu Thr Gln Ser Pro Ala Ser Leu Ala Val Ser Pro Gly
1               5                   10                  15
Gln Arg Ala Thr Ile Thr Cys Arg Ala Ser Glu Ser Val Ser Ile His
            20                  25                  30
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Glu Asp Thr Ala Asn Tyr Tyr Cys Gln Gln Ser Phe
                85                  90                  95
Glu Asp Pro Leu Thr Phe Gly Gln Gly Thr Lys Leu Glu Ile Lys
            100                 105                 110

<210>  10
<211>  327
<212>  PRT
<213>  (Artificial Sequence)

<220>
<221>  DOMAIN
<223>  IgG4-S228P


<400>  10
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
```

```
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210>  11
<211>  330
<212>  PRT
<213>  (Artificial Sequence)

<220>
<221>  DOMAIN
<223>  IgG1


<400>  11
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
```

```
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330
```

<210> 12
<211> 107
<212> PRT
<213> (Artificial Sequence)

<220>
<221> DOMAIN
<223> Kappa

<400> 12

```
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```

<210> 13
<211> 446
<212> PRT
<213> (Artificial Sequence)

<220>
<221> PEPTIDE
<223> h1830

<400> 13

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
```

Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly
435 440 445

<210> 14
<211> 220
<212> PRT
<213> (Artificial Sequence)

<220>
<221> PEPTIDE
<223> h1830

<400> 14
Asp Ile Val Met Thr Gln Ser Pro Asp Ser Leu Ala Val Ser Leu Gly
1 5 10 15
Glu Arg Ala Thr Ile Asn Cys Lys Ser Ser Gln Ser Leu Phe Tyr His

20 25 30
Ser Asn Gln Lys His Ser Leu Ala Trp Tyr Gln Gln Lys Pro Gly Gln
35 40 45
Pro Pro Lys Leu Leu Ile Tyr Gly Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Gln
85 90 95
Tyr Tyr Gly Tyr Pro Tyr Thr Phe Gly Gly Gly Thr Lys Val Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 15
<211> 450
<212> PRT
<213> (Artificial Sequence)

<220>
<221> PEPTIDE
<223> h1830G1

<400> 15
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210> 16
<211> 445
<212> PRT
<213> (Artificial Sequence)

<220>
<221> PEPTIDE
<223> h1831

<400> 16
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp

```
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly
        435                 440                 445
```

<210> 17
<211> 218
<212> PRT
<213> (Artificial Sequence)

<220>
<221> PEPTIDE
<223> h1831

<400> 17

```
Asp Ile Val Leu Thr Gln Ser Pro Ala Ser Leu Ala Val Ser Pro Gly
1               5                   10                  15
Gln Arg Ala Thr Ile Thr Cys Arg Ala Ser Glu Ser Val Ser Ile His
            20                  25                  30
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Glu Asp Thr Ala Asn Tyr Tyr Cys Gln Gln Ser Phe
                85                  90                  95
Glu Asp Pro Leu Thr Phe Gly Gln Gly Thr Lys Leu Glu Ile Lys Arg
            100                 105                 110
```

```
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  18
<211>  449
<212>  PRT
<213>  (Artificial Sequence)

<220>
<221>  PEPTIDE
<223>  h1830G1


<400>  18
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
```

```
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys


<210>  19
<211>  357
<212>  DNA
<213>  Homo sapiens

<220>
<221>  gene
<223>  SIRP  DNA


<400>  19
gaggaggagc tacagatgat tcagcctgag aagctcctgt tggtcacagt tggaaagaca       60
gccactctgc actgcactgt gacctccctg cttcccgtgg gacccgtcct gtggttcaga      120
ggagttggac caggccggga attaatctac aatcaaaaag aaggccactt ccccagggta      180
acaacagttt cagacctcac aaagagaaac aacatggact ttccatccg catcagtagc       240
atcaccccag cagatgtcgg cacatactac tgtgtgaagt ttcgaaaagg gagccctgag      300
aacgtggagt ttaagtctgg accaggcact gagatggctt tgggtgccaa accctct         357


<210>  20
<211>  119
<212>  PRT
<213>  (Artificial Sequence)

<220>
<221>  PEPTIDE
<223>  SIRP


<400>  20
Glu Glu Glu Leu Gln Met Ile Gln Pro Glu Lys Leu Leu Leu Val Thr
1               5                   10                  15
Val Gly Lys Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Asn Gln Lys Glu Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
```

```
                85                  90                  95
Gly Ser Pro Glu Asn Val Glu Phe Lys Ser Gly Pro Gly Thr Glu Met
            100                 105                 110
Ala Leu Gly Ala Lys Pro Ser
        115

<210>  21
<211>  119
<212>  PRT
<213>  (Artificial Sequence)

<220>
<221>  PEPTIDE
<223>  S58


<400>  21
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Met Ser Gly Arg Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115

<210>  22
<211>  119
<212>  PRT
<213>  (Artificial Sequence)

<220>
<221>  PEPTIDE
<223>  S79


<400>  22
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Trp Pro
            20                  25                  30
Val Gly Pro Val Leu Trp Phe Arg Gly Val Gly Pro Gly Arg Glu Leu
        35                  40                  45
Ile Tyr Arg Thr Gly Thr Gly Arg Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Val
            100                 105                 110
Ala Leu Gly Ala Lys Pro Ser
        115

<210>  23
```

<211> 119
<212> PRT
<213> (Artificial Sequence)

<220>
<221> PEPTIDE
<223> S15

<400> 23
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Val Ser Gly Arg Gly His Phe Pro Arg Val Thr Thr Val Ser
50 55 60
Asp Leu Thr Lys Arg Glu Asn Arg Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 24
<211> 119
<212> PRT
<213> (Artificial Sequence)

<220>
<221> PEPTIDE
<223> S12

<400> 24
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Val Ser Gly Arg Gly His Phe Pro Arg Val Thr Thr Val Ser
50 55 60
Asp Leu Thr Lys Arg Glu Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 25
<211> 119
<212> PRT
<213> (Artificial Sequence)

<220>
<221> PEPTIDE
<223> S19

<400> 25
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Val Ser Gly Arg Gly His Phe Pro Arg Val Thr Thr Val Ser
50 55 60
Asp Leu Thr Lys Arg Asn Asn Arg Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 26
<211> 119
<212> PRT
<213> (Artificial Sequence)

<220>
<221> PEPTIDE
<223> S85

<400> 26
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Val Ser Gly Arg Gly His Phe Pro Arg Val Thr Thr Val Ser
50 55 60
Asp Leu Thr Lys Arg Asn Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 27
<211> 119
<212> PRT
<213> (Artificial Sequence)

<220>
<221> PEPTIDE
<223> S37

<400> 27
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30

Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asn Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 28
<211> 119
<212> PRT
<213> (Artificial Sequence)

<220>
<221> PEPTIDE
<223> S38

<400> 28
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Phe Ser Gly Arg Gly His Phe Pro Arg Val Thr Thr Val Ser
50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 29
<211> 119
<212> PRT
<213> (Artificial Sequence)

<220>
<221> PEPTIDE
<223> S22

<400> 29
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Ile Ser Gly Arg Gly His Phe Pro Arg Val Thr Thr Val Ser
50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys

```
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115

<210>  30
<211>  119
<212>  PRT
<213>  (Artificial Sequence)

<220>
<221>  PEPTIDE
<223>  S29


<400>  30
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Leu Ser Gly Arg Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115

<210>  31
<211>  119
<212>  PRT
<213>  (Artificial Sequence)

<220>
<221>  PEPTIDE
<223>  S34


<400>  31
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Arg Ser Gly Arg Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115

<210>  32
```

<211> 119
<212> PRT
<213> (Artificial Sequence)

<220>
<221> PEPTIDE
<223> S41

<400> 32

```
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Val Ser Gly Arg Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115
```

<210> 33
<211> 119
<212> PRT
<213> (Artificial Sequence)

<220>
<221> PEPTIDE
<223> S42

<400> 33

```
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Met Ser Gly Arg Gly His Phe Pro Arg Val Thr Thr Val Ser
    50                  55                  60
Asp Leu Thr Lys Arg Asn Asn Ile Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115
```

<210> 34
<211> 119
<212> PRT
<213> (Artificial Sequence)

<220>
<221> PEPTIDE
<223> S43

<400> 34
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asn Asn Arg Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 35
<211> 119
<212> PRT
<213> (Artificial Sequence)

<220>
<221> PEPTIDE
<223> S44

<400> 35
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asn Asn Val Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 36
<211> 119
<212> PRT
<213> (Artificial Sequence)

<220>
<221> PEPTIDE
<223> S45

<400> 36
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30

Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asp Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 37
<211> 119
<212> PRT
<213> (Artificial Sequence)

<220>
<221> PEPTIDE
<223> S46

<400> 37
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Glu Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser
115

<210> 38
<211> 119
<212> PRT
<213> (Artificial Sequence)

<220>
<221> PEPTIDE
<223> S47

<400> 38
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Glu Asn Lys Asp Phe Ser Ile Arg Ile Ser Ser
65 70 75 80
Ile Thr Pro Ala Asp Val Gly Thr Tyr Tyr Cys Val Lys Phe Arg Lys

```
                85                  90                  95
Gly Ser Pro Glu Asp Val Glu Phe Lys Ser Gly Pro Gly Thr Glu Val
            100                 105                 110
Ala Leu Gly Ala Lys Pro Ser
        115

<210>  39
<211>  119
<212>  PRT
<213>  (Artificial Sequence)

<220>
<221>  PEPTIDE
<223>  S48


<400>  39
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Arg Ser Gly Arg Gly His Phe Pro Arg Val Thr Thr Val Ser
    50                  55                  60
Asp Leu Thr Lys Arg Asn Asn Lys Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115

<210>  40
<211>  119
<212>  PRT
<213>  (Artificial Sequence)

<220>
<221>  PEPTIDE
<223>  S49


<400>  40
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Arg Ser Gly Arg Gly His Phe Pro Arg Val Thr Thr Val Ser
    50                  55                  60
Asp Leu Thr Lys Arg Glu Asn Lys Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser
        115

<210>  41
```

<211> 581
<212> PRT
<213> (Artificial Sequence)

<220>
<221> PEPTIDE
<223> h1830-S58

<400> 41

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
```

```
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Met Ser Gly Arg Gly His Phe Pro Arg Val Thr Thr Val Ser Asp Leu
        515                 520                 525
Thr Lys Arg Asn Asn Met Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580

<210>  42
<211>  581
<212>  PRT
<213>  (Artificial Sequence)

<220>
<221>  PEPTIDE
<223>  h1830-S15


<400>  42
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
```

Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Val Ser Gly Arg Gly His Phe Pro Arg Val Thr Thr Val Ser Asp Leu
        515                 520                 525
Thr Lys Arg Glu Asn Arg Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580

<210> 43
<211> 581
<212> PRT
<213> (Artificial Sequence)

<220>
<221> PEPTIDE
<223> h1830-S12

<400> 43
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser

```
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Val Ser Gly Arg Gly His Phe Pro Arg Val Thr Thr Val Ser Asp Leu
        515                 520                 525
Thr Lys Arg Glu Asn Lys Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
```

Pro Glu Asp Val Glu Phe Lys Ser Gly Pro Gly Thr Glu Met Ala Leu
565 570 575
Gly Ala Lys Pro Ser
580

<210> 44
<211> 581
<212> PRT
<213> (Artificial Sequence)

<220>
<221> PEPTIDE
<223> h1830-S19

<400> 44
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn

370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Val Ser Gly Arg Gly His Phe Pro Arg Val Thr Thr Val Ser Asp Leu
515 520 525
Thr Lys Arg Asn Asn Arg Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 45
<211> 581
<212> PRT
<213> (Artificial Sequence)

<220>
<221> PEPTIDE
<223> h1830-S85

<400> 45
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190

Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Val Ser Gly Arg Gly His Phe Pro Arg Val Thr Thr Val Ser Asp Leu
515 520 525
Thr Lys Arg Asn Asn Lys Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 46
<211> 581
<212> PRT
<213> (Artificial Sequence)

<220>
<221> PEPTIDE
<223> h1830-S37

<400> 46
Gln Val Gln Leu Gln Glu Ser Gly Pro Gly Leu Val Lys Pro Ser Gln

```
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
```

Met Ser Gly Arg Gly His Phe Pro Arg Val Thr Thr Val Ser Asp Leu
515 520 525
Thr Lys Arg Asn Asn Lys Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 47
<211> 584
<212> PRT
<213> (Artificial Sequence)

<220>
<221> PEPTIDE
<223> h1830-19-S79

<400> 47
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile

```
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Ser Gly Gly Gly
    450                 455                 460
Gly Glu Glu Glu Leu Gln Met Ile Gln Pro Glu Lys Leu Leu Leu Val
465                 470                 475                 480
Thr Val Gly Glu Thr Ala Thr Leu His Cys Thr Val Thr Ser Leu Trp
                485                 490                 495
Pro Val Gly Pro Val Leu Trp Phe Arg Gly Val Gly Pro Gly Arg Glu
            500                 505                 510
Leu Ile Tyr Arg Thr Gly Thr Gly Arg Phe Pro Arg Val Thr Thr Val
        515                 520                 525
Ser Asp Leu Thr Lys Arg Asn Asn Met Asp Phe Ser Ile Arg Ile Ser
    530                 535                 540
Ser Ile Thr Pro Ala Asp Val Gly Thr Tyr Tyr Cys Val Lys Phe Arg
545                 550                 555                 560
Lys Gly Ser Pro Glu Asp Val Glu Phe Lys Ser Gly Pro Gly Thr Glu
                565                 570                 575
Val Ala Leu Gly Ala Lys Pro Ser
            580
```

<210> 48
<211> 581
<212> PRT
<213> (Artificial Sequence)

<220>
<221> PEPTIDE
<223> h1830-S79

<400> 48

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
```

```
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Trp Pro Val Gly
                485                 490                 495
Pro Val Leu Trp Phe Arg Gly Val Gly Pro Gly Arg Glu Leu Ile Tyr
            500                 505                 510
Arg Thr Gly Thr Gly Arg Phe Pro Arg Val Thr Thr Val Ser Asp Leu
        515                 520                 525
Thr Lys Arg Asn Asn Met Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Val Ala Leu
                565                 570                 575
Gly Ala Lys Pro Ser
            580

<210>  49
<211>  589
<212>  PRT
<213>  (Artificial Sequence)

<220>
```

<221> PEPTIDE
<223> h1830G1-19-S79

<400> 49
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Gly Gly Gly Gly Gly Ser Gly Gly Gly Gly Ser Gly Gly Gly
450 455 460

Gly Ser Gly Gly Gly Gly Glu Glu Glu Leu Gln Met Ile Gln Pro Glu
465 470 475 480
Lys Leu Leu Leu Val Thr Val Gly Glu Thr Ala Thr Leu His Cys Thr
485 490 495
Val Thr Ser Leu Trp Pro Val Gly Pro Val Leu Trp Phe Arg Gly Val
500 505 510
Gly Pro Gly Arg Glu Leu Ile Tyr Arg Thr Gly Thr Gly Arg Phe Pro
515 520 525
Arg Val Thr Thr Val Ser Asp Leu Thr Lys Arg Asn Asn Met Asp Phe
530 535 540
Ser Ile Arg Ile Ser Ser Ile Thr Pro Ala Asp Val Gly Thr Tyr Tyr
545 550 555 560
Cys Val Lys Phe Arg Lys Gly Ser Pro Glu Asp Val Glu Phe Lys Ser
565 570 575
Gly Pro Gly Thr Glu Val Ala Leu Gly Ala Lys Pro Ser
580 585

<210> 50
<211> 581
<212> PRT
<213> (Artificial Sequence)

<220>
<221> PEPTIDE
<223> h1830-S38

<400> 50
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys

275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Phe Ser Gly Arg Gly His Phe Pro Arg Val Thr Thr Val Ser Asp Leu
515 520 525
Thr Lys Arg Asn Asn Met Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 51
<211> 581
<212> PRT
<213> (Artificial Sequence)

<220>
<221> PEPTIDE
<223> h1830-S22

<400> 51
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95

```
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Ile Ser Gly Arg Gly His Phe Pro Arg Val Thr Thr Val Ser Asp Leu
        515                 520                 525
Thr Lys Arg Asn Asn Met Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580
```

<210> 52
<211> 581
<212> PRT
<213> (Artificial Sequence)

<220>
<221> PEPTIDE
<223> h1830-S29

<400> 52
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415

Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Leu Ser Gly Arg Gly His Phe Pro Arg Val Thr Thr Val Ser Asp Leu
515 520 525
Thr Lys Arg Asn Asn Met Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 53
<211> 581
<212> PRT
<213> (Artificial Sequence)

<220>
<221> PEPTIDE
<223> h1830-S34

<400> 53
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val

```
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Arg Ser Gly Arg Gly His Phe Pro Arg Val Thr Thr Val Ser Asp Leu
        515                 520                 525
Thr Lys Arg Asn Asn Met Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580
```

<210> 54
<211> 581
<212> PRT
<213> (Artificial Sequence)

<220>
<221> PEPTIDE
<223> h1830-S41

<400> 54

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
```

Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Val Ser Gly Arg Gly His Phe Pro Arg Val Thr Thr Val Ser Asp Leu
        515                 520                 525
Thr Lys Arg Asn Asn Met Asp Phe Ser Ile Arg Ile Ser Ser Ile Thr
    530                 535                 540
Pro Ala Asp Val Gly Thr Tyr Tyr Cys Val Lys Phe Arg Lys Gly Ser

```
545                 550                 555                 560
Pro Glu Asp Val Glu Phe Lys Ser Gly Pro Gly Thr Glu Met Ala Leu
                565                 570                 575
Gly Ala Lys Pro Ser
            580
```

<210> 55
<211> 581
<212> PRT
<213> (Artificial Sequence)

<220>
<221> PEPTIDE
<223> h1830-S42

<400> 55

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
```

Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Met Ser Gly Arg Gly His Phe Pro Arg Val Thr Thr Val Ser Asp Leu
515 520 525
Thr Lys Arg Asn Asn Ile Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 56
<211> 581
<212> PRT
<213> (Artificial Sequence)

<220>
<221> PEPTIDE
<223> h1830-S43

<400> 56
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro

```
                180                 185                 190
Ser Ser Ser Leu Gly Thr Lys Thr Tyr Thr Cys Asn Val Asp His Lys
            195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Met Ser Gly Arg Gly His Phe Pro Arg Val Thr Thr Val Ser Asp Leu
        515                 520                 525
Thr Lys Arg Asn Asn Arg Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580
```

<210> 57
<211> 581
<212> PRT
<213> (Artificial Sequence)

<220>
<221> PEPTIDE
<223> h1830-S44

<400> 57

Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr

```
                500                 505                 510
Met Ser Gly Arg Gly His Phe Pro Arg Val Thr Thr Val Ser Asp Leu
            515                 520                 525
Thr Lys Arg Asn Asn Val Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
                580
```

<210> 58
<211> 581
<212> PRT
<213> (Artificial Sequence)

<220>
<221> PEPTIDE
<223> h1830-S45

<400> 58

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
```

Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Met Ser Gly Arg Gly His Phe Pro Arg Val Thr Thr Val Ser Asp Leu
515 520 525
Thr Lys Arg Asp Asn Met Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 59
<211> 581
<212> PRT
<213> (Artificial Sequence)

<220>
<221> PEPTIDE
<223> h1830-S46

<400> 59
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala

```
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Met Ser Gly Arg Gly His Phe Pro Arg Val Thr Thr Val Ser Asp Leu
        515                 520                 525
Thr Lys Arg Glu Asn Lys Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580
```

<210> 60
<211> 581
<212> PRT
<213> (Artificial Sequence)

<220>
<221> PEPTIDE
<223> h1830-S47

<400> 60

```
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Asp Gly
            20                  25                  30
Ser Ala Tyr Trp Ser Trp Ile Arg Gln His Pro Gly Lys Gly Leu Glu
        35                  40                  45
Tyr Ile Gly Phe Ile Ser Arg Ala Gly Ser Thr Tyr Asn Thr Pro Ser
    50                  55                  60
Leu Lys Gly Arg Val Thr Ile Ser Arg Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
```

450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Met Ser Gly Arg Gly His Phe Pro Arg Val Thr Thr Val Ser Asp Leu
515 520 525
Thr Lys Arg Glu Asn Lys Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Val Ala Leu
565 570 575
Gly Ala Lys Pro Ser
580

<210> 61
<211> 581
<212> PRT
<213> (Artificial Sequence)

<220>
<221> PEPTIDE
<223> h1830-S48

<400> 61
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr
85 90 95
Cys Ala Arg Ser Gly Gly Trp Leu Ala Pro Phe Asp Tyr Trp Gly Arg
100 105 110
Gly Thr Leu Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Lys Thr Tyr Thr Cys Asn Val Asp His Lys
195 200 205
Pro Ser Asn Thr Lys Val Asp Lys Arg Val Glu Ser Lys Tyr Gly Pro
210 215 220
Pro Cys Pro Pro Cys Pro Ala Pro Glu Phe Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285
Thr Lys Pro Arg Glu Glu Gln Phe Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Gly Leu Pro Ser Ser Ile Glu Lys Thr Ile
325 330 335
Ser Lys Ala Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Arg Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Leu Gly Gly Gly
435 440 445
Gly Gly Ser Gly Gly Gly Gly Ser Gly Gly Gly Gly Gly Gly Glu Glu
450 455 460
Glu Leu Gln Met Ile Gln Pro Glu Lys Leu Leu Leu Val Thr Val Gly
465 470 475 480
Glu Thr Ala Thr Leu His Cys Thr Val Thr Ser Leu Leu Pro Val Gly
485 490 495
Pro Val Leu Trp Phe Arg Gly Val Gly Pro Gly Arg Glu Leu Ile Tyr
500 505 510
Arg Ser Gly Arg Gly His Phe Pro Arg Val Thr Thr Val Ser Asp Leu
515 520 525
Thr Lys Arg Asn Asn Lys Asp Phe Ser Ile Arg Ile Ser Ser Ile Thr
530 535 540
Pro Ala Asp Val Gly Thr Tyr Tyr Cys Val Lys Phe Arg Lys Gly Ser
545 550 555 560
Pro Glu Asp Val Glu Phe Lys Ser Gly Pro Gly Thr Glu Met Ala Leu
565 570 575
Gly Ala Lys Pro Ser
580

<210> 62
<211> 581
<212> PRT
<213> (Artificial Sequence)

<220>
<221> PEPTIDE
<223> h1830-S49

<400> 62
Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Asp Gly
20 25 30
Ser Ala Tyr Trp Ser Trp Ile Arg Gln His Pro Gly Lys Gly Leu Glu
35 40 45
Tyr Ile Gly Phe Ile Ser Arg Ala Gly Ser Thr Tyr Asn Thr Pro Ser
50 55 60
Leu Lys Gly Arg Val Thr Ile Ser Arg Asp Thr Ser Lys Asn Gln Phe
65 70 75 80
Ser Leu Lys Leu Ser Ser Val Thr Ala Ala Asp Thr Ala Val Tyr Tyr

```
                85                  90                  95
Cys Ala Arg Ser Gly Gly Trp Leu Ala Pro Phe Asp Tyr Trp Gly Arg
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Gly Gly
        435                 440                 445
Gly Gly Ser Gly Gly Gly Gly Ser Gly Gly Gly Gly Gly Gly Glu Glu
    450                 455                 460
Glu Leu Gln Met Ile Gln Pro Glu Lys Leu Leu Leu Val Thr Val Gly
465                 470                 475                 480
Glu Thr Ala Thr Leu His Cys Thr Val Thr Ser Leu Leu Pro Val Gly
                485                 490                 495
Pro Val Leu Trp Phe Arg Gly Val Gly Pro Gly Arg Glu Leu Ile Tyr
            500                 505                 510
Arg Ser Gly Arg Gly His Phe Pro Arg Val Thr Thr Val Ser Asp Leu
        515                 520                 525
Thr Lys Arg Glu Asn Lys Asp Phe Ser Ile Arg Ile Ser Ser Ile Thr
    530                 535                 540
Pro Ala Asp Val Gly Thr Tyr Tyr Cys Val Lys Phe Arg Lys Gly Ser
545                 550                 555                 560
Pro Glu Asp Val Glu Phe Lys Ser Gly Pro Gly Thr Glu Met Ala Leu
                565                 570                 575
Gly Ala Lys Pro Ser
            580
```

<210> 63
<211> 583
<212> PRT
<213> (Artificial Sequence)

<220>
<221> PEPTIDE
<223> h1831-19-S58

<400> 63
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp

405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Ser Gly Gly Gly Gly
450 455 460
Glu Glu Glu Leu Gln Met Ile Gln Pro Glu Lys Leu Leu Leu Val Thr
465 470 475 480
Val Gly Glu Thr Ala Thr Leu His Cys Thr Val Thr Ser Leu Leu Pro
485 490 495
Val Gly Pro Val Leu Trp Phe Arg Gly Val Gly Pro Gly Arg Glu Leu
500 505 510
Ile Tyr Met Ser Gly Arg Gly His Phe Pro Arg Val Thr Thr Val Ser
515 520 525
Asp Leu Thr Lys Arg Asn Asn Met Asp Phe Ser Ile Arg Ile Ser Ser
530 535 540
Ile Thr Pro Ala Asp Val Gly Thr Tyr Tyr Cys Val Lys Phe Arg Lys
545 550 555 560
Gly Ser Pro Glu Asp Val Glu Phe Lys Ser Gly Pro Gly Thr Glu Met
565 570 575
Ala Leu Gly Ala Lys Pro Ser
580

<210> 64
<211> 580
<212> PRT
<213> (Artificial Sequence)

<220>
<221> PEPTIDE
<223> h1831-S15

<400> 64
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220

Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro
485 490 495
Val Leu Trp Phe Arg Gly Val Gly Pro Gly Arg Glu Leu Ile Tyr Val
500 505 510
Ser Gly Arg Gly His Phe Pro Arg Val Thr Thr Val Ser Asp Leu Thr
515 520 525
Lys Arg Glu Asn Arg Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 65
<211> 580
<212> PRT
<213> (Artificial Sequence)

<220>
<221> PEPTIDE
<223> h1831-S12

<400> 65
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met

```
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Val
            500                 505                 510
Ser Gly Arg Gly His Phe Pro Arg Val Thr Thr Val Ser Asp Leu Thr
        515                 520                 525
Lys Arg Glu Asn Lys Asp Phe Ser Ile Arg Ile Ser Ser Ile Thr Pro
    530                 535                 540
```

Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 66
<211> 580
<212> PRT
<213> (Artificial Sequence)

<220>
<221> PEPTIDE
<223> h1831-S19

<400> 66
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val

355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro
485 490 495
Val Leu Trp Phe Arg Gly Val Gly Pro Gly Arg Glu Leu Ile Tyr Val
500 505 510
Ser Gly Arg Gly His Phe Pro Arg Val Thr Thr Val Ser Asp Leu Thr
515 520 525
Lys Arg Asn Asn Arg Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 67
<211> 580
<212> PRT
<213> (Artificial Sequence)

<220>
<221> PEPTIDE
<223> h1831-S85

<400> 67
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175

```
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Val
            500                 505                 510
Ser Gly Arg Gly His Phe Pro Arg Val Thr Thr Val Ser Asp Leu Thr
        515                 520                 525
Lys Arg Asn Asn Lys Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580
```

<210> 68
<211> 580
<212> PRT
<213> (Artificial Sequence)

<220>
<221> PEPTIDE
<223> h1831-S37

<400> 68

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
```

Val Leu Trp Phe Arg Gly Val Gly Pro Gly Arg Glu Leu Ile Tyr Met
500 505 510
Ser Gly Arg Gly His Phe Pro Arg Val Thr Thr Val Ser Asp Leu Thr
515 520 525
Lys Arg Asn Asn Lys Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 69
<211> 583
<212> PRT
<213> (Artificial Sequence)

<220>
<221> PEPTIDE
<223> h1831-19-S79

<400> 69
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys

305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Ser Gly Gly Gly Gly
450 455 460
Glu Glu Glu Leu Gln Met Ile Gln Pro Glu Lys Leu Leu Leu Val Thr
465 470 475 480
Val Gly Glu Thr Ala Thr Leu His Cys Thr Val Thr Ser Leu Trp Pro
485 490 495
Val Gly Pro Val Leu Trp Phe Arg Gly Val Gly Pro Gly Arg Glu Leu
500 505 510
Ile Tyr Arg Thr Gly Thr Gly Arg Phe Pro Arg Val Thr Thr Val Ser
515 520 525
Asp Leu Thr Lys Arg Asn Asn Met Asp Phe Ser Ile Arg Ile Ser Ser
530 535 540
Ile Thr Pro Ala Asp Val Gly Thr Tyr Tyr Cys Val Lys Phe Arg Lys
545 550 555 560
Gly Ser Pro Glu Asp Val Glu Phe Lys Ser Gly Pro Gly Thr Glu Val
565 570 575
Ala Leu Gly Ala Lys Pro Ser
580

<210> 70
<211> 580
<212> PRT
<213> (Artificial Sequence)

<220>
<221> PEPTIDE
<223> h1831-S38

<400> 70
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125

```
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Phe
            500                 505                 510
Ser Gly Arg Gly His Phe Pro Arg Val Thr Thr Val Ser Asp Leu Thr
        515                 520                 525
Lys Arg Asn Asn Met Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580
```

<210> 71
<211> 580
<212> PRT
<213> (Artificial Sequence)

<220>
<221> PEPTIDE
<223> h1831-S22

<400> 71

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
```

Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Ile
            500                 505                 510
Ser Gly Arg Gly His Phe Pro Arg Val Thr Thr Val Ser Asp Leu Thr
        515                 520                 525
Lys Arg Asn Asn Met Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580

<210>  72
<211>  580
<212>  PRT
<213>  (Artificial Sequence)

<220>
<221>  PEPTIDE
<223>  h1831-S29

<400>  72
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val

260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro
485 490 495
Val Leu Trp Phe Arg Gly Val Gly Pro Gly Arg Glu Leu Ile Tyr Leu
500 505 510
Ser Gly Arg Gly His Phe Pro Arg Val Thr Thr Val Ser Asp Leu Thr
515 520 525
Lys Arg Asn Asn Met Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 73
<211> 580
<212> PRT
<213> (Artificial Sequence)

<220>
<221> PEPTIDE
<223> h1831-S34

<400> 73
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro
485 490 495
Val Leu Trp Phe Arg Gly Val Gly Pro Gly Arg Glu Leu Ile Tyr Arg
500 505 510
Ser Gly Arg Gly His Phe Pro Arg Val Thr Thr Val Ser Asp Leu Thr
515 520 525
Lys Arg Asn Asn Met Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser

580

<210> 74
<211> 580
<212> PRT
<213> (Artificial Sequence)

<220>
<221> PEPTIDE
<223> h1831-S41

<400> 74
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400

```
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Val
            500                 505                 510
Ser Gly Arg Gly His Phe Pro Arg Val Thr Thr Val Ser Asp Leu Thr
        515                 520                 525
Lys Arg Asn Asn Met Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580
```

<210> 75
<211> 580
<212> PRT
<213> (Artificial Sequence)

<220>
<221> PEPTIDE
<223> h1831-S42

<400> 75

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
```

```
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Met
            500                 505                 510
Ser Gly Arg Gly His Phe Pro Arg Val Thr Thr Val Ser Asp Leu Thr
        515                 520                 525
Lys Arg Asn Asn Ile Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580

<210>  76
<211>  580
<212>  PRT
<213>  (Artificial Sequence)

<220>
<221>  PEPTIDE
<223>  h1831-S43


<400>  76
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
```

```
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Met
            500                 505                 510
Ser Gly Arg Gly His Phe Pro Arg Val Thr Thr Val Ser Asp Leu Thr
        515                 520                 525
Lys Arg Asn Asn Arg Asp Phe Ser Ile Arg Ile Ser Ser Ile Thr Pro
```

```
        530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580

<210>  77
<211>  580
<212>  PRT
<213>  (Artificial Sequence)

<220>
<221>  PEPTIDE
<223>  h1831-S44


<400>  77
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Thr Pro Ser Ser Gly Phe Ala Met Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
```

Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro
485 490 495
Val Leu Trp Phe Arg Gly Val Gly Pro Gly Arg Glu Leu Ile Tyr Met
500 505 510
Ser Gly Arg Gly His Phe Pro Arg Val Thr Thr Val Ser Asp Leu Thr
515 520 525
Lys Arg Asn Asn Val Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 78
<211> 580
<212> PRT
<213> (Artificial Sequence)

<220>
<221> PEPTIDE
<223> h1831-S45

<400> 78
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu

```
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Met
            500                 505                 510
Ser Gly Arg Gly His Phe Pro Arg Val Thr Thr Val Ser Asp Leu Thr
        515                 520                 525
Lys Arg Asp Asn Met Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580
```

<210> 79
<211> 580
<212> PRT
<213> (Artificial Sequence)

<220>
<221> PEPTIDE
<223> h1831-S46

<400> 79
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro

485 490 495
Val Leu Trp Phe Arg Gly Val Gly Pro Gly Arg Glu Leu Ile Tyr Met
500 505 510
Ser Gly Arg Gly His Phe Pro Arg Val Thr Thr Val Ser Asp Leu Thr
515 520 525
Lys Arg Glu Asn Lys Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Met Ala Leu Gly
565 570 575
Ala Lys Pro Ser
580

<210> 80
<211> 580
<212> PRT
<213> (Artificial Sequence)

<220>
<221> PEPTIDE
<223> h1831-S47

<400> 80
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300

Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Gly Gly Glu Glu Glu
450 455 460
Leu Gln Met Ile Gln Pro Glu Lys Leu Leu Leu Val Thr Val Gly Glu
465 470 475 480
Thr Ala Thr Leu His Cys Thr Val Thr Ser Leu Leu Pro Val Gly Pro
485 490 495
Val Leu Trp Phe Arg Gly Val Gly Pro Gly Arg Glu Leu Ile Tyr Met
500 505 510
Ser Gly Arg Gly His Phe Pro Arg Val Thr Thr Val Ser Asp Leu Thr
515 520 525
Lys Arg Glu Asn Lys Asp Phe Ser Ile Arg Ile Ser Ser Ile Thr Pro
530 535 540
Ala Asp Val Gly Thr Tyr Tyr Cys Val Lys Phe Arg Lys Gly Ser Pro
545 550 555 560
Glu Asp Val Glu Phe Lys Ser Gly Pro Gly Thr Glu Val Ala Leu Gly
565 570 575
Ala Lys Pro Ser
580

<210> 81
<211> 580
<212> PRT
<213> (Artificial Sequence)

<220>
<221> PEPTIDE
<223> h1831-S48

<400> 81
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe

```
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Gly Gly Gly
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Arg
            500                 505                 510
Ser Gly Arg Gly His Phe Pro Arg Val Thr Thr Val Ser Asp Leu Thr
        515                 520                 525
Lys Arg Asn Asn Lys Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580
```

<210> 82
<211> 580
<212> PRT

<213> (Artificial Sequence)

<220>
<221> PEPTIDE
<223> h1831-S49

<400> 82
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly

```
        435                 440                 445
Gly Ser Gly Gly Gly Gly Ser Gly Gly Gly Gly Gly Gly Glu Glu Glu
    450                 455                 460
Leu Gln Met Ile Gln Pro Glu Lys Leu Leu Leu Val Thr Val Gly Glu
465                 470                 475                 480
Thr Ala Thr Leu His Cys Thr Val Thr Ser Leu Leu Pro Val Gly Pro
                485                 490                 495
Val Leu Trp Phe Arg Gly Val Gly Pro Gly Arg Glu Leu Ile Tyr Arg
            500                 505                 510
Ser Gly Arg Gly His Phe Pro Arg Val Thr Thr Val Ser Asp Leu Thr
        515                 520                 525
Lys Arg Glu Asn Lys Asp Phe Ser Ile Arg Ile Ser Ser Ile Thr Pro
    530                 535                 540
Ala Asp Val Gly Thr Tyr Tyr Cys Val Lys Phe Arg Lys Gly Ser Pro
545                 550                 555                 560
Glu Asp Val Glu Phe Lys Ser Gly Pro Gly Thr Glu Met Ala Leu Gly
                565                 570                 575
Ala Lys Pro Ser
            580

<210>  83
<211>  444
<212>  PRT
<213>  (Artificial Sequence)

<220>
<221>  PEPTIDE
<223>  Hu5F9


<400>  83
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Asn Met His Trp Val Arg Gln Ala Pro Gly Gln Arg Leu Glu Trp Met
        35                  40                  45
Gly Thr Ile Tyr Pro Gly Asn Asp Asp Thr Ser Tyr Asn Gln Lys Phe
    50                  55                  60
Lys Asp Arg Val Thr Ile Thr Ala Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Arg Ala Met Asp Tyr Trp Gly Gln Gly Thr Leu
            100                 105                 110
Val Thr Val Ser Ser Ala Ser Thr Lys Gly Pro Ser Val Phe Pro Leu
        115                 120                 125
Ala Pro Cys Ser Arg Ser Thr Ser Glu Ser Thr Ala Ala Leu Gly Cys
    130                 135                 140
Leu Val Lys Asp Tyr Phe Pro Glu Pro Val Thr Val Ser Trp Asn Ser
145                 150                 155                 160
Gly Ala Leu Thr Ser Gly Val His Thr Phe Pro Ala Val Leu Gln Ser
                165                 170                 175
Ser Gly Leu Tyr Ser Leu Ser Ser Val Val Thr Val Pro Ser Ser Ser
            180                 185                 190
Leu Gly Thr Lys Thr Tyr Thr Cys Asn Val Asp His Lys Pro Ser Asn
        195                 200                 205
Thr Lys Val Asp Lys Arg Val Glu Ser Lys Tyr Gly Pro Pro Cys Pro
    210                 215                 220
Pro Cys Pro Ala Pro Glu Phe Leu Gly Gly Pro Ser Val Phe Leu Phe
225                 230                 235                 240
Pro Pro Lys Pro Lys Asp Thr Leu Met Ile Ser Arg Thr Pro Glu Val
                245                 250                 255
```

Thr Cys Val Val Val Asp Val Ser Gln Glu Asp Pro Glu Val Gln Phe
260 265 270
Asn Trp Tyr Val Asp Gly Val Glu Val His Asn Ala Lys Thr Lys Pro
275 280 285
Arg Glu Glu Gln Phe Asn Ser Thr Tyr Arg Val Val Ser Val Leu Thr
290 295 300
Val Leu His Gln Asp Trp Leu Asn Gly Lys Glu Tyr Lys Cys Lys Val
305 310 315 320
Ser Asn Lys Gly Leu Pro Ser Ser Ile Glu Lys Thr Ile Ser Lys Ala
325 330 335
Lys Gly Gln Pro Arg Glu Pro Gln Val Tyr Thr Leu Pro Pro Ser Gln
340 345 350
Glu Glu Met Thr Lys Asn Gln Val Ser Leu Thr Cys Leu Val Lys Gly
355 360 365
Phe Tyr Pro Ser Asp Ile Ala Val Glu Trp Glu Ser Asn Gly Gln Pro
370 375 380
Glu Asn Asn Tyr Lys Thr Thr Pro Pro Val Leu Asp Ser Asp Gly Ser
385 390 395 400
Phe Phe Leu Tyr Ser Arg Leu Thr Val Asp Lys Ser Arg Trp Gln Glu
405 410 415
Gly Asn Val Phe Ser Cys Ser Val Met His Glu Ala Leu His Asn His
420 425 430
Tyr Thr Gln Lys Ser Leu Ser Leu Ser Leu Gly Lys
435 440

<210> 84
<211> 219
<212> PRT
<213> (Artificial Sequence)

<220>
<221> PEPTIDE
<223> Hu5F9

<400> 84
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Ile Val Tyr Ser
20 25 30
Asn Gly Asn Thr Tyr Leu Gly Trp Tyr Leu Gln Lys Pro Gly Gln Ser
35 40 45
Pro Gln Leu Leu Ile Tyr Lys Val Ser Asn Arg Phe Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Phe Gln Gly
85 90 95
Ser His Val Pro Tyr Thr Phe Gly Gln Gly Thr Lys Leu Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu Gln
145 150 155 160
Ser Gly Asn Ser Gln Glu Ser Val Thr Glu Gln Asp Ser Lys Asp Ser
165 170 175
Thr Tyr Ser Leu Ser Ser Thr Leu Thr Leu Ser Lys Ala Asp Tyr Glu
180 185 190
Lys His Lys Val Tyr Ala Cys Glu Val Thr His Gln Gly Leu Ser Ser
195 200 205
Pro Val Thr Lys Ser Phe Asn Arg Gly Glu Cys

210 215

<210> 85
<211> 351
<212> PRT
<213> (Artificial Sequence)

<220>
<221> PEPTIDE
<223> SIRP-CV

<400> 85
Glu Glu Glu Leu Gln Ile Ile Gln Pro Asp Lys Ser Val Leu Val Ala
1 5 10 15
Ala Gly Glu Thr Ala Thr Leu Arg Cys Thr Ile Thr Ser Leu Phe Pro
20 25 30
Val Gly Pro Ile Gln Trp Phe Arg Gly Ala Gly Pro Gly Arg Val Leu
35 40 45
Ile Tyr Asn Gln Arg Gln Gly Pro Phe Pro Arg Val Thr Thr Val Ser
50 55 60
Asp Thr Thr Lys Arg Asn Asn Met Asp Phe Ser Ile Arg Ile Gly Asn
65 70 75 80
Ile Thr Pro Ala Asp Ala Gly Thr Tyr Tyr Cys Ile Lys Phe Arg Lys
85 90 95
Gly Ser Pro Asp Asp Val Glu Phe Lys Ser Gly Ala Gly Thr Glu Leu
100 105 110
Ser Val Arg Ala Lys Pro Ser Glu Pro Lys Ser Ser Asp Lys Thr His
115 120 125
Thr Cys Pro Pro Cys Pro Ala Pro Glu Leu Leu Gly Gly Pro Ser Val
130 135 140
Phe Leu Phe Pro Pro Lys Pro Lys Asp Thr Leu Met Ile Ser Arg Thr
145 150 155 160
Pro Glu Val Thr Cys Val Val Val Asp Val Ser His Glu Asp Pro Glu
165 170 175
Val Lys Phe Asn Trp Tyr Val Asp Gly Val Glu Val His Asn Ala Lys
180 185 190
Thr Lys Pro Arg Glu Glu Gln Tyr Asn Ser Thr Tyr Arg Val Val Ser
195 200 205
Val Leu Thr Val Leu His Gln Asp Trp Leu Asn Gly Lys Glu Tyr Lys
210 215 220
Cys Lys Val Ser Asn Lys Ala Leu Pro Ala Pro Ile Glu Lys Thr Ile
225 230 235 240
Ser Lys Ala Lys Gly Gln Pro Arg Glu Pro Gln Val Tyr Thr Leu Pro
245 250 255
Pro Ser Arg Asp Glu Leu Thr Lys Asn Gln Val Ser Leu Thr Cys Leu
260 265 270
Val Lys Gly Phe Tyr Pro Ser Asp Ile Ala Val Glu Trp Glu Ser Asn
275 280 285
Gly Gln Pro Glu Asn Asn Tyr Lys Thr Thr Pro Pro Val Leu Asp Ser
290 295 300
Asp Gly Ser Phe Phe Leu Tyr Ser Lys Leu Thr Val Asp Lys Ser Arg
305 310 315 320
Trp Gln Gln Gly Asn Val Phe Ser Cys Ser Val Met His Glu Ala Leu
325 330 335
His Asn His Tyr Thr Gln Lys Ser Leu Ser Leu Ser Pro Gly Lys
340 345 350

<210> 86
<211> 348
<212> PRT
<213> (Artificial Sequence)

<220>
<221> PEPTIDE
<223> S58-Fc

<400> 86
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 87
<211> 447
<212> PRT
<213> (Artificial Sequence)

<220>
<221> PEPTIDE
<223> Hu167-IgG4 AA

<400> 87
Gln Val Gln Leu Val Gln Ser Gly Ala Glu Val Lys Lys Pro Gly Ala

```
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Asn Ile Asp Pro Ser Asp Ser Glu Thr His Tyr Asn Gln Lys Phe
    50                  55                  60
Lys Asp Arg Val Thr Met Thr Arg Asp Thr Ser Ile Ser Thr Ala Tyr
65                  70                  75                  80
Met Glu Leu Ser Arg Leu Arg Ser Asp Asp Thr Ala Val Tyr Tyr Cys
                85                  90                  95
Ala Arg Trp Gly Tyr Leu Gly Arg Ser Ala Met Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Ala Ala Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Lys
        435                 440                 445
```

<210> 88
<211> 214
<212> PRT
<213> (Artificial Sequence)

<220>
<221> PEPTIDE

<223> Hu167-IgG4 AA

<400> 88

Asp Val Gln Ile Thr Gln Ser Pro Ser Ser Leu Ser Ala Ser Val Gly
1 5 10 15
Asp Arg Val Thr Ile Thr Cys Arg Thr Ser Lys Ser Ile Ser Lys Phe
20 25 30
Leu Ala Trp Tyr Gln Gln Lys Pro Gly Lys Ala Pro Lys Leu Leu Ile
35 40 45
Tyr Ser Gly Ser Thr Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln His Asn Glu Tyr Pro Trp
85 90 95
Thr Phe Gly Gly Gly Thr Lys Val Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 89
<211> 5
<212> PRT
<213> (Artificial Sequence)

<220>
<221> DOMAIN
<223> GGGGS

<400> 89

Gly Gly Gly Gly Ser
1 5

<210> 90
<211> 16
<212> PRT
<213> (Artificial Sequence)

<220>
<221> DOMAIN
<223> GS16

<400> 90

Gly Gly Gly Gly Ser Gly Gly Gly Gly Ser Gly Gly Gly Gly Gly Gly
1 5 10 15

<210> 91
<211> 17

<212> PRT
<213> (Artificial Sequence)

<220>
<221> DOMAIN
<223> GS17

<400> 91
Gly Gly Gly Gly Ser Gly Gly Gly Gly Ser Gly Gly Gly Gly Ser Gly
1 5 10 15
Gly

<210> 92
<211> 18
<212> PRT
<213> (Artificial Sequence)

<220>
<221> DOMAIN
<223> GGGGSGGGGSGGGGSGGG

<400> 92
Gly Gly Gly Gly Ser Gly Gly Gly Gly Ser Gly Gly Gly Gly Ser Gly
1 5 10 15
Gly Gly

<210> 93
<211> 19
<212> PRT
<213> (Artificial Sequence)

<220>
<221> DOMAIN
<223> GS19

<400> 93
Gly Gly Gly Gly Ser Gly Gly Gly Gly Ser Gly Gly Gly Gly Ser Gly
1 5 10 15
Gly Gly Gly

<210> 94
<211> 30
<212> PRT
<213> (Artificial Sequence)

<220>
<221> DOMAIN
<223> 30AA-linker

<400> 94
Asp Pro Ala Leu Val His Gln Arg Pro Ala Pro Pro Gly Gly Gly Gly
1 5 10 15
Ser Gly Gly Gly Gly Ser Gly Gly Gly Gly Ser Gly Gly Gly
20 25 30

<210> 95

<211> 5
<212> PRT
<213> (Artificial Sequence)

<220>
<221> DOMAIN
<223> GKPGS

<400> 95
Gly Lys Pro Gly Ser
1 5

<210> 96
<211> 5
<212> PRT
<213> (Artificial Sequence)

<220>
<221> DOMAIN
<223> GGGES

<400> 96
Gly Gly Gly Glu Ser
1 5

<210> 97
<211> 7
<212> PRT
<213> (Artificial Sequence)

<220>
<221> DOMAIN
<223> 9-2 H5L11 HCDR1

<400> 97
Asp Gly Ser Ala Tyr Trp Ser
1 5

<210> 98
<211> 16
<212> PRT
<213> (Artificial Sequence)

<220>
<221> DOMAIN
<223> 9-2 H5L11 HCDR2

<400> 98
Phe Ile Ser Arg Ala Gly Ser Thr Tyr Asn Thr Pro Ser Leu Lys Gly
1 5 10 15

<210> 99
<211> 10
<212> PRT
<213> (Artificial Sequence)

<220>
<221> DOMAIN

<223> 9-2 H5L11 HCDR3

<400> 99
Ser Gly Gly Trp Leu Ala Pro Phe Asp Tyr
1 5 10

<210> 100
<211> 17
<212> PRT
<213> (Artificial Sequence)

<220>
<221> DOMAIN
<223> 9-2 H5L11 LCDR1

<400> 100
Lys Ser Ser Gln Ser Leu Phe Tyr His Ser Asn Gln Lys His Ser Leu
1 5 10 15
Ala

<210> 101
<211> 7
<212> PRT
<213> (Artificial Sequence)

<220>
<221> DOMAIN
<223> 9-2 H5L11 LCDR2

<400> 101
Gly Ala Ser Thr Arg Glu Ser
1 5

<210> 102
<211> 9
<212> PRT
<213> (Artificial Sequence)

<220>
<221> DOMAIN
<223> 9-2 H5L11 LCDR3

<400> 102
Gln Gln Tyr Tyr Gly Tyr Pro Tyr Thr
1 5

<210> 103
<211> 5
<212> PRT
<213> (Artificial Sequence)

<220>
<221> DOMAIN
<223> 24D5 H12L61 HCDR1

<400> 103
Ser Tyr Trp Met His

1 5

<210> 104
<211> 17
<212> PRT
<213> (Artificial Sequence)

<220>
<221> DOMAIN
<223> 24D5 H12L61 HCDR2

<400> 104
Arg Ile Thr Pro Ser Ser Gly Phe Ala Met Tyr Asn Glu Lys Phe Lys
1 5 10 15
Asn

<210> 105
<211> 10
<212> PRT
<213> (Artificial Sequence)

<220>
<221> DOMAIN
<223> 24D5 H12L61 HCDR3

<400> 105
Gly Gly Ser Ser Tyr Asp Tyr Phe Asp Tyr
1 5 10

<210> 106
<211> 15
<212> PRT
<213> (Artificial Sequence)

<220>
<221> DOMAIN
<223> 24D5 H12L61 LCDR1

<400> 106
Arg Ala Ser Glu Ser Val Ser Ile His Gly Thr His Leu Met His
1 5 10 15

<210> 107
<211> 7
<212> PRT
<213> (Artificial Sequence)

<220>
<221> DOMAIN
<223> 24D5 H12L61 LCDR2

<400> 107
Ala Ala Ser Asn Leu Glu Ser
1 5

<210> 108
<211> 9
<212> PRT

<213> (Artificial Sequence)

<220>
<221> DOMAIN
<223> 24D5 H12L61 LCDR3

<400> 108
Gln Gln Ser Phe Glu Asp Pro Leu Thr
1 5

<210> 109
<211> 583
<212> PRT
<213> (Artificial Sequence)

<220>
<221> CHAIN
<223> h1831-19-S37

<400> 109
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Gly Leu Glu Trp Met
35 40 45
Gly Arg Ile Thr Pro Ser Ser Gly Phe Ala Met Tyr Asn Glu Lys Phe
50 55 60
Lys Asn Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Gly Gly Ser Ser Tyr Asp Tyr Phe Asp Tyr Trp Gly Gln Gly
100 105 110
Thr Thr Val Thr Val Ser Ser Ala Ser Thr Lys Gly Pro Ser Val Phe
115 120 125
Pro Leu Ala Pro Cys Ser Arg Ser Thr Ser Glu Ser Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Lys Thr Tyr Thr Cys Asn Val Asp His Lys Pro
195 200 205
Ser Asn Thr Lys Val Asp Lys Arg Val Glu Ser Lys Tyr Gly Pro Pro
210 215 220
Cys Pro Pro Cys Pro Ala Pro Glu Phe Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser Gln Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Tyr Arg Val Val Ser Val
290 295 300
Leu Thr Val Leu His Gln Asp Trp Leu Asn Gly Lys Glu Tyr Lys Cys
305 310 315 320

Lys Val Ser Asn Lys Gly Leu Pro Ser Ser Ile Glu Lys Thr Ile Ser
325 330 335
Lys Ala Lys Gly Gln Pro Arg Glu Pro Gln Val Tyr Thr Leu Pro Pro
340 345 350
Ser Gln Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Arg Leu Thr Val Asp Lys Ser Arg Trp
405 410 415
Gln Glu Gly Asn Val Phe Ser Cys Ser Val Met His Glu Ala Leu His
420 425 430
Asn His Tyr Thr Gln Lys Ser Leu Ser Leu Ser Leu Gly Gly Gly Gly
435 440 445
Gly Ser Gly Gly Gly Gly Ser Gly Gly Gly Gly Ser Gly Gly Gly Gly
450 455 460
Glu Glu Glu Leu Gln Met Ile Gln Pro Glu Lys Leu Leu Leu Val Thr
465 470 475 480
Val Gly Glu Thr Ala Thr Leu His Cys Thr Val Thr Ser Leu Leu Pro
485 490 495
Val Gly Pro Val Leu Trp Phe Arg Gly Val Gly Pro Gly Arg Glu Leu
500 505 510
Ile Tyr Met Ser Gly Arg Gly His Phe Pro Arg Val Thr Thr Val Ser
515 520 525
Asp Leu Thr Lys Arg Asn Asn Lys Asp Phe Ser Ile Arg Ile Ser Ser
530 535 540
Ile Thr Pro Ala Asp Val Gly Thr Tyr Tyr Cys Val Lys Phe Arg Lys
545 550 555 560
Gly Ser Pro Glu Asp Val Glu Phe Lys Ser Gly Pro Gly Thr Glu Met
565 570 575
Ala Leu Gly Ala Lys Pro Ser
580

<210> 110
<211> 348
<212> PRT
<213> (Artificial Sequence)

<220>
<221> PEPTIDE
<223> S37-Fc

<400> 110
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asn Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe

130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 111
<211> 218
<212> PRT
<213> (Artificial Sequence)

<220>
<221> CHAIN
<223> h1831K

<400> 111
Asp Ile Val Leu Thr Gln Ser Pro Ala Ser Leu Ala Val Ser Pro Gly
1 5 10 15
Gln Arg Ala Thr Ile Thr Cys Arg Ala Ser Glu Ser Val Ser Ile His
20 25 30
Gly Thr His Leu Met His Trp Tyr Gln Gln Lys Pro Gly Gln Pro Pro
35 40 45
Lys Leu Leu Ile Tyr Ala Ala Ser Lys Leu Glu Ser Gly Val Pro Ala
50 55 60
Arg Phe Ser Gly Ser Gly Ser Gly Thr Asp Phe Thr Leu Thr Ile Asn
65 70 75 80
Pro Val Glu Ala Glu Asp Thr Ala Asn Tyr Tyr Cys Gln Gln Ser Phe
85 90 95
Glu Asp Pro Leu Thr Phe Gly Gln Gly Thr Lys Leu Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190

His Lys Val Tyr Ala Cys Glu Val Thr His Gln Gly Leu Ser Ser Pro
195 200 205
Val Thr Lys Ser Phe Asn Arg Gly Glu Cys
210 215

<210> 112
<211> 7
<212> PRT
<213> (Artificial Sequence)

<220>
<221> DOMAIN
<223> h1831K LCDR2

<400> 112
Ala Ala Ser Lys Leu Glu Ser
1 5

<210> 113
<211> 111
<212> PRT
<213> (Artificial Sequence)

<220>
<221> DOMAIN
<223> h1831K

<400> 113
Asp Ile Val Leu Thr Gln Ser Pro Ala Ser Leu Ala Val Ser Pro Gly
1 5 10 15
Gln Arg Ala Thr Ile Thr Cys Arg Ala Ser Glu Ser Val Ser Ile His
20 25 30
Gly Thr His Leu Met His Trp Tyr Gln Gln Lys Pro Gly Gln Pro Pro
35 40 45
Lys Leu Leu Ile Tyr Ala Ala Ser Lys Leu Glu Ser Gly Val Pro Ala
50 55 60
Arg Phe Ser Gly Ser Gly Ser Gly Thr Asp Phe Thr Leu Thr Ile Asn
65 70 75 80
Pro Val Glu Ala Glu Asp Thr Ala Asn Tyr Tyr Cys Gln Gln Ser Phe
85 90 95
Glu Asp Pro Leu Thr Phe Gly Gln Gly Thr Lys Leu Glu Ile Lys
100 105 110

<210> 114
<211> 348
<212> PRT
<213> (Artificial Sequence)

<220>
<221> PEPTIDE
<223> S79-Fc

<400> 114
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Trp Pro
20 25 30
Val Gly Pro Val Leu Trp Phe Arg Gly Val Gly Pro Gly Arg Glu Leu
35 40 45
Ile Tyr Arg Thr Gly Thr Gly Arg Phe Pro Arg Val Thr Thr Val Ser

50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Val
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 115
<211> 348
<212> PRT
<213> (Artificial Sequence)

<220>
<221> PEPTIDE
<223> S15-Fc

<400> 115
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Val Ser Gly Arg Gly His Phe Pro Arg Val Thr Thr Val Ser
50 55 60
Asp Leu Thr Lys Arg Glu Asn Arg Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110

Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 116
<211> 348
<212> PRT
<213> (Artificial Sequence)

<220>
<221> PEPTIDE
<223> S12-Fc

<400> 116
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Val Ser Gly Arg Gly His Phe Pro Arg Val Thr Thr Val Ser
50 55 60
Asp Leu Thr Lys Arg Glu Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe

```
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345
```

```
<210>  117
<211>  348
<212>  PRT
<213>  (Artificial Sequence)

<220>
<221>  PEPTIDE
<223>  S19-Fc


<400>  117
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Val Ser Gly Arg Gly His Phe Pro Arg Val Thr Thr Val Ser
    50                  55                  60
Asp Leu Thr Lys Arg Asn Asn Arg Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
```

```
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345

<210>  118
<211>  348
<212>  PRT
<213>  (Artificial Sequence)

<220>
<221>  PEPTIDE
<223>  S85-Fc


<400>  118
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Val Ser Gly Arg Gly His Phe Pro Arg Val Thr Thr Val Ser
    50                  55                  60
Asp Leu Thr Lys Arg Asn Asn Lys Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
```

```
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345


<210>  119
<211>  348
<212>  PRT
<213>  (Artificial Sequence)

<220>
<221>  PEPTIDE
<223>  S38-Fc


<400>  119
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Phe Ser Gly Arg Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
```

Tyr Thr Gln Lys Ser Leu Ser Leu Ser Leu Gly Lys
340 345

<210> 120
<211> 348
<212> PRT
<213> (Artificial Sequence)

<220>
<221> PEPTIDE
<223> S22-Fc

<400> 120
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Ile Ser Gly Arg Gly His Phe Pro Arg Val Thr Thr Val Ser
50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 121
<211> 348
<212> PRT
<213> (Artificial Sequence)

<220>
<221> PEPTIDE
<223> S29-Fc

<400> 121
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Leu Ser Gly Arg Gly His Phe Pro Arg Val Thr Thr Val Ser
50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 122
<211> 348
<212> PRT
<213> (Artificial Sequence)

<220>
<221> PEPTIDE
<223> S34-Fc

<400> 122

Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Arg Ser Gly Arg Gly His Phe Pro Arg Val Thr Thr Val Ser
    50                  55                  60
Asp Leu Thr Lys Arg Asn Asn Met Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345

<210> 123
<211> 348
<212> PRT
<213> (Artificial Sequence)

<220>
<221> PEPTIDE
<223> S41-Fc

<400> 123
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Val Ser Gly Arg Gly His Phe Pro Arg Val Thr Thr Val Ser

50 55 60
Asp Leu Thr Lys Arg Asn Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 124
<211> 348
<212> PRT
<213> (Artificial Sequence)

<220>
<221> PEPTIDE
<223> S42-Fc

<400> 124
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asn Asn Ile Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110

```
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345

<210>  125
<211>  348
<212>  PRT
<213>  (Artificial Sequence)

<220>
<221>  PEPTIDE
<223>  S43-Fc


<400>  125
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Met Ser Gly Arg Gly His Phe Pro Arg Val Thr Thr Val Ser
    50                  55                  60
Asp Leu Thr Lys Arg Asn Asn Arg Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
```

```
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345
```

<210> 126
<211> 348
<212> PRT
<213> (Artificial Sequence)

<220>
<221> PEPTIDE
<223> S44-Fc

<400> 126

```
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Met Ser Gly Arg Gly His Phe Pro Arg Val Thr Thr Val Ser
    50                  55                  60
Asp Leu Thr Lys Arg Asn Asn Val Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
```

Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 127
<211> 348
<212> PRT
<213> (Artificial Sequence)

<220>
<221> PEPTIDE
<223> S45-Fc

<400> 127
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Met Ser Gly Arg Gly His Phe Pro Arg Val Thr Thr Val Ser
50 55 60
Asp Leu Thr Lys Arg Asp Asn Met Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro

```
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345

<210>  128
<211>  348
<212>  PRT
<213>  (Artificial Sequence)

<220>
<221>  PEPTIDE
<223>  S46-Fc


<400>  128
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Met Ser Gly Arg Gly His Phe Pro Arg Val Thr Thr Val Ser
    50                  55                  60
Asp Leu Thr Lys Arg Glu Asn Lys Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
```

```
Tyr Thr Gln Lys Ser Leu Ser Leu Ser Leu Gly Lys
            340                 345

<210>  129
<211>  348
<212>  PRT
<213>  (Artificial Sequence)

<220>
<221>  PEPTIDE
<223>  S47-Fc


<400>  129
Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Met Ser Gly Arg Gly His Phe Pro Arg Val Thr Thr Val Ser
    50                  55                  60
Asp Leu Thr Lys Arg Glu Asn Lys Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Val
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345

<210>  130
<211>  348
<212>  PRT
<213>  (Artificial Sequence)
```

<220>
<221> PEPTIDE
<223> S48-Fc

<400> 130
Glu Glu Glu Leu Gln Met Ile Gln Pro Glu Lys Leu Leu Leu Val Thr
1 5 10 15
Val Gly Glu Thr Ala Thr Leu His Cys Thr Val Thr Ser Leu Leu Pro
20 25 30
Val Gly Pro Val Leu Trp Phe Arg Gly Val Gly Pro Gly Arg Glu Leu
35 40 45
Ile Tyr Arg Ser Gly Arg Gly His Phe Pro Arg Val Thr Thr Val Ser
50 55 60
Asp Leu Thr Lys Arg Asn Asn Lys Asp Phe Ser Ile Arg Ile Ser Ser
65 70 75 80
Ile Thr Pro Ala Asp Val Gly Thr Tyr Tyr Cys Val Lys Phe Arg Lys
85 90 95
Gly Ser Pro Glu Asp Val Glu Phe Lys Ser Gly Pro Gly Thr Glu Met
100 105 110
Ala Leu Gly Ala Lys Pro Ser Glu Ser Lys Tyr Gly Pro Pro Cys Pro
115 120 125
Pro Cys Pro Ala Pro Glu Phe Leu Gly Gly Pro Ser Val Phe Leu Phe
130 135 140
Pro Pro Lys Pro Lys Asp Thr Leu Met Ile Ser Arg Thr Pro Glu Val
145 150 155 160
Thr Cys Val Val Val Asp Val Ser Gln Glu Asp Pro Glu Val Gln Phe
165 170 175
Asn Trp Tyr Val Asp Gly Val Glu Val His Asn Ala Lys Thr Lys Pro
180 185 190
Arg Glu Glu Gln Phe Asn Ser Thr Tyr Arg Val Val Ser Val Leu Thr
195 200 205
Val Leu His Gln Asp Trp Leu Asn Gly Lys Glu Tyr Lys Cys Lys Val
210 215 220
Ser Asn Lys Gly Leu Pro Ser Ser Ile Glu Lys Thr Ile Ser Lys Ala
225 230 235 240
Lys Gly Gln Pro Arg Glu Pro Gln Val Tyr Thr Leu Pro Pro Ser Gln
245 250 255
Glu Glu Met Thr Lys Asn Gln Val Ser Leu Thr Cys Leu Val Lys Gly
260 265 270
Phe Tyr Pro Ser Asp Ile Ala Val Glu Trp Glu Ser Asn Gly Gln Pro
275 280 285
Glu Asn Asn Tyr Lys Thr Thr Pro Pro Val Leu Asp Ser Asp Gly Ser
290 295 300
Phe Phe Leu Tyr Ser Arg Leu Thr Val Asp Lys Ser Arg Trp Gln Glu
305 310 315 320
Gly Asn Val Phe Ser Cys Ser Val Met His Glu Ala Leu His Asn His
325 330 335
Tyr Thr Gln Lys Ser Leu Ser Leu Ser Leu Gly Lys
340 345

<210> 131
<211> 348
<212> PRT
<213> (Artificial Sequence)

<220>
<221> PEPTIDE
<223> S49-Fc

<400> 131

Glu Glu Glu Leu Gln Met Ile Gln Pro Glu Lys Leu Leu Leu Val Thr
1               5                   10                  15
Val Gly Glu Thr Ala Thr Leu His Cys Thr Val Thr Ser Leu Leu Pro
            20                  25                  30
Val Gly Pro Val Leu Trp Phe Arg Gly Val Gly Pro Gly Arg Glu Leu
        35                  40                  45
Ile Tyr Arg Ser Gly Arg Gly His Phe Pro Arg Val Thr Thr Val Ser
    50                  55                  60
Asp Leu Thr Lys Arg Glu Asn Lys Asp Phe Ser Ile Arg Ile Ser Ser
65                  70                  75                  80
Ile Thr Pro Ala Asp Val Gly Thr Tyr Tyr Cys Val Lys Phe Arg Lys
                85                  90                  95
Gly Ser Pro Glu Asp Val Glu Phe Lys Ser Gly Pro Gly Thr Glu Met
            100                 105                 110
Ala Leu Gly Ala Lys Pro Ser Glu Ser Lys Tyr Gly Pro Pro Cys Pro
        115                 120                 125
Pro Cys Pro Ala Pro Glu Phe Leu Gly Gly Pro Ser Val Phe Leu Phe
    130                 135                 140
Pro Pro Lys Pro Lys Asp Thr Leu Met Ile Ser Arg Thr Pro Glu Val
145                 150                 155                 160
Thr Cys Val Val Val Asp Val Ser Gln Glu Asp Pro Glu Val Gln Phe
                165                 170                 175
Asn Trp Tyr Val Asp Gly Val Glu Val His Asn Ala Lys Thr Lys Pro
            180                 185                 190
Arg Glu Glu Gln Phe Asn Ser Thr Tyr Arg Val Val Ser Val Leu Thr
        195                 200                 205
Val Leu His Gln Asp Trp Leu Asn Gly Lys Glu Tyr Lys Cys Lys Val
    210                 215                 220
Ser Asn Lys Gly Leu Pro Ser Ser Ile Glu Lys Thr Ile Ser Lys Ala
225                 230                 235                 240
Lys Gly Gln Pro Arg Glu Pro Gln Val Tyr Thr Leu Pro Pro Ser Gln
                245                 250                 255
Glu Glu Met Thr Lys Asn Gln Val Ser Leu Thr Cys Leu Val Lys Gly
            260                 265                 270
Phe Tyr Pro Ser Asp Ile Ala Val Glu Trp Glu Ser Asn Gly Gln Pro
        275                 280                 285
Glu Asn Asn Tyr Lys Thr Thr Pro Pro Val Leu Asp Ser Asp Gly Ser
    290                 295                 300
Phe Phe Leu Tyr Ser Arg Leu Thr Val Asp Lys Ser Arg Trp Gln Glu
305                 310                 315                 320
Gly Asn Val Phe Ser Cys Ser Val Met His Glu Ala Leu His Asn His
                325                 330                 335
Tyr Thr Gln Lys Ser Leu Ser Leu Ser Leu Gly Lys
            340                 345

<210> 132
<211> 124
<212> PRT
<213> Artificial Sequence

<220>
<221> PEPTIDE
<223> HisSIRP

<400> 132
Glu Glu Glu Leu Gln Val Ile Gln Pro Asp Lys Ser Val Ser Val Ala
1               5                   10                  15
Ala Gly Glu Ser Ala Ile Leu His Cys Thr Val Thr Ser Leu Ile Pro
            20                  25                  30
Val Gly Pro Ile Gln Trp Phe Arg Gly Ala Gly Pro Ala Arg Glu Leu
        35                  40                  45

Ile Tyr Asn Gln Lys Glu Gly His Phe Pro Arg Val Thr Thr Val Ser
    50                  55                  60
Glu Ser Thr Lys Arg Glu Asn Met Asp Phe Ser Ile Ser Ile Ser Asn
65                  70                  75                  80
Ile Thr Pro Ala Asp Ala Gly Thr Tyr Tyr Cys Val Lys Phe Arg Lys
                85                  90                  95
Gly Ser Pro Asp Thr Glu Phe Lys Ser Gly Ala Gly Thr Glu Leu Ser
            100                 105                 110
Val Arg Ala Lys Pro Ser His His His His His His
        115                 120

<210> 133
<211> 345
<212> PRT
<213> (Artificial Sequence)

<220>
<221> PEPTIDE
<223> TTI-621

<400> 133
Glu Glu Glu Leu Gln Val Ile Gln Pro Asp Lys Ser Val Ser Val Ala
1               5                   10                  15
Ala Gly Glu Ser Ala Ile Leu His Cys Thr Val Thr Ser Leu Ile Pro
            20                  25                  30
Val Gly Pro Ile Gln Trp Phe Arg Gly Ala Gly Pro Ala Arg Glu Leu
        35                  40                  45
Ile Tyr Asn Gln Lys Glu Gly His Phe Pro Arg Val Thr Thr Val Ser
    50                  55                  60
Glu Ser Thr Lys Arg Glu Asn Met Asp Phe Ser Ile Ser Ile Ser Asn
65                  70                  75                  80
Ile Thr Pro Ala Asp Ala Gly Thr Tyr Tyr Cys Val Lys Phe Arg Lys
                85                  90                  95
Gly Ser Pro Asp Thr Glu Phe Lys Ser Gly Ala Gly Thr Glu Leu Ser
            100                 105                 110
Val Arg Ala Lys Pro Ser Asp Lys Thr His Thr Cys Pro Pro Cys Pro
        115                 120                 125
Ala Pro Glu Leu Leu Gly Gly Pro Ser Val Phe Leu Phe Pro Pro Lys
    130                 135                 140
Pro Lys Asp Thr Leu Met Ile Ser Arg Thr Pro Glu Val Thr Cys Val
145                 150                 155                 160
Val Val Asp Val Ser His Glu Asp Pro Glu Val Lys Phe Asn Trp Tyr
                165                 170                 175
Val Asp Gly Val Glu Val His Asn Ala Lys Thr Lys Pro Arg Glu Glu
            180                 185                 190
Gln Tyr Asn Ser Thr Tyr Arg Val Val Ser Val Leu Thr Val Leu His
        195                 200                 205
Gln Asp Trp Leu Asn Gly Lys Glu Tyr Lys Cys Lys Val Ser Asn Lys
    210                 215                 220
Ala Leu Pro Ala Pro Ile Glu Lys Thr Ile Ser Lys Ala Lys Gly Gln
225                 230                 235                 240
Pro Arg Glu Pro Gln Val Tyr Thr Leu Pro Pro Ser Arg Asp Glu Leu
                245                 250                 255
Thr Lys Asn Gln Val Ser Leu Thr Cys Leu Val Lys Gly Phe Tyr Pro
            260                 265                 270
Ser Asp Ile Ala Val Glu Trp Glu Ser Asn Gly Gln Pro Glu Asn Asn
        275                 280                 285
Tyr Lys Thr Thr Pro Pro Val Leu Asp Ser Asp Gly Ser Phe Phe Leu
    290                 295                 300
Tyr Ser Lys Leu Thr Val Asp Lys Ser Arg Trp Gln Gln Gly Asn Val
305                 310                 315                 320
Phe Ser Cys Ser Val Met His Glu Ala Leu His Asn His Tyr Thr Gln

```
                    325                 330                 335
Lys Ser Leu Ser Leu Ser Pro Gly Lys
            340                 345
```

## Description

## TECHNICAL FIELD

The present disclosure relates to a bifunctional fusion protein that specifically binds PD-L1 and CD47, a pharmaceutical composition comprising the bifunctional fusion protein, and use thereof as an anticancer agent.

## BACKGROUND OF THE INVENTION

The statements here only provide background information related to the present disclosure and do not necessarily constitute the prior art.

Programmed death-1 (PD-1) is a protein receptor expressed on the surface of T cells discovered in 1992, which participates in the process of cell apoptosis. PD-1 belongs to the CD28 family and has 23% amino acid homology with cytotoxic T lymphocyte antigen 4 (CTLA-4). However, the expression of PD-1 is different from that of CTLA-4, mainly on activated T cells, B cells and myeloid cells. PD-1 has two ligands, PD-L1 and PD-L2, respectively. PD-L1 is mainly expressed on T cells, B cells, macrophages and dendritic cells (DCs), and the expression on cells can be up-regulated upon activation. The expression of PD-L2 is relatively limited, mainly on antigen-presenting cells, such as activated macrophages and dendritic cells.

New research has found that high expression of PD-L1 protein is detected in human tumor tissues, such as breast cancer, lung cancer, stomach cancer, bowel cancer, kidney cancer, melanoma, and the expression level of PD-L1 is closely related to the clinic and prognosis of patients. Since PD-L1 plays the role of the second signal pathway to inhibit T cell proliferation, blocking the binding of PD-L1/PD-1 has become a very potential emerging target in the field of tumor immunotherapy.

The cell surface protein CD47 is expressed or overexpressed on many tumor types, including acute myeloid leukemia, various subtypes of B-cell non-Hodgkin's lymphoma, and many human solid tumor cells. Binding of CD47 to signal regulatory protein α (SIRPα) on macrophages is a "do not eat me" signal on the surface of tumor cells. Recent data indicate that anti-CD47 antibodies also help to improve the effective anti-tumor T cell response in immune-tolerant mice. Therefore, anti-CD47 antibodies are a new class of immune checkpoint inhibitors that regulate the innate immune system and the adaptive immune system.

Currently, there have been related CD47 patents, such as WO2016065329, WO2016109415, WO2014087248, WO2014093678, CN107849143A, CN108350048, CN106535914, WO2016023001A, CN107459578A, CN2017110167989, etc. For example, WO2016023001A describes a multispecific PD-1 mimetic peptide comprising a high-affinity PD-1 mimetic peptide and a high-affinity SIRP-α that specifically binds to CD47, and use thereof; CN107459578A describes a recombinant fusion protein comprising a SIRPα mutant and an anti-PD-L1 antibody that targets CD47 and PD-L1 molecules; CN201711016798.9 discloses a multifunctional fusion protein comprising the extracellular part of SIRPα and the extracellular part of PD-1.

However, many therapies in current preclinical and clinical studies are directed at the CD47/SIRPα interaction, including anti-CD47 antibody, SIRPα receptor protein and engineered SIRPα receptor protein, anti-SIRPa antibody and bispecific antibody, etc., and there is no related report on a multispecific fusion protein comprising a SIRPγ peptide.

SIRPγ is expressed on T cells and activated NK cells, and compared with SIRPα, SIRPγ binds CD47 with 10-fold lower affinity. The CD47-SIRPγ interaction participates in the contact between antigen presenting cells and T cells, as well as co-stimulates T cell activation and promotes T cell proliferation (Piccio et al., Blood 2005, 105, 2421-2427). In addition, the CD47-SIRPγ interaction plays a role in the transendothelial migration of T cells (Stefanisakis et al., Blood 2008, 112, 1280-1289).

## SUMMARY OF THE INVENTION

The present disclosure provides a bifunctional fusion protein comprising a SIRPγ peptide variant. Compared with the wild-type SIRPγ peptide, the SIRPγ peptide variant has significantly improved affinity to CD47.

In some embodiments, provided is a bifunctional fusion protein comprising a SIRPγ peptide variant and an anti-human PD-L1 antibody, the SIRPγ peptide variant being linked to the polypeptide chain of the anti-human PD-L1 antibody, the SIRPγ peptide variant is a SIRPγ peptide variant with a substitution mutation at position N51 relative to the wild-type SIRPγ peptide as shown in SEQ ID NO: 20. In some embodiments, the aforementioned SIRPγ peptide variant has the activity of binding to CD47 on the surface of tumor cells. Preferably, the SIRPγ peptide variant has more enhanced activity of binding to CD47 on the surface of tumor cells than the wild-type SIRPγ peptide.

In some embodiments, provided is a bifunctional fusion protein comprising a human SIRPγ peptide variant and an anti-human PD-L1 antibody, the SIRPγ peptide variant being linked to the polypeptide chain of the anti-human PD-L1 antibody,

wherein the SIRPγ peptide variant is a SIRPγ peptide variant with a substitution mutation at position N51 relative to the wild-type SIRPγ peptide as shown in SEQ ID NO: 20. In some embodiments, the aforementioned SIRPγ peptide variant has the activity of binding to CD47 on the surface of tumor cells. Preferably, the SIRPγ peptide variant has more enhanced activity of binding to CD47 on the surface of tumor cells than the wild-type SIRPγ peptide. In some embodiments, the aforementioned bifunctional fusion protein, wherein the SIRPγ peptide variant and the polypeptide chain of the anti-human PD-L1 antibody are directly linked by a peptide bond or covalently linked through a linker.

Preferably, the linker can be selected from any one of the linkers shown in the group consisiting of SEQ ID NO: 89-96, (GGGGS)n, (GGGES)n and (GKPGS)n, wherein n is an integer of 2 to 7.

In some embodiments, the aforementioned bifunctional fusion protein, wherein the carboxyl terminal of the SIRPγ peptide variant is linked to the amino terminal of the heavy chain variable region of the anti-human PD-L1 antibody,

or the carboxyl terminal of the SIRPγ peptide variant is linked to the amino terminal of the light chain variable region of the anti-human PD-L1 antibody,
or the carboxyl terminal of the heavy chain of the anti-human PD-L1 antibody is linked to the amino terminal of the SIRPγ peptide variant,
or the carboxyl terminal of the light chain of the anti-human PD-L1 antibody is linked to the amino terminal of the SIRPγ peptide variant.

In some preferred embodiments, the aforementioned bifunctional fusion protein, wherein the SIRPγ peptide variant is a SIRPγ peptide variant further comprising amino acid substitution(s) at one or more positions selected from the group consisiting of K19, K53, N101, L31, Q52, E54, H56, N70, M72 and M112 relative to the wild-type SIRPγ peptide.

In some preferred embodiments, the aforementioned bifunctional fusion protein, wherein the SIRPγ peptide variant is a SIRPγ peptide variant further comprising amino acid substitutions at one or more positions selected from K19, K53 and N101 relative to the wild-type SIRPγ peptide.

In some preferred embodiments, the aforementioned bifunctional fusion protein, wherein the SIRPγ peptide variant is a SIRPγ peptide variant with a N51R substitution mutation relative to the wild-type SIRPγ peptide as shown in SEQ ID NO: 20.

In some preferred embodiments, the aforementioned bifunctional fusion protein, wherein the SIRPγ peptide variant with a substitution mutation at position N51 does not substantially bind to CD47 on the surface of red blood cells, preferably, the SIRPγ peptide variant with a substitution mutation at position N51 is a SIRPγ peptide variant comprising a N51F, N51I, N51L, N51M or N51V substitution mutation.

In some preferred embodiments, the aforementioned bifunctional fusion protein, wherein the SIRPγ peptide variant is a SIRPγ peptide variant further comprising K19E, K53G and N101D substitution mutations relative to the wild-type SIRPγ peptide as shown in SEQ ID NO: 20.

In some preferred embodiments, the aforementioned bifunctional fusion protein, wherein the SIRPγ peptide variant further comprises K19E, N51V, Q52S, K53G, E54R, M72K and N101D mutations relative to the wild-type SIRPγ peptide as shown in SEQ ID NO: 20.

In some preferred embodiments, the aforementioned bifunctional fusion protein, wherein the SIRPγ peptide variant further comprises K19E, N51M, Q52S, K53G, E54R, M72K and N101D mutations relative to the wild-type SIRPγ peptide as shown in SEQ ID NO: 20.

In some preferred embodiments, the aforementioned bifunctional fusion protein, wherein the SIRPγ peptide variant is a SIRPγ peptide variant further comprising amino acid substitutions at one or more positions selected from the group consisiting of M6, V27, L30, V33, V36, L37, V42, E47, L66, T67, V92 and S98.

In some preferred embodiments, the aforementioned bifunctional fusion protein, wherein the amino acid sequence of the SIRPγ peptide variant (general formula I) is as shown in SEQ ID NO: 1:

EEELQMIQPE KLLLVTVGET ATLHCTVTSL $X_1$PVGPVLWFR GVGPGRELIY $X_2X_3$G$X_4$G$X_5$FPRV TTVSDLTKR$X_6$ NX7DFSIRISS ITPADVGTYY CVKFRKGSPE DVEFKSGPGT E$X_8$ALGAKPS (SEQ ID NO: 1)

wherein, $X_1$ is selected from L or W, $X_2$ is selected from M, V, F, I or L, $X_3$ is selected from Q, S or T, $X_4$ is selected from E, T or R, $X_5$ is selected from H or R, $X_6$ is selected from D, N or E, $X_7$ is selected from I, V, M, R or K, and $X_8$ is selected from M or V.

In some embodiments, the aforementioned bifunctional fusion protein, the amino acid sequence of the SIRPγ peptide variant (general formula II) is as shown in SEQ ID NO: 2:

EEELQMIQPE KLLLVTVGET ATLHCTVTSL $X_1$PVGPVLWFR GVGPGRELIY R$X_3$G$X_4$G$X_5$FPRV TTVS-DLTK.R$X_6$ N$X_7$DFSIRISS ITPADVGTYY CVKFRKGSPE DVEFKSGPGT E$X_8$ALGAKPS (SEQ ID NO: 2)

wherein, $X_1$ is selected from L or W, $X_3$ is selected from Q, S or T, $X_4$ is selected from E, T or R, $X_5$ is selected from H or R, $X_6$ is selected from D, N or E, $X_7$ is selected from I, V, M, R or K, and $X_8$ is selected from M or V

In further preferred embodiments, the aforementioned bifunctional fusion protein, wherein the SIRPγ peptide variant is as shown in the group consisiting of SEQ ID NO: 21, 22, 23, 24, 25, 26, 27, 28, 29, 30, 31, 32, 33, 34, 35, 36, 37, 38, 39 and 40, preferably as shown in SEQ ID NO: 26 or 27.

In some embodiments, the aforementioned bifunctional fusion protein, wherein the anti-human PD-L1 antibody is selected from the group consisiting of Avelumab, Atezolizumab, Durvalumab, JS-003, CS-1001, LY-3300054, KD-033, CK-301, CCX-4503, CX-072, KN-035, HRP00052, HRP00049, FAZ-053, GR-1405, KD-005, HLX-20, KL-A167, CBT-502, STI-A1014, REMD-290, BGB-A333, BCD-135 and MCLA-145.

In some embodiments, the aforementioned bifunctional fusion protein, wherein the anti-human PD-L1 antibody comprises a heavy chain variable region and a light chain variable region, wherein:

the heavy chain variable region comprises HCDR1, HCDR2 and HCDR3 regions with the same sequence(s) as those in the heavy chain variable region as shown in SEQ ID NO: 6, and
the light chain variable region comprises LCDR1, LCDR2 and LCDR3 regions with the same sequence(s) as those in the light chain variable region as shown in SEQ ID NO: 7; or
the heavy chain variable region comprises HCDR1, HCDR2 and HCDR3 regions with the same sequence(s) as those in the heavy chain variable region as shown in SEQ ID NO: 8, and
the light chain variable region comprises LCDR1, LCDR2 and LCDR3 regions with the same sequence(s) as those in the light chain variable region as shown in SEQ ID NO: 9; or
the heavy chain variable region comprises HCDR1, HCDR2 and HCDR3 regions with the same sequence(s) as those in the heavy chain variable region as shown in SEQ ID NO: 8, and
the light chain variable region comprises LCDR1, LCDR2 and LCDR3 regions with the same sequence(s) as those in the light chain variable region as shown in SEQ ID NO: 113. Furthermore, in some embodiments, the HCDR1, HCDR2 and HCDR3 regions and the LCDR1, LCDR2 and LCDR3 regions are defined by the Kabat numbering criteria.

In some embodiments, the aforementioned bifunctional fusion protein, the heavy chain variable region of the anti-human PD-L1 antibody comprises the HCDR1, HCDR2, and HCDR3 regions as shown in SEQ ID NO: 97, 98 and 99, respectively, and the light chain variable region of the anti-human PD-L1 antibody comprises the LCDR1, LCDR2 and LCDR3 regions as shown in SEQ ID NO: 100, 101 and 102, respectively, or

the heavy chain variable region of the anti-human PD-L1 antibody comprises the HCDR1, HCDR2, and HCDR3 regions as shown in SEQ ID NO: 103, 104 and 105, respectively, and the light chain variable region of the anti-human PD-L1 antibody comprises the LCDR1, LCDR2 and LCDR3 regions as shown in SEQ ID NO: 106, 107 and 108, respectively;
or the heavy chain variable region of the anti-human PD-L1 antibody comprises the HCDR1, HCDR2, and HCDR3 regions as shown in SEQ ID NO: 103, 104 and 105, respectively, and the light chain variable region of the anti-human PD-L1 antibody comprises the LCDR1, LCDR2 and LCDR3 regions as shown in SEQ ID NO: 106, 112 and 108, respectively.

In some embodiments, the aforementioned bifunctional fusion protein, the anti-human PD-L1 antibody comprises a heavy chain variable region and a light chain variable region, wherein:

the heavy chain variable region is shown in SEQ ID NO: 6, and the light chain variable region is shown in SEQ ID NO: 7; or
the heavy chain variable region is shown in SEQ ID NO: 8, and the light chain variable region is shown in SEQ ID NO: 113;
or
the heavy chain variable region is shown in SEQ ID NO: 8, and the light chain variable region is shown in SEQ ID NO: 9.

In some embodiments, the aforementioned bifunctional fusion protein, wherein the anti-human PD-L1 antibody further comprises a heavy chain constant region and a light chain constant region, preferably, the heavy chain constant region is as shown in SEQ ID NO: 10 or 11, and the light chain constant region is as shown in SEQ ID NO: 12.

In some embodiments, the aforementioned bifunctional fusion protein, wherein the anti-human PD-L1 antibody comprises a heavy chain and a light chain, wherein: the heavy chain is as shown in SEQ ID NO: 13 or 15, and the light chain is as shown in SEQ ID NO: 14; or

the heavy chain is as shown in SEQ ID NO: 16 or 18, and the light chain is as shown in SEQ ID NO: 17; or
the heavy chain is as shown in SEQ ID NO: 16 or 18, and the light chain is as shown in SEQ ID NO: 111.

In some embodiments, the aforementioned bifunctional fusion protein, wherein the bifunctional fusion protein comprises a first polypeptide and a second polypeptide, wherein:

the first polypeptide is selected from the polypeptide as shown in any one of the group consisiting of SEQ ID NO: 41, 42, 43, 44, 45, 46, 47, 48, 49, 50, 51, 52, 53, 54, 55, 56, 57, 58, 59, 60, 61 and 62, and the second polypeptide is selected from the polypeptide as shown in SEQ ID NO: 14; or

the first polypeptide is selected from the polypeptide as shown in any one of the group consisiting of SEQ ID NO: 63, 64, 65, 66, 67, 68, 69, 70, 71, 72, 73, 74, 75, 76, 77, 78, 79, 80, 81, 82 and 109, and the second polypeptide is selected from the polypeptide as shown in SEQ ID NO: 17; or

the first polypeptide is selected from the polypeptide as shown in any one of the group consisiting of SEQ ID NO: 63, 64, 65, 66, 67, 68, 69, 70, 71, 72, 73, 74, 75, 76, 77, 78, 79, 80, 81, 82 and 109, and the second polypeptide is selected from the polypeptide as shown in SEQ ID NO: 111.

In some other embodiments of the present disclosure, provided is a SIRPγ peptide variant that is a SIRPγ peptide variant with a substitution mutation at position N51 relative to the wild-type SIRPγ peptide as shown in SEQ ID NO: 20. In some embodiments, the aforementioned SIRPγ peptide variant has the activity of binding with CD47 on the surface of tumor cells. Preferably, the SIRPγ peptide variant has more enhanced activity of binding with CD47 on the surface of tumor cells than the wild-type SIRPγ peptide.

In some preferred embodiments, the aforementioned SIRPγ peptide variant, wherein the SIRPγ peptide variant is a SIRPγ peptide variant with amino acid substitutions at one or more positions selected from the group consisiting of K19, K53, N101, L31, Q52, E54, H56, N70, M72 and M112 relative to the wild-type SIRPγ peptide.

In some preferred embodiments, the aforementioned SIRPγ peptide variant, wherein the SIRPγ peptide variant is a SIRPγ peptide variant further comprising amino acid substitutions at one or more positions selected from K19, K53 and N101 relative to the wild-type SIRPγ peptide.

In some preferred embodiments, the aforementioned SIRPγ peptide variant, wherein the SIRPγ peptide variant is a SIRPγ peptide variant with a N51R substitution mutation relative to the wild-type SIRPγ peptide as shown in SEQ ID NO: 20.

In some preferred embodiments, the aforementioned SIRPγ peptide variant, wherein the SIRPγ peptide variant with a substitution mutation at position N51 does not substantially bind to CD47 on the surface of red blood cells, preferably, the SIRPγ peptide variant with a substitution mutation at position N51 is a SIRPγ peptide variant comprising N51F, N51I, N51L, N51M or N51V substitution mutation.

In some preferred embodiments, the aforementioned SIRPγ peptide variant, wherein the SIRPγ peptide variant is a SIRPγ peptide variant comprising K19E, K53G and N101D substitution mutations relative to the wild-type SIRPγ as shown in SEQ ID NO: 20.

In some preferred embodiments, the aforementioned SIRPγ peptide variant, wherein the SIRPγ peptide variant comprises K19E, N51V, Q52S, K53G, E54R, M72K and N101D mutations relative to the wild-type SIRPγ peptide as shown in SEQ ID NO: 20.

In some preferred embodiments, the aforementioned SIRPγ peptide variant, wherein the SIRPγ peptide variant comprises K19E, N51M, Q52S, K53G, E54R, M72K and N101D mutations relative to the wild-type SIRPγ peptide as shown in SEQ ID NO: 20.

In some preferred embodiments, the aforementioned SIRPγ peptide variant, wherein the SIRPγ peptide variant is a SIRPγ peptide variant further comprising amino acid substitutions at one or more positions selected from the group consisiting of M6, V27, L30, V33, V36, L37, V42, E47, L66, T67, V92 and S98.

In some preferred embodiments, the aforementioned SIRPγ peptide variant, wherein the SIRPγ peptide variant is as shown in SEQ ID NO: 1,

EEELQMIQPE KLLLVTVGET ATLHCTVTSL $X_1$PVGPVLWFR GVGPGRELIY $X_2X_3$G$X_4$G$X_5$FPRV TTVSDLTKR$X_6$ N$X_7$DFSIRISS ITPADVGTYY CVKFRKGSPE DVEFKSGPGT E$X_8$ALGAKPS (SEQ ID NO: 1)

wherein, $X_1$ is selected from L or W, $X_2$ is selected from M, V, F, I or L, $X_3$ is selected from Q, S or T, $X_4$ is selected from E, T or R, $X_5$ is selected from H or R, $X_6$ is selected from D, N or E, $X_7$ is selected from I, V, M, R or K, and $X_8$ is selected from M or V.

In some preferred embodiments, the aforementioned SIRPγ peptide variant, the SIRPγ peptide variant is as shown in SEQ ID NO: 2:

EEELQMIQPE KLLLVTVGET ATLHCTVTSL $X_1$PVGPVLWFR GVGPGRELIY R$X_3$G$X_4$G$X_5$FPRV TTVSDLTKR$X_6$ N$X_7$DFSIRISS ITPADVGTYY CVKFRKGSPE DVEFKSGPGT E$X_8$ALGAKPS (SEQ ID NO: 2)

wherein, $X_1$ is selected from L or W, $X_3$ is selected from Q, S or T, $X_4$ is selected from E, T or R, $X_5$ is selected from H or R, $X_6$ is selected from D, N or E, $X_7$ is selected from I, V, M, R or K, and $X_8$ is selected from M or V.

In some preferred embodiments, the aforementioned SIRPγ peptide variant, wherein the SIRPγ peptide variant is as shown in the group consisiting of SEQ ID NO: 21, 22, 23, 24, 25, 26, 27, 28, 29, 30, 31, 32, 33, 34, 35, 36, 37, 38, 39 and 40.

In other aspects of the present disclosure, also provided is a fusion protein comprising a SIRPγ peptide variant and an antibody Fc fragment, the SIRPγ peptide variant being the SIRPγ peptide variant according to any one described above; in some embodiments, the antibody Fc fragment is a human antibody Fc fragment; in some preferred embodiments, the antibody Fc fragment sequence is the same as the Fc fragment sequence in the heavy chain constant region as shown in SEQ ID NO: 10 or 11; in some preferred embodiments, the amino acid sequence of the fusion protein is as shown in the group consisiting of SEQ ID NO: 86, 110, 114, 115, 116, 117, 118, 119, 120, 121, 122, 123, 124, 125, 126, 127, 128, 129, 130 and 131.

In other aspects of the present disclosure, also provided is an anti-human PD-L1 antibody comprising a light chain variable region and a heavy chain variable region of antibody, the heavy chain variable region comprises HCDR1, HCDR2 and HCDR3 regions as shown in SEQ ID NO: 103, 104 and 105, respectively, and the light chain variable region comprises LCDR1, LCDR2 and LCDR3 regions as shown in SEQ ID NO: 106, 112 and 108, respectively.

In some embodiments, the aforementioned anti-human PD-L1 antibody, the heavy chain variable region is shown in SEQ ID NO: 8, and the light chain variable region is shown in SEQ ID NO: 113.

In some embodiments, the aforementioned anti-human PD-L1 antibody, wherein the anti-human PD-L1 antibody is a full-length antibody, further comprising an antibody constant region, preferably, the heavy chain constant region of the antibody is selected from the constant regions of human IgG1, IgG2, IgG3 and IgG4, the light chain constant region of the antibody is selected from the constant regions of human antibody κ and λ chains, more preferably, the full-length antibody comprises the heavy chain constant region as shown in SEQ ID NO: 10 or 11 and the light chain constant region as shown in SEQ ID NO: 12.

In some preferred embodiments, the aforementioned anti-human PD-L1 antibody, the antibody comprises a heavy chain as shown in SEQ ID NO: 16 or 18, and a light chain as shown in SEQ ID NO: 111.

In other aspects, the present disclosure also provides a pharmaceutical composition comprising a therapeutically effective amount of the aforementioned bifunctional fusion protein, or the aforementioned SIRPγ peptide variant, or the aforementioned fusion protein, or the aforementioned anti-human PD-L1 antibody, and one or more pharmaceutically acceptable carriers, diluents, buffers or excipients. In some embodiments, the therapeutically effective amount is a unit dose of the composition comprising 0.1 to 3000 mg of the aforementioned bifunctional fusion protein, or the SIRPγ peptide variant according to the description above, or the fusion protein according to the description above, or the anti-human PD-L1 antibody according to the description above.

In other aspects, the present disclosure also provides an isolated nucleic acid molecule encoding the aforementioned bifunctional fusion protein, or encoding the aforementioned SIRPγ peptide variant.

In other aspects, the present disclosure also provides an isolated nucleic acid molecule encoding the aforementioned anti-human PD-L1 antibody.

In other aspects, the present disclosure also provides a recombinant vector comprising the aforementioned isolated nucleic acid molecule.

In other aspects, the present disclosure also provides a host cell transformed with a recombinant vector according to the description above, which is selected from prokaryotic cells and eukaryotic cells, preferably eukaryotic cells, more preferably mammalian cells or insect cells.

In other aspects, the present disclosure also provides a method for producing the aforementioned bifunctional fusion protein, or a method for producing the SIRPγ peptide variant according to the description above, or a method for producing the fusion protein according to the description above, or a method for producing the anti-human PD-L1 antibody according to the description above, the method comprises culturing the aforementioned host cell in culture medium to form and accumulate the aforementioned bifunctional fusion protein, or the SIRPγ peptide variant according to the description above, and recovering the bifunctional fusion protein or SIRPγ peptide variant, or the fusion protein according to the description above, or the anti-human PD-L1 antibody according to the description above from the culture.

In other aspects, the present disclosure also provides a method for eliminating immunosuppression-related diseases in a subject, which comprises administering to the subject a therapeutically effective amount of the aforementioned bifunctional fusion protein, or the SIRPγ peptide variant according to the description above, or the fusion protein according to the description above, or the anti-human PD-L1 antibody according to the description above, or the aforementioned pharmaceutical composition, or the aforementioned isolated nucleic acid molecule, preferably, the therapeutically effective amount is a unit dose of the composition comprising 0.1 to 3000 mg of the aforementioned bifunctional fusion protein, or the SIRPγ peptide variant according to the description above, or the anti-human PD-L1 antibody according to the description above.

In some embodiments, the PD-L1-CD47 bifunctional fusion protein, the SIRPγ peptide variant, or the fusion protein according to the description above, or the anti-human PD-L1 antibody according to the description above, is administered to individuals in single or accumulative administrations at a dose of about 10 μg/kg, about 50 μg/kg, about 100 μg/kg, about 200 μg/kg, about 300 μg/kg, about 400 μg/kg, about 500 μg/kg, about 600 μg/kg, about 700 μg/kg, about 800 μg/kg, about 900 μg/kg, about 1000 g/kg, about 1100 g/kg, 1200 g/kg, 1300 g/kg, 1400 g/kg, 1500 g/kg, 1600 g/kg, 1700 g/kg, 1800 g/kg, 1900 g/kg, about 2000 g/kg, about 3000 g/kg, about 4000 g/kg, about 5000 g/kg, about 6000 g/kg, about 7000 g/kg, about 8000 g/kg, about 9000 g/kg, about 10 mg/kg, about 20 mg/kg, about 30 mg/kg, about 40 mg/kg, about 50 mg/kg, about 60 mg/kg, about 70 mg/kg, about 80 mg/kg, about 90 mg/kg, about 100 mg/kg, about 200 mg/kg, about 300 mg/kg, about 400 mg/kg, about 500 mg/kg, about 600 mg/kg, about 700 mg/kg, about 800 mg/kg, about 900 mg/kg or about 1000 mg/kg.

In other aspects, the present disclosure also provides use of the aforementioned bifunctional fusion protein, or the SIRPγ peptide variant according to the description above, or the fusion protein according to the description above, or the anti-human PD-L1 antibody according to the description above, or the aforementioned pharmaceutical composition, or the aforementioned isolated nucleic acid molecule, in the preparation of medicament for eliminating immunosuppression-related diseases in a subject, preferably, the unit dose of pharmaceutical composition comprises 0.1 to 3000 mg of the aforementioned bifunctional fusion protein, or the aforementioned SIRPγ peptide variant, or the aforementioned anti-human PD-L1 antibody.

In other aspects, the present disclosure also provides the aforementioned bifunctional fusion protein, or the aforementioned SIRPγ peptide variant, or the fusion protein according to the description above, or the anti-human PD-L1 antibody according to the description above, or the aforementioned pharmaceutical composition, or the aforementioned isolated nucleic acid molecule used as medicament for eliminating immunosuppression-related diseases in a subject, preferably, the unit dose of the pharmaceutical composition comprises 0.1 to 3000 mg of the aforementioned bifunctional fusion protein, or the aforementioned SIRPγ peptide variant, or the aforementioned anti-human PD-L1 antibody.

In another aspect, the present disclosure also provides the aforementioned bifunctional fusion protein, or the aforementioned SIRPγ peptide variant, or the fusion protein according to the description above, or the anti-human PD-L1 antibody according to the description above, or the aforementioned pharmaceutical composition, or the aforementioned isolated nucleic acid molecule used as medicament, preferably, the unit dose of the pharmaceutical composition comprises 0.1 to 3000 mg of the aforementioned bifunctional fusion protein, or the aforementioned SIRPγ peptide variant, or the aforementioned anti-human PD-L1 antibody.

In some embodiments, eliminating the aforementioned immunosuppression-related diseases in a subject include cancer, bacterial or viral infection. The cancer includes but is not limited to carcinoma, lymphoma, blastoma, sarcoma and leukemia or lymphoid malignancy. More specific examples of the cancer include squamous cell carcinoma, myeloma, small cell lung cancer, non-small cell lung cancer (NSCLC), head and neck squamous cell carcinoma (HNSCC), glioma, Hodgkin's lymphoma, non-Hodgkin's lymphoma, diffuse large B-cell lymphoma (DLBCL), follicular lymphoma, acute lymphoblastic leukemia (ALL), acute myelocytic leukemia (AML), chronic lymphocytic leukemia (CLL), chronic myelocytic leukemia (CML), primary mediastinal large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, bladder cancer, hepatoma, breast cancer, colon cancer, hepatocellular carcinoma (HCC), clear cell renal cell carcinoma (RCC), head and neck cancer, pharyngolaryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myelodysplastic tumor, neuroendocrine tumor, Merkel cell carcinoma, testicular cancer and skin cancer.

## DESCRIPTION OF THE DRAWINGS

Figure 1: Schematic diagram of the PD-L1-CD47 bifunctional fusion protein in some embodiments.

Figure 2A to Figure 2C: The binding ability test of PD-L1-CD47 bifunctional fusion protein to CD47 on the surface of human red blood cells, the negative controls (control) on the right side represent cell + secondary antibody. Figure 2A and Figure 2B represent the binding ability test of different PD-L1-CD47 bifunctional fusion proteins (10 μg/ml) to CD47 on the surface of red blood cells; Figure 2C represents the binding ability test of different PD-L1-CD47 bifunctional fusion proteins (10 μg/ml and 1 μg/ml) to CD47 on the surface of red blood cells.

Figure 3: The binding ability test of PD-L1-CD47 bifunctional fusion protein to CD47 on the surface of Raji cells, the negative control on the right side represents cell + secondary antibody.

Figure 4: red blood cell phagocytosis mediated by PD-L1-CD47 bifunctional fusion protein.

Figure 5A to Figure 5B: Phagocytosis of tumor cells (Molp-8 cells) mediated by PD-L1-CD47 bifunctional fusion protein. Figures 5A and Figure 5B represent the phagocytosis of tumor cells mediated by different PD-L1-CD47 bifunctional fusion proteins detected in different batches of experiments.

Figure 6: Red blood cell coagulation mediated by PD-L1-CD47 bifunctional fusion protein.

Figure 7A to Figure 7E: IFN-γ secretion mediated by PD-L1-CD47 bifunctional fusion protein. Figure 7A, Figure 7B, Figure 7C, Figure 7D and Figure 7E represent the results of IFN-γ secretion mediated by different PD-L1-CD47 bifunctional fusion proteins.

Figure 8: The effect of different PD-L1-CD47 bifunctional fusion proteins on the tumor volume of MC38/H-11-hCD47 (#5-4) tumor-bearing B-hCD274/hCD47/hSIRPα mousemodel .

Figure 9: The effect of different PD-L1-CD47 bifunctional fusion proteins on the tumor volume of MC38-hPD-L1-hCD47 tumor-bearing C57/BL-6 mouse model .

Figure 10: The effect of different PD-L1-CD47 bifunctional fusion proteins on the tumor volume of MC38-hPD-L1 tumor-bearing C57/BL-6 mouse model .

Figure 11: The effect of different PD-L1-CD47 bifunctional fusion proteins on the tumor volume of Molp-8 tumor-bearing nude mice *in vivo* model. This model focuses on investigating the anti-tumor effect of the bifunctional fusion protein on CD47-targeting pathway.

## DETAILED DESCRIPTION OF THE EMBODIMENTS

Terms

The three-letter codes and one-letter codes of amino acids used in the present disclosure are as described in J. biol. chem, 243, p3558 (1968).

The term "bifunctional fusion protein" refers to a protein molecule that can bind to two target proteins or target antigens. The bifunctional fusion protein in the present disclosure mainly comprises a protein capable of binding to PD-L1 and CD47 on the cell surface, which is a fusion protein formed by linking an anti-PD-L1 antibody to a SIRPγ polypeptide variant.

The term "PD-L1" refers to programmed death ligand 1, also known as CD274 or B7H1. The amino acid sequence of human full-length PD-L1 is provided in GenBank under the accession number NP_054862.1. Unless specified to be from a non-human species, the term "PD-L1" means human PD-L1.

Anti-human PD-L1 antibody refers to an antibody capable of binding to human PD-L1 and capable of blocking the binding of PD-1 to PD-L1. The anti-human PD-L1 antibody can be selected from Avelumab, Atezolizumab, Durvalumab, JS-003, CS-1001, LY-3300054, KD-033, CK-301, CCX-4503, CX-072, KN-035, HRP00052, HRP00049, FAZ-053, GR-1405, KD-005, HLX-20, KL-A167, CBT-502, STI-A1014, REMD-290, BGB-A333, BCD-135, MCLA-145, etc. In addition, the anti-human PD-L1 antibody in the present disclosure can also be selected from full-length antibodies h1830, h1831, or an anti-PD-L1 antibody or antigen-binding fragment thereof that has the same CDR combination as that of the h1830 and h1831 antibodies, respectively.

"SIRPγ peptide" refers to a human SIRPγ-D1 domain peptide (the amino acid sequence of the wild-type SIRPγ peptide is shown in SEQ ID NO: 20), which has the activity of binding to human CD47. The SIRPγ peptides can also include a human SIRPγ-D1 domain peptide mutant, or a "SIRPγ peptide variant", which is with amino acid substitutions at one or more positions relative to the wild-type SIRPγ peptide, the number of the amino acid substitution mutations is no more than 20, 19, 18, 17, 16, 15, 14, 13, 12, 11, 10, 9, 8, 7, 6, 5, 4, 3, 2, and the SIRPγ peptide variant has enhanced activity of binding to CD47 on the surface of tumor cells than the wild-type SIRPγ peptide (the affinity of the wild-type SIRPγ binding with CD47 is at micromolar level). Furthermore, in some specific embodiments, the SIRPγ peptide variant gains the property of not binding or (relative to the binding activity to CD47 on the surface of tumor cells) reduced binding to CD47 on the surface of human red blood cells. As shown in Table 1 below, for example, the S58 peptide is a mutant substituted at position K19 for K19E, position N51 for N51M, position Q52 for Q52S, position K53 for K53G, position E54 for E54R, and position N101 for N101D, relative to the wild-type SIRPγ peptide shown in SEQ ID NO: 20.

In some specific embodiments, the alternative positions of the amino acid substitution mutation can include amino acid substitution mutations at one or more position(s) selected from K19, K53, N101, L31, N51, Q52, E54, H56, N70, M72, M112, M6, V27, L30, V33, V36, L37, V42, E47, L66, T67, V92 or S98.

In some specific embodiments, the SIRPγ peptide variant is as shown in SEQ ID NO: 1:

EEELQMIQPE KLLLVTVGET ATLHCTVTSL $X_1$PVGPVLWFR GVGPGRELIY $X_2X_3$G$X_4$G$X_5$FPRV TTVSDLTKR$X_6$ N$X_7$DFSIRISS ITPADVGTYY CVKFRKGSPE DVEFKSGPGT E$X_8$ALGAKPS (SEQ ID NO: 1) wherein, $X_1$ is selected from L or W, $X_2$ is selected from M, V, F, I or L, $X_3$ is selected from Q, S or T, $X_4$ is selected from E, T or R, $X_5$ is selected from H or R, $X_6$ is selected from D, N or E, $X_7$ is selected from I, V, M, R or K, and $X_8$ is selected from M or V.

In some specific embodiments, the SIRPγ peptide variant is as shown in SEQ ID NO: 2:

EEELQMIQPE KLLLVTVGET ATLHCTVTSL $X_1$PVGPVLWFR GVGPGRELIY R$X_3$G$X_4$G$X_5$FPRV TTVSDLTKR$X_6$ N$X_7$DFSIRISS ITPADVGTYY CVKFRKGSPE DVEFKSGPGT E$X_8$ALGAKPS (SEQ ID NO: 2) wherein, $X_1$ is selected from L or W, $X_3$ is selected from Q, S or T, $X_4$ is selected from E, T or R, $X_5$ is selected from H or R, $X_6$ is selected from D, N or E, $X_7$ is selected from I, V, M, R or K, and $X_8$ is selected from M or V.

The table below shows the amino acid substitution mutation positions and exemplary substituted amino acid residues of different SIRPγ peptide variants relative to the wild-type SIRPγ peptide.

Table 1

| SIRPγ peptide (wild type) amino acid residue and position | K19 | L31 | N51 | Q52 | K53 | E54 | H56 | N70 | M72 | N10 1 | M11 2 |
|---|---|---|---|---|---|---|---|---|---|---|---|
| S58 | E | | M | S | G | R | | | | D | |
| S79 | E | W | R | T | G | T | R | | | D | V |
| S15 | E | | V | S | G | R | | E | R | D | |
| S12 | E | | V | S | G | R | | E | K | D | |
| S19 | E | | V | S | G | R | | | R | D | |
| S85 | E | | V | S | G | R | | | K | D | |
| S37 | E | | M | S | G | R | | | K | D | |
| S38 | E | | F | S | G | R | | | | D | |
| S22 | E | | I | S | G | R | | | | D | |
| S29 | E | | L | S | G | R | | | | D | |
| S34 | E | | R | S | G | R | | | | D | |
| S41 | E | | V | S | G | R | | | | D | |
| S42 | E | | M | S | G | R | | | I | D | |
| S43 | E | | M | S | G | R | | | R | D | |
| S44 | E | | M | S | G | R | | | V | D | |
| S45 | E | | M | S | G | R | | D | | D | |
| S46 | E | | M | S | G | R | | E | K | D | |
| S47 | E | | M | S | G | R | | E | K | D | V |
| S48 | E | | R | S | G | R | | | K | D | |
| S49 | E | | R | S | G | R | | E | K | D | |

The term "antibody (Ab)" comprises any antigen-binding molecule or molecular complex that comprises at least one complementarity determining region (CDR) that specifically binds or interacts with a specific antigen (or epitope thereof, for example PD-L1 antigen or epitope thereof). The term "antibody" comprises: immunoglobulin molecules comprising four polypeptide chains, two heavy (H) chains and two light (L) chains, interconnected by disulfide bond(s), and multimers thereof (for example IgM). Each heavy chain comprises a heavy chain variable region (abbreviated as HCVR or VH herein) and a heavy chain constant region (CH). This heavy chain constant region comprises three regions (domains), CHI, CH2 and CH3. Each light chain comprises a light chain variable region (abbreviated as LCVR or VL herein) and a light chain constant region (CL). The light chain constant region comprises one region (domain, CL). The VH and VL regions can be further subdivided into hypervariable regions, called complementarity determining regions (CDR), between which more conservative regions (called framework regions, FR) are interspersed. Each VH and VL consists of three CDRs and four FRs, arranged from the amino terminal to the carboxyl terminal in the following order: FR1, CDR1, FR2, CDR2, FR3, CDR3, FR4. In different examples of the present disclosure, the FRs of the anti-PD-L1 antibody (or antigen-binding fragment thereof) can be the same as the human germline sequence, or can be naturally or artificially modified. The antibodies can be antibodies of different subclasses, for example, IgG (for example, IgG1, IgG2, IgG3 or IgG4 subclasses), IgA1, IgA2, IgD, IgE or IgM antibodies.

The terms "full-length antibody", "intact antibody", "complete antibody" and "whole antibody" are used interchangeably herein and refer to an antibody in a substantially intact form, as distinguished from the antigen-binding fragments defined below. The terms specifically refer to an antibody in which the heavy chain comprises VH region, CH1 region, hinge region and Fc region from the amino terminal to the carboxyl terminal, and the light chain comprises VL region and CL region from the amino terminal to the carboxyl terminal.

Non-limiting examples of antigen-binding fragment include: (i) Fab fragment; (ii) F(ab')2 fragment; (iii) Fd fragment; (iv) Fv fragment; (v) single-chain Fv (scFv) molecule; (vi) dAb fragment; and (vii) the smallest recognition unit consisting of amino acid residues mimicking the antibody hypervariable region (for example isolated complementarity determining regions (CDR), for example CDR3 peptide) or restrictive FR3-CDR3-FR4 peptide. Other engineered molecules, for example region-specific antibody, single-domain antibody, region-deleted antibody, chimeric antibody, CDR-grafted antibody, diabody, triabody, tetrabody, minibody, nanobody (e.g. monovalent nanobody, bivalent nanobody, etc.), small modular immunopharmaceutical (SMIP) and shark variable IgNAR region, are also encompassed in the term "antigen-binding fragment" as used herein.

The antigen-binding fragment of an antibody will typically comprise at least one variable region. The variable region can be a region of any size or amino acid composition, and will generally comprise CDRs adjacent to one or more framework sequence(s) or within the framework. In an antigen-binding fragment with VH regions and VL regions, the VH and VL regions can be positioned opposite to each other in any suitable arrangement. For example, the variable region could be dimerized and contain VH-VL or VL-VH dimers.

In certain examples, in any configuration of the variable region and the constant region of the antigen-binding fragment, the variable region and the constant region can be directly linked to each other or can be linked through an intact or partial hinge or linker region. The hinge region can consist of at least 2 (for example 5, 10, 15, 20, 40, 60 or more) amino acids, so that it produces flexible and semi-flexible connection between adjacent variable and/or constant regions in a single polypeptide molecule. Besides, the antigen-binding fragment of the present disclosure involves homodimers or heterodimers (or other multimers) comprising variable regions and constant regions that are noncovalently linked to each other and/or linked to one or more monomer VH or VL regions (for example by disulfide bond).

"Murine antibody" in the present disclosure is a mouse or rat-derived monoclonal antibody prepared according to the knowledge and skills in the art. During preparation, the test subject is injected with antigen, and then hybridomas expressing antibodies with the desired sequence or functional properties are isolated. When the injected test subject is a mouse, the antibody produced is a mouse-derived antibody, and when the injected test subject is a rat, the antibody produced is a rat-derived antibody.

"Chimeric antibody" is an antibody formed by fusing the variable region of an antibody of the first species (such as mouse) with the constant region of an antibody of the second species (such as human). Establishing a chimeric antibody requires first establishing a hybridoma secreting monoclonal antibodies of the first species, then cloning the variable region gene from the hybridoma cells, and then cloning the antibody constant region gene of the second species as necessary, linking the variable region gene of the first species with the constant region gene of the second species to form a chimeric gene which is then inserted into an expression vector, and finally expressing the chimeric antibody molecule in a eukaryotic system or a prokaryotic system. In a preferred embodiment of the present disclosure, the antibody light chain of the chimeric antibody further comprises a light chain constant region of a human κ, λ chain or variant thereof. The antibody heavy chain of the chimeric antibody further comprises the heavy chain constant region of human IgG1, IgG2, IgG3, IgG4 or variant thereof, preferably the heavy chain constant region of human IgG1, IgG2 or IgG4, or the heavy chain constant region variants of IgG1, IgG2 or IgG4 comprising amino acid mutations (such as YTE mutation, back mutation, L234A and/or L235A mutation or S228P mutation).

The term "humanized antibody", including CDR-grafted antibody, refers to the antibody produced by grafting CDR sequences of an antibody derived from animals (for example murine) into the framework regions of a human antibody variable region. The humanized antibody can overcome the heterogeneous reaction induced by the chimeric antibody carrying a large amount of heterogeneous protein components. Such framework sequences can be obtained from public DNA databases or published references that include germline antibody gene sequences. For example, the germline DNA sequences of the human heavy chain and light chain variable region genes can be found in the "VBase" human germline sequence database (available on the Internet http://www.vbase2.org/), as well as in Kabat, E. A., et al., 1991, Sequences of Proteins of Immunological Interest, 5th edition. In order to avoid the decrease in activity caused by the decrease in immunogenicity, the FR sequence in human antibody variable region can be subjected to a small amount of back mutations to maintain activity. The humanized antibody of the present disclosure also includes a humanized antibody that has been further displayed by phage and subjected to affinity maturation for the CDR.

Due to the contact residues of the antigen, CDR grafting could result in reduced affinity of the produced antibody or antigen-binding fragment thereof to the antigen due to the framework residues in contact with the antigen. Such interactions could be the result of hypermutation of somatic cells. Therefore, it could still be necessary to graft such donor framework amino acids to the framework of the humanized antibody. The amino acid residues from non-human antibodies or antigen-binding fragments thereof which involve in antigen binding can be identified by examining the sequence and structure of the animal monoclonal antibody variable region. Residues in the CDR donor framework that differ from the germline can be considered related. If the closest germline cannot be determined, the sequence can be compared with the consensus sequence of a subclass or animal antibody sequence with a high percentage of similarity. Rare framework residues are thought to be the result of hypermutation of somatic cells and thus play an important role in binding.

In an embodiment of the present disclosure, the antibody or antigen-binding fragment thereof could further comprise the light chain constant region of human or murine κ, λ chain or variant thereof, or further comprise the heavy chain constant region of human or murine IgG1, IgG2, IgG3, IgG4 or variant thereof.

"Conventional variant" of the human antibody heavy chain constant region and the human antibody light chain constant region refers to the variant of heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are such as YTE mutations, L234A and/or L235A mutations, or S228P mutations, or mutations to obtain a knob-into-hole structure (so that the antibody heavy chain has a combination of knob-Fc and hole-Fc) known in the art. These mutations have been confirmed to make the antibody have new properties, but do not change the function of the antibody variable region.

"Human antibody" and "human-derived antibody" can be used interchangeably, and can be an antibody derived from human or an antibody obtained from a transgenic organism which is "engineered" to produce specific human antibodies in response to antigen stimulation and can be produced by any method known in the art. In some technologies, the elements of human heavy chain and light chain gene loci are introduced into cell lines in which the endogenous heavy chain and light chain gene loci are target disrupted. The transgenic organism can synthesize human antibodies specific to antigens, and the organism can be used to produce human antibody-secreting hybridomas. A human antibody can also be an antibody in which the heavy chain and light chain are encoded by nucleotide sequences derived from one or more human DNA origins. A fully human antibody can also be constructed by gene or chromosome transfection methods and phage display technology, or constructed by B cells activated *in vitro,* all of which are known in the art.

"Monoclonal antibody" refers to an antibody obtained from a population of substantially homogeneous antibodies, that is, except for possible variant antibodies (for example, comprising naturally-occurring mutations or mutations generated during the manufacture of monoclonal antibody preparations, these variants are usually present in a small amount), the individual antibodies constituting the population recognize the same and/or bind with the same epitope. Each monoclonal antibody of a monoclonal antibody preparation (formulation) is directed against a single determinant on an antigen. Therefore, the modifier "monoclonal" indicates the characteristics of the antibody as obtained from a substantially homogeneous antibody population, and should not be interpreted as requiring any specific method to manufacture the antibody. For example, monoclonal antibodies used according to the present disclosure can be prepared by various techniques, including but not limited to hybridoma methods, recombinant DNA methods, phage display methods, as well as methods that utilizes transgenic animals comprising the complete or partial human immunoglobulin gene loci. Such methods and other exemplary methods for preparing monoclonal antibodies are described herein.

In addition, although the two domains VL and VH of the Fv fragment are encoded by separate genes, recombination methods can be used to connect them by synthetic linkers, so that they can be produced as a single protein chain in which the VL and VH regions pair to form a monovalent molecule (referred to as single-chain Fv (scFv); see, for example, Bird et al. (1988) Science 242: 423-426; and Huston et al. (1988) Proc. Natl. Acad. Sci USA 85: 5879-5883). Such single chain antibodies are also intended to be included in the term "antigen-binding fragment" of antibody. Such antibody fragments are obtained by using conventional techniques known to those skilled in the art, and the fragments are screened for function in the same manner as that used for screening intact antibodies. The antigen binding moiety can be produced by recombinant DNA technology or by enzymatic or chemical fragmentation of the intact immunoglobulin.

The antigen-binding fragment can also be incorporated into a single-chain molecule comprising a pair of tandem Fv fragments (VH-CH1-VH-CH1), which together with a complementary light chain polypeptide forms a pair of antigen-binding regions (Zapata et al., 1995, Protein Eng. 8(10): 1057-1062; and U.S. Patent No. 5,641,870).

Fab is an antibody fragment with a molecular weight of about 50,000 Da and with antigen-binding activity, obtained by treating an IgG antibody with the protease papain (which cleaves the amino acid residue at position 224 in the H chain), in which about half of the H chain at N-terminal side and the entire L chain are bound together by disulfide bond(s).

F(ab')2 is is an antibody fragment with a molecular weight of about 100,000 Da and with antigen-binding activity, obtained by digesting the downstream part of the two disulfide bonds in the hinge region of IgG with pepsin, and comprises two Fab regions linked at the hinge position.

Fab' is an antibody fragment with a molecular weight of about 50,000 Da and with antigen-binding activity, obtained by cleaving the disulfide bond in the hinge region of the aforementioned F(ab')2. Fab' can be produced by treating the F(ab')2 that specifically recognizes and binds an antigen with reducing agents, for example dithiothreitol.

In addition, Fab' can be expressed by inserting the DNA encoding the Fab' fragment of the antibody into a prokaryotic expression vector or a eukaryotic expression vector and introducing the vector into a prokaryotic organism or eukaryotic organism.

The term "single-chain antibody", "single-chain Fv" or "scFv" refers to molecules comprising an antibody heavy chain variable domain (or region; VH) and an antibody light chain variable domain (or region; VL) linked by a linker. Such scFv molecules can be represented by a general formula: $NH_2$-VL-linker-VH-COOH or $NH_2$-VH-linker-VL-COOH. Suitable linkers of prior art consist of repeating GGGGS amino acid sequences or variants thereof, for example 1 to 4 (including 1, 2, 3 or 4) repeated variants (Holliger et al. (1993), Proc. Natl. Acad. Sci. USA 90: 6444-6448). Other linkers that can be used in the present disclosure are described in Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol Immunother. 50:51-59.

"Anti-human PD-L1 antibody" includes a full-length antibody capable of specifically binding to human PD-L1, as well as an antigen-binding fragment comprising the variable region of the light chain and the variable region of the heavy chain of the full-length antibody, including but not limited to a single chain antibody (scFv), Fab fragment, or other antigen-binding fragment comprising scFv or Fab comprising the light chain variable region and the heavy chain variable region of the full-length antibody.

The "linked" when used in the expression "the SIRPγ peptide is linked to the polypeptide chain of the anti-human PD-L1 antibody" refers to an effective connection between the polypeptides, including, for example, connection via a peptide bond, or connection through a linker. The connection will not lead to loss of the respective functions of the SIRPγ peptide and the anti-human PD-L1 antibody.

"Linker" refers to a connective polypeptide sequence used to connect protein domains or different proteins or different polypeptides, usually with a certain degree of flexibility. The use of linkers will not lead to loss of the original functions of the protein domains. Exemplary linkers are shown in the table below.

Table 2. Sequences of exemplary linkers

| Name | Sequence | SEQ ID NO |
|---|---|---|
| GGGGS | GGGGS | 89 |
| GS16 | GGGGSGGGGSGGGGGG | 90 |
| GS17 | GGGGSGGGGSGGGGSGG | 91 |
| GS18 | GGGGSGGGGSGGGGSGGG | 92 |
| GS19 | GGGGSGGGGSGGGGSGGGG | 93 |
| 30AA-linker | DPALVHQRPAPPGGGGSGGGGSGGGGSGGG | 94 |
| GKPGS | GKPGS | 95 |
| GGGES | GGGES | 96 |
| (GGGGS)n, n is an integer of 2 to 7 | | |
| (GGGES)n, n is an integer of 2 to 7 | | |
| (GKPGS)n, n is an integer of 2 to 7 | | |

In some embodiments, the anti-PD-L1 antibodies can be linked to the SIRPγ peptide variant by linker(s). Some exemplary bifunctional fusion proteins include the fusion proteins shown below:

Table 3. PD-L1-CD47 bifunctional fusion proteins

| SIRPγ | h1830 GS16 linker | h1830 GS19 linker | h1831 GS16 linker | h1831 GS19 linker | h1831 GS16 linker | h1831 GS19 linker |
|---|---|---|---|---|---|---|
| S58 | h1830-S58 | h1830-19-S58 | h1831-S58 | h1831-19-S58 | h1831K-S58 | h1831K-19-S58 |
| S15 | h1830-S15 | h1830-19-S15 | h1831-S15 | h1831-19-S15 | h1831K-S15 | h1831K-19-S15 |
| S12 | h1830-S12 | h1830-19-S12 | h1831-S12 | h1831-19-S12 | h1831K-S12 | h1831K-19-S12 |
| S19 | h1830-S19 | h1830-19-S19 | h1831-S19 | h1831-19-S19 | h1831K-S19 | h1831K-19-S19 |
| S85 | h1830-S85 | h1830-19-S85 | h1831-S85 | h1831-19-S85 | h1831K-S85 | h1831K-19-S85 |
| S37 | h1830-S37 | h1830-19-S37 | h1831-S37 | h1831-19-S37 | h1831K-S37 | h1831K-19-S37 |

(continued)

| SIRPγ | h1830 GS16 linker | h1830 GS19 linker | h1831 GS16 linker | h1831 GS19 linker | h1831 GS16 linker | h1831 GS19 linker |
|---|---|---|---|---|---|---|
| S79 | h1830-S79 | h1830-19-S79 | h1831-S79 | h1831-19-S79 | h1831K-S79 | h1831K-19-S79 |
| S38 | h1830-S38 | h1830-19-S38 | h1831-S38 | h1831-19-S38 | h1831K-S38 | h1831K-19-S38 |
| S22 | h1830-S22 | h1830-19-S22 | h1831-S22 | h1831-19-S22 | h1831K-S22 | h1831K-19-S22 |
| S29 | h1830-S29 | h1830-19-S29 | h1831-S29 | h1831-19-S29 | h1831K-S29 | h1831K-19-S29 |
| S34 | h1830-S34 | h1830-19-S34 | h1831-S34 | h1831-19-S34 | h1831K-S34 | h1831K-19-S34 |
| S41 | h1830-S41 | h1830-19-S41 | h1831-S41 | h1831-19-S41 | h1831K-S41 | h1831K-19-S41 |
| S42 | h1830-S42 | h1830-19-S42 | h1831-S42 | h1831-19-S42 | h1831K-S42 | h1831K-19-S42 |
| S43 | h1830-S43 | h1830-19-S43 | h1831-S43 | h1831-19-S43 | h1831K-S43 | h1831K-19-S43 |
| S44 | h1830-S44 | h1830-19-S44 | h1831-S44 | h1831-19-S44 | h1831K-S44 | h1831K-19-S44 |
| S45 | h1830-S45 | h1830-19-S45 | h1831-S45 | h1831-19-S45 | h1831K-S45 | h1831K-19-S45 |
| S46 | h1830-S46 | h1830-19-S46 | h1831-S46 | h1831-19-S46 | h1831K-S46 | h1831K-19-S46 |
| S47 | h1830-S47 | h1830-19-S47 | h1831-S47 | h1831-19-S47 | h1831K-S47 | h1831K-19-S47 |
| S48 | h1830-S48 | h1830-19-S48 | h1831-S48 | h1831-19-S48 | h1831K-S48 | h1831K-19-S48 |
| S49 | h1830-S49 | h1830-19-S49 | h1831-S49 | h1831-19-S49 | h1831K-S49 | h1831K-19-S49 |

Diabody refers to an antibody fragment of dimerized scFv, and is an antibody fragment with bivalent antigen binding activity. In the bivalent antigen binding activity, the two antigens can be the same or different.

The dsFv is obtained by connecting polypeptides (in which one amino acid residue in each of VH and VL is substituted with a cysteine residue) via a disulfide bond between cysteine residues. The amino acid residues substituted with cysteine residues can be selected according to a known method (Protein Engineering. 7:697 (1994)) based on the three-dimensional structure prediction of the antibody.

The antigen-binding fragment in some examples of the present disclosure can be produced by the following steps: obtaining the cDNA encoding the VH and/or VL and other required domains of the monoclonal antibody of the present disclosure that specifically recognizes and binds to an antigen, constructing the DNA encoding the antigen-binding fragment, inserting the DNA into a prokaryotic expression vector or a eukaryotic expression vector, and then introducing the expression vector into a prokaryote or eukaryote to express the antigen-binding fragment.

"Fc region" can be a native sequence Fc region or a variant Fc region. Although the boundaries of the Fc region of an immunoglobulin heavy chain could vary, the Fc region of human IgG heavy chain is usually defined as extending from the amino acid residue at position Cys226 or from Pro230 to its carboxyl terminal. The numbering of residues in the Fc region is as the numbering of the EU index in Kabat. Kabat et al., Sequences of Proteins of Immunological Interest, 5th Edition, Public Health Service, National Institutes of Health, Bethesda, Md., 1991. The Fc region of immunoglobulin usually has two constant region domains, CH2 and CH3.

The term "amino acid difference" or "amino acid mutation" refers to the presence of amino acid changes or mutations in the variant protein or polypeptide compared with the original protein or polypeptide, including insertion, deletion or substitution of one or more amino acid, on the basis of the original protein or polypeptide.

"Variable region" of an antibody refers to the antibody light chain variable region (VL) or the antibody heavy chain variable region (VH), alone or in combination. As known in the art, the variable regions of the heavy chain and the light chain each consist of 4 framework regions (FRs) linked by 3 complementarity determining regions (CDRs) (also called hypervariable regions). The CDRs in each chain are held tightly together by FRs, and contribute to the formation of the antigen binding site of the antibody together with the CDRs from the other chain. At least two techniques for determining CDR can be mentioned: (1) a method based on cross-species sequence variability (i.e., Kabat et al., Sequences of Proteins of Immunological Interest, (5th edition, 1991, National Institutes of Health, Bethesda MD)); and (2) a method based on the crystallographic study of antigen-antibody complexes (Al-Lazikani et al., J. Molec. Biol. 273:927-948 (1997)). As used herein, CDR can refer to a CDR determined by either method or a combination of the two methods.

The term "antibody framework" or "FR region" refers to a moiety of the variable domain VL or VH, which serves as a scaffold for the antigen binding loop (CDR) of the variable domain. Essentially, it is a variable domain without CDR.

The term "complementarity determining region" and "CDR" refer to one of the six hypervariable regions in the variable domain of an antibody that mainly contribute to antigen binding. Generally, three CDRs (HCDR1, HCDR2, HCDR3) are present in each heavy chain variable region, and three CDRs (LCDR1, LCDR2, LCDR3) are present in each light chain variable region. Any one of a variety of well-known schemes can be used to determine the amino acid sequence boundaries of a CDR, including the "Kabat" numbering criteria (see Kabat et al. (1991), "Sequences of Proteins of Immunological Interest", 5th edition, Public Health Service, National Institutes of Health, Bethesda, MD), "Chothia" numbering criteria (Al-Lazikani et al., (1997) JMB 273:927-948) and ImMunoGenTics (IMGT) numbering criteria (Lefranc M. P., Immunologist, 7, 132-136 (1999); Lefranc, M. P. et al., Dev. Comp. Immunol., 27, 55-77 (2003)), etc. For example, for the classical format, following the Kabat criteria, the CDR amino acid residue numbers in the heavy chain variable domain (VH) are 31-35 (HCDR1), 50-65 (HCDR2) and 95-102 (HCDR3); the CDR amino acid residue numbers in the light chain variable domain (VL) are 24-34 (LCDR1), 50-56 (LCDR2) and 89-97 (LCDR3). Following the Chothia criteria, the CDR amino acid numbers in VH are 26-32 (HCDR1), 52-56 (HCDR2) and 95-102 (HCDR3); and the amino acid residue numbers in VL are 26-32 (LCDR1), 50- 52 (LCDR2) and 91-96 (LCDR3). By combining the CDR definitions of both Kabat and Chothia, the CDR consists of amino acid residues 26-35 (HCDR1), 50-65 (HCDR2) and 95-102 (HCDR3) in human VH and amino acid residues 24-34 (LCDR1), 50-56 (LCDR2) and 89-97 (LCDR3) in human VL. Following IMGT criteria, the CDR amino acid residue numbers in VH are roughly 26-35 (CDR1), 51-57 (CDR2) and 93-102 (CDR3), and the CDR amino acid residue numbers in VL are roughly 27-32 (CDR1), 50-52 (CDR2) and 89-97 (CDR3). Following IMGT criteria, the CDR region of an antibody can be determined by using the program IMGT/DomainGap Align.

"Antibody constant region domain" refers to the domain derived from the constant regions of the light chain and heavy chain of an antibody, including CL and CHI, CH2, CH3 and CH4 domains derived from different types of antibodies.

"Epitope" or "antigenic determinant" refers to a site on an antigen to which an immunoglobulin or an antibody specifically binds. Epitopes usually include at least 3, 4, 5, 6, 7, 8, 9, 10, 11, 12, 13, 14 or 15 consecutive or non-consecutive amino acids in a unique spatial conformation. See, for example, Epitope Mapping Protocols in Methods in Molecular Biology, Vol. 66, G. E. Morris, Ed. (1996).

The terms "specifically bind", "selectively bind", "selective binding" and "specific binding" refer to the binding of an antibody to an epitope on a predetermined antigen.

The term "affinity" refers to the strength of the interaction between an antibody and an antigen at a single epitope. Within each antigenic site, the variable region of the antibody "arm" interacts with the antigen at multiple amino acid positions through weak non-covalent forces; the greater the interaction, the stronger the affinity. As used herein, the term "high affinity" of an antibody or antigen-binding fragment thereof (e.g. Fab fragment) generally refers to an antibody or antigen-binding fragment with a $K_D$ of $1E^{-9}M$ or less (e.g., a $K_D$ of $1E^{-10}M$ or less, a $K_D$ of $1E^{-11}M$ or less, a $K_D$ of $1E^{-12}M$ or less, a $K_D$ of $1E^{-13}M$ or less, a $K_D$ of $1E^{-14}M$ or less, etc.).

The term "KD" or "$K_D$" refers to the dissociation equilibrium constant of a specific antibody-antigen interaction. Generally, an antibody binds to an antigen with a dissociation equilibrium constant (KD) of less than about $1E^{-8}M$, for example, less than about $1K^{-9}M$, $1E^{-10}M$ or $1E^{-11}M$ or less, for example, as measured in a BIACORE instrument using surface plasmon resonance (SPR) technology. The smaller the KD value, the greater the affinity is.

The term "nucleic acid molecule" refers to DNA molecules and RNA molecules. A nucleic acid molecule can be a single-stranded or double-stranded DNA molecule or RNA molecule, for example, a double-stranded DNA or mRNA. When a nucleic acid is placed in a functional relationship with another nucleic acid sequence, the nucleic acid is "operatively linked". For example, if a promoter or enhancer affects the transcription of a coding sequence, then the promoter or enhancer is operatively linked to the coding sequence.

The term "vector" means a construct capable of delivering one or more target genes or sequences and preferably expressing the same in a host cell. Examples of vectors include, but are not limited to, viral vector, naked DNA or RNA expression vector, plasmid, cosmid or phage vector, DNA or RNA expression vector associated with cationic flocculant, DNA or RNA expression vector encapsulated in liposome, and certain eukaryotic cell such as producer cell.

The methods for producing and purifying antibodies and antigen-binding fragments are well known in the prior art, such as Antibodies: A Laboratory Manual, Cold Spring Harbor, Chapters 5-8 and 15. For example, mice can be immunized with an antigen or fragment thereof, and the obtained antibody can be renatured and purified, and amino acid sequencing can be performed by using conventional methods. Antigen-binding fragments can also be prepared by using conventional methods. The antibody or antigen-binding fragment according to the present disclosure is genetically engineered to add one or more human FR regions onto the non-human CDR regions. The human FR germline sequences can be obtained from the website http://www.imgt.org/ by aligning against the IMGT human antibody variable region germline gene database via MOE software, or be obtained from The Immunoglobulin FactsBook, 2001ISBN012441351.

The term "host cell" refers to a cell into which an expression vector has been introduced. Host cells can include bacteria, microorganisms, plant or animal cells. Bacteria that can be easily transformed include members of the *enterobacteriaceae,* for example *Escherichia coli* or *Salmonella* strains; *Bacillaceae*, for example *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese Hamster Ovary Cell Line), HEK293 cells (non-limiting examples such as HEK293E cells) and NS0 cells.

The engineered antibody or antigen-binding fragment can be prepared and purified by conventional methods. For example, the cDNA sequences encoding the heavy chain and light chain can be cloned and recombined into a GS expression vector. The recombinant immunoglobulin expression vectors can be stably transfected into CHO cells. As an alternative prior art, mammalian expression systems can lead to glycosylation of antibodies, especially at highly conserved N-terminal positions of the Fc region. Stable clones are obtained by expressing antibodies that specifically bind with antigens. Positive clones are expanded in serum-free medium of bioreactors to produce antibodies. The culture medium into which the antibodies are secreted can be purified by conventional techniques. For example, Protein A or Protein G Sepharose FF column comprising adjusted buffer can be used for purification. Non-specifically bound components are washed away. Then the bound antibodies are eluted by the pH gradient, and the antibody fragments are detected by SDS-PAGE and collected. The antibodies can be filtered and concentrated by conventional methods. Soluble mixtures and multimers can also be removed by conventional methods, for example molecular sieves and ion exchange. The resulting product needs to be frozen immediately, such as at -70°C, or lyophilized.

"Administering", "administration","giving" and "treating", when applied to animals, humans, experimental subjects, cells, tissues, organs or biological fluids, refer to the contact of exogenous medicament, therapeutic agent, diagnostic agent, composition or manual operation (for example "euthanasia" in the example) with the animals, humans, subjects, cells, tissues, organs or biological fluids. "Giving" and "treating" can refer to for example treatment, pharmacokinetics, diagnosis, research and experimental methods. The treatment of cells includes contact of reagents with cells, and contact of reagents with fluids, in which the fluids are in contact with the cells. "Giving" and "treating" also mean treating for example cells by reagents, diagnostic agent, binding compositions or by another kind of cells *in vitro* and *ex vivo.* "Treating" when applied to human, veterinary or research subjects, refers to therapeutic treatment, prevention or prophylactic measures, research and diagnostic applications.

"Treatment" means applying an internal or external therapeutic agent, for example a composition comprising any one of the compounds of the present disclosure, to a patient (or subject) who has (or is suspected of having, or is susceptible to) one or more disease symptoms on which the therapeutic agent is known to have therapeutic effect. Generally, the therapeutic agent is given at an amount effective to alleviate one or more disease symptoms in the treated patient (or subject) or population to induce the regression of such symptoms or inhibit the development of such symptoms to any clinically detectable extent. The amount of therapeutic agent that is effective to alleviate any specific disease symptom (also referred to as a "therapeutically effective amount") can vary according to a variety of factors, for example the patient's (or subject's) disease state, age and body weight, and the ability of the agent to produce the desired therapeutic effect in the patient (or subject). Whether the disease symptoms have been alleviated can be evaluated through any clinical testing methods commonly used by doctors or other health care professionals to evaluate the severity or progression of the symptoms. Although the embodiments of the present disclosure (for example treatment methods or products) are ineffective in alleviating each target disease symptom, but shall reduce the target disease symptom in a statistically significant number of patients (or subjects), as determined according to any statistical test methods known in the art, such as Student t-test, chi-square test, Mann and Whitney's U test, Kruskal-Wallis test (H test), Jonckheere-Terpstra test and Wilcoxon test.

"Amino acid conservative modification" or "amino acid conservative substitution" refers to the substitution of amino acids in a protein or polypeptide with other amino acids with similar characteristics (for example charge, side chain size, hydrophobicity/hydrophilicity, main chain conformation and rigidity, etc.), thereby allowing frequent changes without changing the biological activity or other required characteristics (for example antigen affinity and/or specificity) of the protein or polypeptide. Those skilled in the art know that, generally, a single amino acid substitution in a non-essential region of a polypeptide does not substantially change the biological activity (see, for example, Watson et al.,

(1987) Molecular Biology of the Gene, The Benjamin/Cummings Pub. Co., Page 224, (4th edition)). In addition, the substitution of amino acids with similar structure or function is unlikely to disrupt the biological activity. Exemplary conservative substitutions are stated in the table "Exemplary conservative substitutions of amino acid" below.

Table 4. Exemplary conservative substitutions of amino acid

| Original residue | Conservative substitution |
|---|---|
| Ala(A) | Gly; Ser |
| Arg(R) | Lys; His |
| Asn(N) | Gln; His; Asp |
| Asp(D) | Glu; Asn |
| Cys(C) | Ser; Ala; Val |
| Gln(Q) | Asn; Glu |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala |
| Ser(S) | Thr |
| Thr(T) | Ser |
| Trp(W) | Tyr; Phe |
| Tyr(Y) | Trp; Phe |
| Val(V) | Ile; Leu |

"Effective amount", "effective dose" refers to the amount of an agent, compound or pharmaceutical composition necessary to obtain any one or more beneficial or desired therapeutic results. For prophylactic use, the beneficial or desired results include elimination or reduction of risk, reduction of severity or delay of the disease onset, including the biochemistry, histology and/or behavioral symptoms of the disease, complications thereof and intermediate pathological phenotypes that appear during the developmental process of the disease. For therapeutic applications, the beneficial or desired results include clinical results, for example reducing the incidence of various target antigen-related disorders of the present disclosure or improving one or more symptoms of the disorder, reducing the dose of other agents required to treat the disorder, enhancing the therapeutic effect of another agent, and/or delaying the progression of target antigen-related disorder of the present disclosure in the patient (or subject).

"Exogenous" refers to substances produced outside organisms, cells or human bodies according to circumstances.

"Endogenous" refers to substances produced inside cells, organisms or human bodies according to circumstances.

"Isolated" refers to a purified state, and in this case means that the designated molecule is substantially free of other biomolecules, for example nucleic acids, proteins, lipids, carbohydrates or other materials, for example cell debris and growth medium. Generally, the term "isolated" is not intended to mean the complete absence of these materials or the absence of water, buffer or salt, unless they are present in an amount that significantly interferes with the experimental or therapeutic use of the compound as described herein.

"Optional" or "optionally" means that the event or environment that follows the term can but does not have to occur, and this description includes occasions where the event or environment occurs or does not occur.

"Pharmaceutical composition" means a mixture comprising one or more of the compounds described in the present disclosure, or a physiologically/pharmaceutically acceptable salt or a prodrug thereof, and other chemical compositions, for example physiological/pharmaceutically acceptable carriers and excipients. The purpose of the pharmaceutical composition is to promote the administration to organisms, which facilitates the absorption of the active ingredient and thereby exerts biological activity.

The term "pharmaceutically acceptable carrier" refers to any inactive substance suitable for use in a formulation for the delivery of antibodies or antigen-binding fragments. The carrier can be an anti-adhesive agent, binder, coating, disintegrant, filler or diluent, preservative (such as antioxidant, antibacterial or antifungal agent), sweetener, absorption delaying agent, wetting agent, emulsifier, buffer, etc. Examples of suitable pharmaceutically acceptable carriers include water, ethanol, polyol (for example glycerol, propanediol, polyethylene glycol, etc.) dextrose, vegetable oil (for example olive oil), saline, buffer, buffered saline, and isotonic agent for example saccharide, polyol, sorbitol and sodium chloride.

In addition, another aspect of the present disclosure relates to methods for immunodetection or determination of target antigens, reagents for immunodetection or determination of target antigens, methods for immunodetection or determination of cells expressing target antigens and diagnostic agents for diagnosing diseases related to target antigen-positive cells, which includes the monoclonal antibody or antibody fragment, or fusion protein, or bifunctional fusion protein of the present disclosure (which specifically recognizes and binds target antigen) used as an active ingredient.

The terms "cancer", "cancerous" or "malignant tumor" refer to or describe the physiological condition in mammals that is generally characterized by unregulated cell growth, and are used interchangeably in the present disclosure. Examples of the cancer or malignant tumor include but are not limited to carcinoma, lymphoma, blastoma, sarcoma and leukemia or lymphoid malignancy. More specific examples of the cancer include squamous cell carcinoma, myeloma, small cell lung cancer, non-small cell lung cancer (NSCLC), head and neck squamous cell carcinoma (HNSCC), glioma, Hodgkin's lymphoma, non-Hodgkin's lymphoma, diffuse large B-cell lymphoma (DLBCL), follicular lymphoma, acute lymphoblastic leukemia (ALL), acute myelocytic leukemia (AML), chronic lymphocytic leukemia (CLL), chronic myelocytic leukemia (CML), primary mediastinal large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, pharyngolaryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myelodysplastic tumor, neuroendocrine tumor, Merkel cell carcinoma, testicular cancer and skin cancer.

"Inflammatory disorder" refers to any disease, disorder or syndrome in which an excessive or unregulated inflammatory response results in excessive inflammatory symptoms, host tissue damage or loss of tissue function. "Inflammatory disease" also refers to a pathological state mediated by the chemotactic pooling of leukocytes or neutrophils.

"Inflammation" refers to a local protective response caused by tissue damage or destruction, which is used to destroy, weaken or eliminate (isolate) harmful substances and injured tissues. Inflammation is significantly related to the chemotactic pooling of leukocytes or neutrophils. Inflammation can be caused by pathogenic organisms and viruses as well as non-infectious causes such as trauma or reperfusion or stroke after myocardial infarction, immune response to exogenous antigens, and autoimmune response.

The aforementioned diseases related to target antigen-positive cells can be diagnosed by detecting or measuring cells expressing the target antigen using the monoclonal antibody or antibody fragment of the present disclosure.

In order to detect cells expressing the polypeptide, known immunodetection methods can be used, preferably using immunoprecipitation, fluorescent cell staining, immunohistochemical staining, etc. In addition, fluorescent antibody staining method utilizing the FMAT8100HTS system (Applied Biosystem) can be used.

In the present disclosure, there is no particular limitation on the *in vivo* sample used for detection or measurement of the target antigen, as long as it has the possibility of comprising cells expressing the target antigen, for example histocyte, blood, plasma, serum, pancreatic juice, urine, feces, tissue fluid or culture fluid.

According to the required diagnostic method, the diagnostic agent comprising the monoclonal antibody or antibody fragment thereof of the present disclosure can further comprise reagents for performing antigen-antibody reaction or reagents for detecting the reaction. The reagents used to perform the antigen-antibody reaction include buffer, salt, etc. The reagents used for detection include reagents commonly used in immunodetection or measurement methods, for example labeled second antibodies that recognize the monoclonal antibody, antibody fragment thereof or conjugate thereof, and a substrate corresponding to the label, etc.

The details of one or more embodiments of the present invention are presented in the above specification. Although any methods and materials similar or identical to those described herein can be used in the practice or testing of the present disclosure, the preferred methods and materials are described below. The other features, purposes and advantages of the present disclosure will be apparent through the specification and the claims. In the specification and the claims, unless otherwise clearly indicated in the context, the singular form includes the cases of plural form. Unless otherwise defined, all technical and scientific terms used herein have the general meanings understood by those of ordinary skill in the art to which the present invention belongs. All patents and publications cited in the specification are incorporated by reference. The following examples are provided to more fully illustrate the preferred embodiments disclosed in the present invention. These examples should not be construed as limiting the scope of the present disclosure in any way, and the scope of the present disclosure is defined by the claims.

## Examples

### Example 1. Preparation of CD47 antigen and proteins for detection

UniProt leukocyte surface antigen CD47 (human CD47 protein, Uniprot number: Q08722) was used as a template for CD47. The amino acid sequence of the antigen and proteins for detection involved in the present disclosure were designed, alternatively different tags (such as his tag or Fc, etc.) were fused on the basis of the CD47 protein.

1. The extracellular domain of CD47 protein with His tag (CD47-ECD-His): (SEQ ID NO: 3)

QLLFNKTKSVEFTFCNDTVVIPCFVTNMEAQNTTEVYVKWKFKGRDIYTF
DGALNKSTVPTDFSSAKIEVSQLLKGDASLKMDKSDAVSHTGNYTCEVTELTR
EGETIIELKYRVVSWFSPNEHHHHHH

Note: the underlined part was a 6×his tag.

2. CD47 extracellular domain and human IgGlFc fusion protein (CD47-ECD-Fc) as a detection reagent: (SEQ ID NO: 4)

QLLFNKTKSVEFTFCNDTVVIPCFVTNMEAQNTTEVYVKWKFKGRDIYTFDGA
LNKSTVPTDFSSAKIEVSQLLKGDASLKMDKSDAVSHTGNYTCEVTELTREGET
IIELKYRVVSWFSPNEEPKSSDKTHTCPPCPAPELLGGPSVFLFPPKPKDTLMISR
TPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDK
SRWQQGNVFSCSVMHEALHNHYTQKSLSLSPGK

Note: the underlined part was the human IgG1-Fc moiety.

3. Fusion protein of human SIRPα and human IgGlFc (SIRPα-Fc) as a binding and blocking detection reagent: (SEQ ID NO: 5)

EEELQIIQPDKSVSVAAGESAILHCTITSLFPVGPIQWFRGAGPARVLIYNQR
QGPFPRVTTVSETTKRENMDFSISISNITPADAGTYYCIKFRKGSPDTEFKSGAGT
ELSVRAKPSEPKSSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDW
LNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQG
NVFSCSVMHEALHNHYTQKSLSLSPGK

Note: the underlined part was the human IgG1-Fc moiety.

4. The extracellular domain of SIRPα protein with His tag (SIRPα-His)

EEELQVIQPDKSVSVAAGESAILHCTVTSLIPVGPIQWFRGAGPARELIYNQ

KEGHFPRVTTVSESTKRENMDFSISISNITPADAGTYYCVKFRKGSPDTEFKSGA GTELSVRAKPSHHHHHH (SEQ ID NO: 132).

**Example 2. Purification of CD47 and SIRPα-related recombinant proteins**

1. Purification steps of recombinant proteins with His tag:

The cell expression supernatant was centrifuged at high speed to remove impurities, the buffer was replaced with PBS, and imidazole was added to a final concentration of 5 mM. The nickel column was equilibrated with PBS solution comprising 5 mM imidazole, and the column volume was washed 2 to 5 times. The supernatant sample after replacement was loaded onto the IMAC column. The column was washed with PBS solution comprising 5 mM imidazole until the A280 reading dropped to baseline. Then the chromatography column was washed with PBS + 10 mM imidazole to remove non-specifically bound protein impurities, and the effluent was collected. The target protein was then eluted with PBS solution comprising 300 mM imidazole, and the elution peak was collected. The collected eluate was concentrated and further purified by gel chromatography Superdex200 (GE, 28-9893-35), and the mobile phase was PBS. The multimer peak was removed and the elution peak was collected. The obtained protein was identified by electrophoresis, peptide map and LC-MS, and then aliquoted for use.

The obtained CD47-ECD-His (SEQ ID NO: 3) with His tag was used as an immunogen or detection reagent for the antibody of the present disclosure. CD47-ECD-His could also be used as an immunogen to stimulate mouse immunity after coupling reaction with KLH protein by *in vitro* chemical methods.

2. Purification steps of CD47-ECD-Fc and SIRPα-Fc fusion protein:

The cell expression supernatant was centrifuged at high speed to remove impurities, and the supernatant was subjected to MabSelect Sure (GE, 17-5438-01) affinity chromatography. The MabSelect Sure column was first regenerated with 0.1 M NaOH, washed with pure water and then equilibrated with PBS. After the binding of the supernatant, the column was washed with PBS until the A280 reading dropped to the baseline. The target protein was eluted with 0.1 M acetate buffer at pH 3.5, and neutralized with 1 M Tris-HCl. The eluted sample was properly concentrated and then further purified by PBS-balanced gel chromatography Superdex200 (GE, 28-9893-35). The collection tubes with the target protein were pooled and concentrated to an appropriate concentration.

This method was used to purify the CD47-ECD-Fc (SEQ ID NO: 4) and SIRPα-Fc (SEQ ID NO: 5) fusion protein. This method could also be used to purify the humanized antibody proteins involved in the present disclosure.

**Example 3. Construction and expression of anti-PD-L1 humanized antibody (IgG4-S228P form)**

The light and heavy chain variable regions of the anti-PD-L1 antibody were modified from the anti-PD-L1 antibody of WO2017084495A1, its sequence and related properties recorded in the PCT application with application number PCT/CN2019/070982, the entire content of which is incorporated into the present application.

Anti PD-L1 antibody 9-2 H5L11:

9-2 H5 heavy chain variable region (SEQ ID NO: 6)

QVQLQESGPGLVKPSQTLSLTCTVSGGSISDGSAYWSWIRQHPGKGLEYIGFISR AGSTYNTPSLKGRVTISRDTSKNQFSLKLSSVTAADTAVYYCARSGGWLAPFDY WGRGTLVTVSS

9-2 L11 light chain variable region (SEQ ID NO: 7)

DIVMTQSPDSLAVSLGERATINCKSSQSLFYHSNQKHSLAWYQQKPGQPPKLLIYGASTRESGVPDRFSGSGSGTDFTLTISSLQAEDVAVYYCQQYYGYPYTFGGGTKVEIK

Anti PD-L1 antibody 24D5 H12L61:

24D5 H12 heavy chain variable region (SEQ ID NO: 8)

QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSS

24D5 L61 light chain variable region (SEQ ID NO: 9)

DIVLTQSPASLAVSPGQRATITCRASESVSIHGTHLMHWYQQKPGQPPKLLIYAASNLESGVPARFSGSGSGTDFTLTINPVEAEDTANYYCQQSFEDPLTFGQGTKLEIK

Note: The underlined CDRs in the light and heavy chain variable regions derived from the above antibodies 9-2 and 24D5 were the CDRs defined by Kabat numbering criteria.

Table 5. Antibody CDRs defined by Kabat numbering criteria

| Antibod y | Chain | CDR1 | CDR2 | CDR3 |
|---|---|---|---|---|
| 9-2 H5L11 | Heavy chain HCD R | DGSAYWS (SEQ ID NO: 97) | FISRAGSTYNTPSLKG (SEQ ID NO: 98) | SGGWLAPFDY (SEQ ID NO: 99) |
| | Light chain LCDR | KSSQSLFYHSNQKHSLA (SEQ ID NO: 100) | GASTRES (SEQ ID NO: 101) | QQYYGYPYT (SEQ ID NO: 102) |
| 24D5 H12L61 | Heavy chain HCD R | SYWMH (SEQ ID NO: 103) | RITPSSGFAMYNEKFKN (SEQ ID NO: 104) | GGSSYDYFDY (SEQ ID NO: 105) |
| | Light chain LCDR | RASESVSIHGTHLMH (SEQ ID NO: 106) | AASNLES (SEQ ID NO: 107) | QQSFEDPLT (SEQ ID NO: 108) |

Primers were desiged; each humanized antibody VH/VK gene fragment was constructed by PCR, and then the homologous recombination with the expression vector pHr (with signal peptide and constant region gene (CH1-FC/CL) fragment) was carried out to construct full-length antibody expressing vector VH-CH1-FC-pHr/VK-CL-pHr.

The sequence of IgG4-S228P heavy chain constant region was as follows: (SEQ ID NO: 10)

ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK

The sequence of IgG1 heavy chain constant region was as follows: (SEQ ID NO: 11)

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

The sequence of the antibody light chain (Kappa chain) constant region was as follows: (SEQ ID NO: 12)

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

The constructed full-length antibody was as follows:
Anti PD-L1 IgG4 antibody h1830

h1830 heavy chain (SEQ ID NO: 13)

QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLG

h1830 light chain (SEQ ID NO: 14)

DIVMTQSPDSLAVSLGERATINCKSSQSLFYHSNQKHSLAWYQQKPGQPPKLLIY GASTRESGVPDRFSGSGSGTDFTLTISSLQAEDVAVYYCQQYYGYPYTFGGGTK VEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR

GEC

Anti PD-L1 IgG4 antibody h1830G1
h1830G1 heavy chain: (SEQ ID NO: 15)

QVQLQESGPGLVKPSQTLSLTCTVSGGSISDGSAYWSWIRQHPGKGLEYIGFISR AGSTYNTPSLKGRVTISRDTSKNQFSLKLSSVTAADTAVYYCARSGGWLAPFDY WGRGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKC KVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHE ALHNHYTQKSLSLSPGK

h1830G1 light chain (same as h1830 light chain, SEQ ID NO: 14):

DIVMTQSPDSLAVSLGERATINCKSSQSLFYHSNQKHSLAWYQQKPGQPPKLLIY GASTRESGVPDRFSGSGSGTDFTLTISSLQAEDVAVYYCQQYYGYPYTFGGGTK VEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC

PD-L1 antibody h1831:
h1831 heavy chain (SEQ ID NO: 16)

QVQLVQSGAEVKKPGASVKVSCKASGYTFTSYWMHWVRQAPGQGLEWMGRI TPSSGFAMYNEKFKNRVTMTRDTSTSTVYMELSSLRSEDTAVYYCARGGSSYD YFDYWGQGTTVTVSSASTKGPSVFPLAPCSRSTSESTAALGCLVKDYFPEPVTV SWNSGALTSGVHTFPAVLQSSGLYSLSSVVTVPSSSLGTKTYTCNVDHKPSNTK VDKRVESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFY PSDIAVEWESNGQPENNYKTTPPVLDSDGSFFLYSRLTVDKSRWQEGNVFSCSV MHEALHNHYTQKSLSLSLG

h1831 light chain (SEQ ID NO: 17)

DIVLTQSPASLAVSPGQRATITCRASESVSIHGTHLMHWYQQKPGQPPKLLIYAA
SNLESGVPARFSGSGSGTDFTLTINPVEAEDTANYYCQQSFEDPLTFGQGTKLEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

Anti PD-L1 IgG1 antibody h1831G1
h1830G1 heavy chain: (SEQ ID NO: 18)

QVQLVQSGAEVKKPGASVKVSCKASGYTFTSYWMHWVRQAPGQGLEW
MGRITPSSGFAMYNEKFKNRVTMTRDTSTSTVYMELSSLRSEDTAVYYCARGGS
SYDYFDYWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVF
SCSVMHEALHNHYTQKSLSLSPGK

h1831G1 light chain (same as h1831 light chain, SEQ ID NO: 17)

DIVLTQSPASLAVSPGQRATITCRASESVSIHGTHLMHWYQQKPGQPPKLLIYAA
SNLESGVPARFSGSGSGTDFTLTINPVEAEDTANYYCQQSFEDPLTFGQGTKLEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC.

**Example 4. Screening and preparation of SIRPγ mutants**

**4.1 Construction of the affinity maturation phage library of SIRPγ and screening of the library**

In order to obtain CD47 receptors with high affinity, affinity maturation was performed on the CD47 receptor SIRPγ D1 domain through the phage display platform technology. The affinity maturation phage library directed to the CD47 binding domain was designed and prepared based on the wild-type SIRPγ, and screened for new SIRPγ mutants. The specific sequence of the wild-type SIRPγ D1 domain was as follows:

DNA coding sequence of wild-type SIRPγ peptide: (SEQ ID NO: 19)

GAGGAGGAGCTACAGATGATTCAGCCTGAGAAGCTCCTGTTGGTCACA GTTGGAAAGACAGCCACTCTGCACTGCACTGTGACCTCCCTGCTTCCCGTGG GACCCGTCCTGTGGTTCAGAGGAGTTGGACCAGGCCGGGAATTAATCTACA ATCAAAAAGAAGGCCACTTCCCCAGGGTAACAACAGTTTCAGACCTCACAA AGAGAAACAACATGGACTTTTCCATCCGCATCAGTAGCATCACCCCAGCAG ATGTCGGCACATACTACTGTGTGAAGTTTCGAAAAGGGAGCCCTGAGAACG TGGAGTTTAAGTCTGGACCAGGCACTGAGATGGCTTTGGGTGCCAAACCCT CT

Wild-type SIRPγ peptide: (SEQ ID NO: 20)

EEELQMIQPEKLLLVTVGKTATLHCTVTSLLPVGPVLWFRGVGPGRELIYN QKEGHFPRVTTVSDLTKRNNMDFSIRISSITPADVGTYYCVKFRKGSPENVEFKS GPGTEMALGAKPS.

Construction of the phage library: degenerate primers were designed, and the designed mutant amino acids were introduced into the SIRPγ phage mutant library by PCR, with the size of each library of about $10^9$.

Screening of the SIRPγ phagemutant library: the packaged SIRPγ phage mutant library ($1 \times 10^{12}$ -$1 \times 10^{13}$) and 100 μl streptavidin microbeads (Miltenyi Biotec, Auburn, CA) were added to 1 ml of phosphate buffered saline (abbreviated as MPBS) comprising 2% skimmed milk and incubated at room temperature for 1 hour, placed on a magnetic stand, and the supernatant was collected. 10 μg/ml biotinylated human CD47-ECD-his protein (purchased from Sino Biological) was added to the supernatant and incubated for 1 hour at room temperature. Then 100 μl of streptavidin-coated magnetic beads (preincubated with 1 ml MPBS) were added and incubated for 1 hour at room temperature. The sample was loaded on the magnetic stand system for sorting, and the supernatant was removed. 1 ml PBST (phosphate buffer comprising 0.1% Tween-20) was added and turned over several times. Fresh washing solution was added after complete removal of supernatant, the step was repeated 11 times to remove unbound mutants, and 0.5 ml elution solution (50 μl 10 mg/ml trypsin stock solution added into 450 μl PBS) was added. The sample was shaken for 15 min at room temperature, placed on a magnetic stand, and the supernatant was transfered into a new EP tube. TG1 was seeded into the 2YT medium and expanded until when the bacteria density was OD600=0.4. 1.75 ml of TG1 (OD600=0.4) was added to each tube, and 250 μl of eluted phage was added, incubated in a 37°C water bath for 30 min, and spread on plates with gradient dilution for testing the titer. The remaining TG1 solution was centrifuged, spread on plates and incubated overnight at 37°C.

The biotinylated human CD47-ECD-his protein antigen was used for SIRPγ phage mutant library, and after 2-3 rounds of MACS screening (streptavidin magnetic beads, Invitrogen), phage mutant monoclones with higher affinity than the wild-type SIRPγ were finally obtained and subjected to sequencing verification. The sequenced clones were compared and analyzed. After removing redundant sequences, the non-redundant sequences were converted into PDL1-CD47 bifunctional fusion protein for expression in mammalian cell.

### 4.2 Construction of the affinity maturation yeast library of SIRPγ and screening of the library

In order to obtain CD47 receptors with higher affinity, affinity maturation of the CD47 receptor SIRPγ-D1 domain was carried out by yeast display platform technology. The affinity maturation yeast library directed to the CD47-binding domain was designed and prepared on the basis of SIRPγ-D1, and screened for new SIRPγ mutants.

Construction of the yeast library: degenerate primers were designed, and the designed mutant amino acids were introduced into the SIRPγ mutant library by PCR, with the size of each library of about $10^9$. The constructed yeast library was verified for its diversity by the second-generation sequencing method.

In the first round of screening, about $5\times10^{10}$ cells from the SIRPγ mutant library and 10 μg/ml biotinylated human CD47-Fc protein were incubated in 50 ml phosphate buffer (referred to as PBSA) comprising 0.1% bovine serum albumin (BSA) at room temperature for 1 hour. Then, the mixture was washed three times with 0.1% PBSA to remove unbound antibody fragments. Then, 100 μl of streptavidin beads (Milenyi Biotec, Auburn, CA) were added to the SIRPγ mutant library that were bound with the biotinylated human CD47-Fc protein, and were loaded on the AutoMACS system for sorting. Library cells with a high affinity for CD47-Fc were collected and then amplified in SDCAA culture medium (20 g dextrose, 6.7 g Difco yeast nitrogen source-free of amino acids, 5 g Bacto casein amino acids, 5.4 g $Na_2HPO_4$ and 8.56 g $NaH_2PO_4 8 \cdot H_2O$, dissolved in 1 L of distilled water) at 30°C, 250 rpm for 24 hours. Then, the culture was induced in SGCAA culture medium (20 g galactose, 6.7 g Difco yeast nitrogen source-free of amino acids, 5 g Bacto casein amino acids, 5.4 g $Na_2HPO_4$ and 8.56 g $NaH_2PO_4 \cdot H_2O$, dissolved in 1 L of distilled water) at 20°C, 250 rpm for 18 hours. The obtained enriched library was subjected to the second round of screening for binding with biotinylated recombinant human CD47-Fc. In order to ensure sufficient diversity of the antibody library for the second and/or third round of screening, a size of the library which is 100-fold of the previous round was used as the number of input cells.

For the third and fourth rounds of screening, the library cells from the previous round were incubated with 1 μg/ml biotinylated human CD47-Fc protein and 10 μg/ml Mouse Anti-cMyc antibody (9E10, sigma) in 0.1% PBSA at room temperature for 1 hour. The mixture was washed three times with 0.1% PBSA to remove unbound antibody fragments. Goat anti-mouse-Alexa488 (A-11001, life technologies) and Strepavidin-PE (S-866, Life technologies) were added and incubated at 4°C for 1 hour. The mixture was washed three times with 0.1% PBSA to remove unbound antibody fragments. Finally, SIRPγ mutants with high affinity were screened by FACS screening (BD FACSAriaTM FUSION).

The biotinylated human CD47-Fc antigen was used for SIRPγ mutant library; 2 to 3 rounds of MACS screening (streptavidin magnetic beads, Invitrogen) and 2 to 3 rounds of FACS screening (BD FACSAriaTM FUSION) were performed. About 400 yeast monoclones were then selected for culturing and induced expression. The binding of yeast monoclones to human CD47-Fc antigen was detected by using FACS (BD FACSCanto II), and yeast monoclones with higher affinity than the wild-type SIRPγ were selected and subjected to sequencing verification. The sequenced clones were compared and analyzed. After removing redundant sequences, the non-redundant sequences were converted into PDL1-CD47 bifunctional fusion protein for expression in mammalian cell.

After screening, the SIRPγ peptide variants finally obtained were as follows:

S58 (SEQ ID NO: 21)

EEELQMIQPEKLLLVTVGETATLHCTVTSLLPVGPVLWFRGVGPGRELIYMS GRGHFPRVTTVSDLTKRNNMDFSIRISSITPADVGTYYCVKFRKGSPEDVEFKSG PGTEMALGAKPS

S79 (SEQ ID NO: 22)

EEELQMIQPEKLLLVTVGETATLHCTVTSLWPVGPVLWFRGVGPGRELIYR TGTGRFPRVTTVSDLTKRNNMDFSIRISSITPADVGTYYCVKFRKGSPEDVEFKS GPGTEVALGAKPS

S15 (SEQ ID NO: 23)

EEELQMIQPEKLLLVTVGETATLHCTVTSLLPVGPVLWFRGVGPGRELIYVS GRGHFPRVTTVSDLTKRENRDFSIRISSITPADVGTYYCVKFRKGSPEDVEFKSGP GTEMALGAKPS

S12 (SEQ ID NO: 24)

EEELQMIQPEKLLLVTVGETATLHCTVTSLLPVGPVLWFRGVGPGRELIYVS GRGHFPRVTTVSDLTKRENKDFSIRISSITPADVGTYYCVKFRKGSPEDVEFKSGP GTEMALGAKPS

S19 (SEQ ID NO: 25)

EEELQMIQPEKLLLVTVGETATLHCTVTSLLPVGPVLWFRGVGPGRELIYVS GRGHFPRVTTVSDLTKRNNRDFSIRISSITPADVGTYYCVKFRKGSPEDVEFKSGP GTEMALGAKPS

S85 (SEQ ID NO:26)

EEELQMIQPEKLLLVTVGETATLHCTVTSLLPVGPVLWFRGVGPGRELIYVS GRGHFPRVTTVSDLTKRNNKDFSIRISSITPADVGTYYCVKFRKGSPEDVEFKSG PGTEMALGAKPS

S37 (SEQ ID NO:27)

EEELQMIQPEKLLLVTVGETATLHCTVTSLLPVGPVLWFRGVGPGRELIYMS GRGHFPRVTTVSDLTKRNNKDFSIRISSITPADVGTYYCVKFRKGSPEDVEFKSG PGTEMALGAKPS

S38 (SEQ ID NO: 28)

EEELQMIQPEKLLLVTVGETATLHCTVTSLLPVGPVLWFRGVGPGRELIYFS GRGHFPRVTTVSDLTKRNNMDFSIRISSITPADVGTYYCVKFRKGSPEDVEFKSG PGTEMALGAKPS

S22 (SEQ ID NO: 29)

EEELQMIQPEKLLLVTVGETATLHCTVTSLLPVGPVLWFRGVGPGRELIYIS GRGHFPRVTTVSDLTKRNNMDFSIRISSITPADVGTYYCVKFRKGSPEDVEFKSG PGTEMALGAKPS

S29 (SEQ ID NO: 30)

EEELQMIQPEKLLLVTVGETATLHCTVTSLLPVGPVLWFRGVGPGRELIYLS GRGHFPRVTTVSDLTKRNNMDFSIRISSITPADVGTYYCVKFRKGSPEDVEFKSG PGTEMALGAKPS

S34 (SEQ ID NO: 31)

EEELQMIQPEKLLLVTVGETATLHCTVTSLLPVGPVLWFRGVGPGRELIYRS GRGHFPRVTTVSDLTKRNNMDFSIRISSITPADVGTYYCVKFRKGSPEDVEFKSG PGTEMALGAKPS

S41 (SEQ ID NO: 32)

EEELQMIQPEKLLLVTVGETATLHCTVTSLLPVGPVLWFRGVGPGRELIYVSGRGHFPRVTTVSDLTKRNNMDFSIRISSITPADVGTYYCVKFRKGSPEDVEFKSGPGTEMALGAKPS

S42 (SEQ ID NO: 33)

EEELQMIQPEKLLLVTVGETATLHCTVTSLLPVGPVLWFRGVGPGRELIYMSGRGHFPRVTTVSDLTKRNNIDFSIRISSITPADVGTYYCVKFRKGSPEDVEFKSGPGTEMALGAKPS

S43 (SEQ ID NO: 34)

EEELQMIQPEKLLLVTVGETATLHCTVTSLLPVGPVLWFRGVGPGRELIYMSGRGHFPRVTTVSDLTKRNNRDFSIRISSITPADVGTYYCVKFRKGSPEDVEFKSGPGTEMALGAKPS

S44 (SEQ ID NO:35)

EEELQMIQPEKLLLVTVGETATLHCTVTSLLPVGPVLWFRGVGPGRELIYMSGRGHFPRVTTVSDLTKRNNVDFSIRISSITPADVGTYYCVKFRKGSPEDVEFKSGPGTEMALGAKPS

S45 (SEQ ID NO:36)

EEELQMIQPEKLLLVTVGETATLHCTVTSLLPVGPVLWFRGVGPGRELIYMSGRGHFPRVTTVSDLTKRDNMDFSIRISSITPADVGTYYCVKFRKGSPEDVEFKSGPGTEMALGAKPS

S46 (SEQ ID NO:37)

EEELQMIQPEKLLLVTVGETATLHCTVTSLLPVGPVLWFRGVGPGRELIYMSGRGHFPRVTTVSDLTKRENKDFSIRISSITPADVGTYYCVKFRKGSPEDVEFKSGPGTEMALGAKPS

S47 (SEQ ID NO:38)

EEELQMIQPEKLLLVTVGETATLHCTVTSLLPVGPVLWFRGVGPGRELIYMSGRGHFPRVTTVSDLTKRENKDFSIRISSITPADVGTYYCVKFRKGSPEDVEFKSGPGTEVALGAKPS

S48 (SEQ ID NO:39)

EEELQMIQPEKLLLVTVGETATLHCTVTSLLPVGPVLWFRGVGPGRELIYRS GRGHFPRVTTVSDLTKRNNKDFSIRISSITPADVGTYYCVKFRKGSPEDVEFKSG PGTEMALGAKPS

S49 (SEQ ID NO:40)

EEELQMIQPEKLLLVTVGETATLHCTVTSLLPVGPVLWFRGVGPGRELIYRS GRGHFPRVTTVSDLTKRENKDFSIRISSITPADVGTYYCVKFRKGSPEDVEFKSGP GTEMALGAKPS.

**Example 5. Construction and expression of PD-L1-CD47 bifunctional fusion protein**

The obtained anti PD-L1 antibodies were linked to SIRPγ to form fusion proteins, and the PD-L1-CD47 bifunctional fusion proteins were obtained by expression and purification by conventional methods.

Table 6. Sequences of PD-L1-CD47 bifunctional fusion proteins

| Protein | Heavy chain + linker (if present) + SIRP γ variant | Light chain |
|---|---|---|
| h1830-S58 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP | DIVMTQSPDS LAVSLGERATI NCKSSQSLFY HSNQKHSLA WYQQKPGQP PKLLIYGAST RESGVPDRFS |

| | | |
|---|---|---|
| | SVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYMSGRGHFPRVTTVSDLTKRNNMDFSIRISSITPADVGTYYCVKFRKGSPEDVEFKSGPGTEMALGAKPS<br>(SEQ ID NO: 41) | GSGSGTDFTLTISSLQAEDVAVYYCQQYYGYPYTFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC<br>(SEQ ID NO:14,the same as that of h1830 light chain) |
| h1830-S15 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYVSGRGHFPRVTTVSDLTKRENRDFSIRISSITPADVGTYYCVKFRKGSPEDVEFKSGPGTEMALGAKPS<br>(SEQ ID NO: 42) | |
| h1830-S12 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYVSGRGHFPRVTTVSDLTKRENKDFSIRISSITPADVGTYYCVKFRKGSPEDVEFKSGPGTEMALGAKPS<br>(SEQ ID NO: 43) | |
| h1830-S19 | QVQLQESGPGLVKPSQTLSLTCTVSGGSISDGSA | |

| | | |
|---|---|---|
| | YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYVSGRGHFPRVTTVSDLTKRNNRDFSI RISSITPADVGTYYCVKFRKGSPEDVEFKSGPGTE MALGAKPS (SEQ ID NO: 44) | |
| h1830-S85 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYVSGRGHFPRVTTVSDLTKRNNKDFSI RISSITPADVGTYYCVKFRKGSPEDVEFKSGPGTE MALGAKPS (SEQ ID NO: 45) | |
| h1830-S37 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ | |

| | | |
|---|---|---|
| | PEKLLLVTVGETATLHCTVTSLLPVGPVLWFRGVGPGRELIYMSGRGHFPRVTTVSDLTKRNNKDFSIRISSITPADVGTYYCVKFRKGSPEDVEFKSGPGTEMALGAKPS<br>(SEQ ID NO: 46) | |
| h1830-19-S79 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGSGGGGEEELQMIQPEKLLLVTVGETATLHCTVTSLWPVGPVLWFRGVGPGRELIYRTGTGRFPRVTTVSDLTKRNNMDFSIRISSITPADVGTYYCVKFRKGSPEDVEFKSGPGTEVALGAKPS<br>(SEQ ID NO: 47) | |
| h1830-S79 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLWPVGPVLWFRGVGPGRELIYRTGTGRFPRVTTVSDLTKRNNMDFSIRISSITPADVGTYYCVKFRKGSPEDVEFKSGPGTEVALGAKPS<br>(SEQ ID NO: 48) | |
| h1830G1-19-S79 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPSSKSTSGGTAALGCLVKDYFPEPVTVSWNSGALTSGVHTFPAVLQSSGLYSLSSVVTVPSSSLGTQTYIC | |

| | | |
|---|---|---|
| | NVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGKGGGGGSGGGGSGGGGSGGGGEEELQMIQPEKLLLVTVGETATLHCTVTSLWPVGPVLWFRGVGPGRELIYRTGTGRFPRVTTVSDLTKRNNMDFSIRISSITPADVGTYYCVKFRKGSPEDVEFKSGPGTEVALGAKPS (SEQ ID NO: 49) | |
| h1830-S38 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYFSGRGHFPRVTTVSDLTKRNNMDFSIRISSITPADVGTYYCVKFRKGSPEDVEFKSGPGTEMALGAKPS (SEQ ID NO: 50) | |
| h1830-S22 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVS | |

| | | |
|---|---|---|
| | VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYISGRGHFPRVTTVSDLTKRNNMDFSIR ISSITPADVGTYYCVKFRKGSPEDVEFKSGPGTE MALGAKPS (SEQ ID NO:51) | |
| h1830-S29 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYLSGRGHFPRVTTVSDLTKRNNMDFSI RISSITPADVGTYYCVKFRKGSPEDVEFKSGPGTE MALGAKPS (SEQ ID NO:52) | |
| h1830-S34 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG | |

| | | |
|---|---|---|
| | FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYRSGRGHFPRVTTVSDLTKRNNMDFSI RISSITPADVGTYYCVKFRKGSPEDVEFKSGPGTE MALGAKPS (SEQ ID NO:53) | |
| h1830-S41 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYVSGRGHFPRVTTVSDLTKRNNMDFSI RISSITPADVGTYYCVKFRKGSPEDVEFKSGPGTE MALGAKPS (SEQ ID NO:54) | |
| h1830-S42 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT | |

| | | |
|---|---|---|
| | QKSLSLSLGGGGGSGGGGSGGGGGGEEELQMIQ PEKLLLVTVGETATLHCTVTSLLPVGPVLWFRGV GPGRELIYMSGRGHFPRVTTVSDLTKRNNIDFSIR ISSITPADVGTYYCVKFRKGSPEDVEFKSGPGTE MALGAKPS (SEQ ID NO:55) | |
| h1830-S43 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYMSGRGHFPRVTTVSDLTKRNNRDFSI RISSITPADVGTYYCVKFRKGSPEDVEFKSGPGTE MALGAKPS (SEQ ID NO:56) | |
| h1830-S44 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV | |

| | | |
|---|---|---|
| | GPGRELIYMSGRGHFPRVTTVSDLTKRNNVDFSI RISSITPADVGTYYCVKFRKGSPEDVEFKSGPGTE MALGAKPS (SEQ ID NO:57) | |
| h1830-S45 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYMSGRGHFPRVTTVSDLTKRDNMDFSI RISSITPADVGTYYCVKFRKGSPEDVEFKSGPGTE MALGAKPS (SEQ ID NO:58) | |
| h1830-S46 | QVQLQESGPGLVKPSQTLSLTCTVSGGSISDGSA YWSWIRQHPGKGLEYIGFISRAGSTYNTPSLKGR VTISRDTSKNQFSLKLSSVTAADTAVYYCARSGG WLAPFDYWGRGTLVTVSSASTKGPSVFPLAPCS RSTSESTAALGCLVKDYFPEPVTVSWNSGALTSG VHTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGGGGGSGGGGSGGGGGGEEELQMIQ PEKLLLVTVGETATLHCTVTSLLPVGPVLWFRGV GPGRELIYMSGRGHFPRVTTVSDLTKRENKDFSI RISSITPADVGTYYCVKFRKGSPEDVEFKSGPGTE | |

| | | |
|---|---|---|
| | MALGAKPS (SEQ ID NO:59) | |
| h1830-S47 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYMSGRGHFPRVTTVSDLTKRENKDFSIRISSITPADVGTYYCVKFRKGSPEDVEFKSGPGTEVALGAKPS (SEQ ID NO:60) | |
| h1830-S48 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYRSGRGHFPRVTTVSDLTKRNNKDFSIRISSITPADVGTYYCVKFRKGSPEDVEFKSGPGTEMALGAKPS (SEQ ID NO:61) | |

| | | |
|---|---|---|
| h1830-S49 | QVQLQESGPGLVKPSQTLSLTCTVSGGSISDGSAYWSWIRQHPGKGLEYIGFISRAGSTYNTPSLKGRVTISRDTSKNQFSLKLSSVTAADTAVYYCARSGGWLAPFDYWGRGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYRSGRGHFPRVTTVSDLTKRENKDFSIRISSITPADVGTYYCVKFRKGSPEDVEFKSGPGTEMALGAKPS (SEQ ID NO:62) | |
| h1831-19-S58 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGSGGGGEEELQMIQPEKLLLVTVGETATLHCTVTSLLPVGPVLWFRGVGPGRELIYMSGRGHFPRVTTVSDLTKRNNMDFSIRISSITPADVGTYYCVKFRKGSPEDVEFKSGPGTEMALGAKPS (SEQ ID NO: 63) | DIVLTQSPASLAVSPGQRATITCRASESVSIHGTHLMHWYQQKPGQPPKLLIYAASNLESGVPARFSGSGSGTDFTLTINPVEAEDTANYYCQQSFEDPLTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:17 , the same as that of h1831 light |
| h1831-S15 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGT | |

| | | |
|---|---|---|
| | KTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGEEELQMIQPEKLLLVTVGETATLHCTVTSLLPVGPVLWFRGVGPGRELIYVSGRGHFPRVTTVSDLTKRENRDFSIRISSITPADVGTYYCVKFRKGSPEDVEFKSGPGTEMALGAKPS (SEQ ID NO: 64) | chain) |
| h1831-S12 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGEEELQMIQPEKLLLVTVGETATLHCTVTSLLPVGPVLWFRGVGPGRELIYVSGRGHFPRVTTVSDLTKRENKDFSIRISSITPADVGTYYCVKFRKGSPEDVEFKSGPGTEMALGAKPS (SEQ ID NO: 65) | |
| h1831-S19 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFN | |

| | | |
|---|---|---|
| | STYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGEEELQMIQPEKLLLVTVGETATLHCTVTSLLPVGPVLWFRGVGPGRELIYVSGRGHFPRVTTVSDLTKRNNRDFSIRISSITPADVGTYYCVKFRKGSPEDVEFKSGPGTEMALGAKPS (SEQ ID NO:66) | |
| h1831-S85 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGEEELQMIQPEKLLLVTVGETATLHCTVTSLLPVGPVLWFRGVGPGRELIYVSGRGHFPRVTTVSDLTKRNNKDFSIRISSITPADVGTYYCVKFRKGSPEDVEFKSGPGTEMALGAKPS (SEQ ID NO:67) | |
| h1831-S37 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPV | |

| | | |
|---|---|---|
| | LDSDGSFFLYSRLTVDKSRWQEGNVFSCSVMHE ALHNHYTQKSLSLSLGGGGGSGGGGSGGGGGG EEELQMIQPEKLLLVTVGETATLHCTVTSLLPVG PVLWFRGVGPGRELIYMSGRGHFPRVTTVSDLT KRNNKDFSIRISSITPADVGTYYCVKFRKGSPEDV EFKSGPGTEMALGAKPS (SEQ ID NO: 68) | |
| h1831-19-S37 | QVQLVQSGAEVKKPGASVKVSCKASGYTFTSY WMHWVRQAPGQGLEWMGRITPSSGFAMYNEK FKNRVTMTRDTSTSTVYMELSSLRSEDTAVYYC ARGGSSYDYFDYWGQGTTVTVSSASTKGPSVFP LAPCSRSTSESTAALGCLVKDYFPEPVTVSWNSG ALTSGVHTFPAVLQSSGLYSLSSVVTVPSSSLGTK TYTCNVDHKPSNTKVDKRVESKYGPPCPPCPAPE FLGGPSVFLFPPKPKDTLMISRTPEVTCVVVDVS QEDPEVQFNWYVDGVEVHNAKTKPREEQFNST YRVVSVLTVLHQDWLNGKEYKCKVSNKGLPSSI EKTISKAKGQPREPQVYTLPPSQEEMTKNQVSLT CLVKGFYPSDIAVEWESNGQPENNYKTTPPVLDS DGSFFLYSRLTVDKSRWQEGNVFSCSVMHEALH NHYTQKSLSLSLGGGGGSGGGGSGGGGSGGGG EEELQMIQPEKLLLVTVGETATLHCTVTSLLPVG PVLWFRGVGPGRELIYMSGRGHFPRVTTVSDLT KRNNKDFSIRISSITPADVGTYYCVKFRKGSPEDV EFKSGPGTEMALGAKPS (SEQ ID NO: 109) | |
| h1831-19-S79 | QVQLVQSGAEVKKPGASVKVSCKASGYTFTSY WMHWVRQAPGQGLEWMGRITPSSGFAMYNEK FKNRVTMTRDTSTSTVYMELSSLRSEDTAVYYC ARGGSSYDYFDYWGQGTTVTVSSASTKGPSVFP LAPCSRSTSESTAALGCLVKDYFPEPVTVSWNSG ALTSGVHTFPAVLQSSGLYSLSSVVTVPSSSLGTK TYTCNVDHKPSNTKVDKRVESKYGPPCPPCPAPE FLGGPSVFLFPPKPKDTLMISRTPEVTCVVVDVS QEDPEVQFNWYVDGVEVHNAKTKPREEQFNST YRVVSVLTVLHQDWLNGKEYKCKVSNKGLPSSI EKTISKAKGQPREPQVYTLPPSQEEMTKNQVSLT CLVKGFYPSDIAVEWESNGQPENNYKTTPPVLDS DGSFFLYSRLTVDKSRWQEGNVFSCSVMHEALH NHYTQKSLSLSLGGGGGSGGGGSGGGGSGGGG EEELQMIQPEKLLLVTVGETATLHCTVTSLWPVG PVLWFRGVGPGRELIYRTGTGRFPRVTTVSDLTK RNNMDFSIRISSITPADVGTYYCVKFRKGSPEDV EFKSGPGTEVALGAKPS (SEQ ID NO: 69) | |
| h1831-S38 | QVQLVQSGAEVKKPGASVKVSCKASGYTFTSY WMHWVRQAPGQGLEWMGRITPSSGFAMYNEK FKNRVTMTRDTSTSTVYMELSSLRSEDTAVYYC | |

| | | |
|---|---|---|
| | ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGG<br>EEELQMIQPEKLLLVTVGETATLHCTVTSLLPVG<br>PVLWFRGVGPGRELIYFSGRGHFPRVTTVSDLTK<br>RNNMDFSIRISSITPADVGTYYCVKFRKGSPEDV<br>EFKSGPGTEMALGAKPS<br>(SEQ ID NO:70) | |
| h1831-S22 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGG<br>EEELQMIQPEKLLLVTVGETATLHCTVTSLLPVG<br>PVLWFRGVGPGRELIYISGRGHFPRVTTVSDLTK<br>RNNMDFSIRISSITPADVGTYYCVKFRKGSPEDV<br>EFKSGPGTEMALGAKPS<br>(SEQ ID NO:71) | |
| h1831-S29 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT | |

| | | |
|---|---|---|
| | KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGGG<br>EEELQMIQPEKLLLVTVGETATLHCTVTSLLPVG<br>PVLWFRGVGPGRELIYLSGRGHFPRVTTVSDLTK<br>RNNMDFSIRISSITPADVGTYYCVKFRKGSPEDV<br>EFKSGPGTEMALGAKPS<br>(SEQ ID NO:72) | |
| h1831-S34 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGGG<br>EEELQMIQPEKLLLVTVGETATLHCTVTSLLPVG<br>PVLWFRGVGPGRELIYRSGRGHFPRVTTVSDLTK<br>RNNMDFSIRISSITPADVGTYYCVKFRKGSPEDV<br>EFKSGPGTEMALGAKPS<br>(SEQ ID NO:73) | |
| h1831-S41 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN | |

| | | |
|---|---|---|
| | STYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYVSGRGHFPRVTTVSDLTKRNNMDFSIRISSITPADVGTYYCVKFRKGSPEDVEFKSGPGTEMALGAKPS (SEQ ID NO:74) | |
| h1831-S42 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGGGGGSGGGGSGGGGGGEEELQMIQPEKLLLVTVGETATLHCTVTSLLPVGPVLWFRGVGPGRELIYMSGRGHFPRVTTVSDLTKRNNIDFSIRISSITPADVGTYYCVKFRKGSPEDVEFKSGPGTEMALGAKPS (SEQ ID NO:75) | |
| h1831-S43 | QVQLVQSGAEVKKPGASVKVSCKASGYTFTSYWMHWVRQAPGQGLEWMGRITPSSGFAMYNEKFKNRVTMTRDTSTSTVYMELSSLRSEDTAVYYCARGGSSYDYFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPV | |

| | | |
|---|---|---|
| | LDSDGSFFLYSRLTVDKSRWQEGNVFSCSVMHE<br>ALHNHYTQKSLSLSLGGGGGSGGGGSGGGGGG<br>EEELQMIQPEKLLLVTVGETATLHCTVTSLLPVG<br>PVLWFRGVGPGRELIYMSGRGHFPRVTTVSDLT<br>KRNNRDFSIRISSITPADVGTYYCVKFRKGSPEDV<br>EFKSGPGTEMALGAKPS<br>(SEQ ID NO:76) | |
| h1831-S44 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGGG<br>EEELQMIQPEKLLLVTVGETATLHCTVTSLLPVG<br>PVLWFRGVGPGRELIYMSGRGHFPRVTTVSDLT<br>KRNNVDFSIRISSITPADVGTYYCVKFRKGSPEDV<br>EFKSGPGTEMALGAKPS<br>(SEQ ID NO:77) | |
| h1831-S45 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGGG<br>EEELQMIQPEKLLLVTVGETATLHCTVTSLLPVG | |

| | | |
|---|---|---|
| | PVLWFRGVGPGRELIYMSGRGHFPRVTTVSDLT<br>KRDNMDFSIRISSITPADVGTYYCVKFRKGSPED<br>VEFKSGPGTEMALGAKPS<br>(SEQ ID NO:78) | |
| h1831-S46 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGG<br>EEELQMIQPEKLLLVTVGETATLHCTVTSLLPVG<br>PVLWFRGVGPGRELIYMSGRGHFPRVTTVSDLT<br>KRENKDFSIRISSITPADVGTYYCVKFRKGSPEDV<br>EFKSGPGTEMALGAKPS<br>(SEQ ID NO:79) | |
| h1831-S47 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGG<br>EEELQMIQPEKLLLVTVGETATLHCTVTSLLPVG<br>PVLWFRGVGPGRELIYMSGRGHFPRVTTVSDLT<br>KRENKDFSIRISSITPADVGTYYCVKFRKGSPEDV<br>EFKSGPGTEVALGAKPS | |

| | | |
|---|---|---|
| | (SEQ ID NO:80) | |
| h1831-S48 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGG<br>EEELQMIQPEKLLLVTVGETATLHCTVTSLLPVG<br>PVLWFRGVGPGRELIYRSGRGHFPRVTTVSDLTK<br>RNNKDFSIRISSITPADVGTYYCVKFRKGSPEDVE<br>FKSGPGTEMALGAKPS<br>(SEQ ID NO:81) | |
| h1831-S49 | QVQLVQSGAEVKKPGASVKVSCKASGYTFTSY<br>WMHWVRQAPGQGLEWMGRITPSSGFAMYNEK<br>FKNRVTMTRDTSTSTVYMELSSLRSEDTAVYYC<br>ARGGSSYDYFDYWGQGTTVTVSSASTKGPSVFP<br>LAPCSRSTSESTAALGCLVKDYFPEPVTVSWNSG<br>ALTSGVHTFPAVLQSSGLYSLSSVVTVPSSSLGT<br>KTYTCNVDHKPSNTKVDKRVESKYGPPCPPCPA<br>PEFLGGPSVFLFPPKPKDTLMISRTPEVTCVVVD<br>VSQEDPEVQFNWYVDGVEVHNAKTKPREEQFN<br>STYRVVSVLTVLHQDWLNGKEYKCKVSNKGLP<br>SSIEKTISKAKGQPREPQVYTLPPSQEEMTKNQV<br>SLTCLVKGFYPSDIAVEWESNGQPENNYKTTPPV<br>LDSDGSFFLYSRLTVDKSRWQEGNVFSCSVMHE<br>ALHNHYTQKSLSLSLGGGGGSGGGGSGGGGG<br>EEELQMIQPEKLLLVTVGETATLHCTVTSLLPVG<br>PVLWFRGVGPGRELIYRSGRGHFPRVTTVSDLTK<br>RENKDFSIRISSITPADVGTYYCVKFRKGSPEDVE<br>FKSGPGTEMALGAKPS<br>(SEQ ID NO:82) | |

The following proteins were also prepared and purified by conventional methods as positive or negative controls.

**Anti-CD47 antibody hu5F9 (the sequence was from** US09017675B**)**

**Hu5F9 heavy chain** (SEQ ID NO: 83)

QVQLVQSGAEVKKPGASVKVSCKASGYTFTNYNMHWVRQAPGQRLEWMGTI
YPGNDDTSYNQKFKDRVTITADTSASTAYMELSSLRSEDTAVYYCARGGYRAM
DYWGQGTLVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK;

**Hu5F9 light chain** (SEQ ID NO: 84)

DIVMTQSPLSLPVTPGEPASISCRSSQSIVYSNGNTYLGWYLQKPGQSPQLLI
YKVSNRFSGVPDRFSGSGSGTDFTLKISRVEAEDVGVYYCFQGSHVPYTFG
QGTKLEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQG
LSSPVTKSFNRGEC;

**SIRPα-CV** (synthesized referring to Engineered SIRPα Variants as Immunotherapeutic Adjuvants to Anticancer Antibodies, Science. 2013 Jul 5;341(6141):88-91, SEQ ID NO: 85)

EEELQIIQPDKSVLVAAGETATLRCTITSLFPVGPIQWFRGAGPGRVLIYNQ
RQGPFPRVTTVSDTTKRNNMDFSIRIGNITPADAGTYYCIKFRKGSPDDVEF
KSGAGTELSVRAKPSEPKSSDKTHTCPPCPAPELLGGPSVFLFPPKPKDTLM
ISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK;

TTI-621: (sequence from WO2014094122A1, SEQ ID NO: 133)

EEELQVIQPDKSVSVAAGESAILHCTVTSLIPVGPIQWFRGAGPARELIYNQ KEGHFPRVTTVSESTKRENMDFSISISNITPADAGTYYCVKFRKGSPDTEFK SGAGTELSVRAKPSDKTHTCPPCPAPELLGGPSVFLFPPKPKDTLMISRTPE VTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSRDEL TKNQVSLTCLVKGFYPSDIAVEWESNGQPENNYKTTPPVLDSDGSFFLYSK LTVDKSRWQQGNVFSCSVMHEALHNHYTQKSLSLSPGK;

**S58-Fc** (SEQ ID NO: 86)

EEELQMIQPEKLLLVTVGETATLHCTVTSLLPVGPVLWFRGVGPGRELIYMSGR GHFPRVTTVSDLTKRNNMDFSIRISSITPADVGTYYCVKFRKGSPEDVEFKSGPG TEMALGAKPSESKYGPPCPPCPAPEFLGGPSVFLFPPKPKDTLMISRTPEVTCVV VDVSQEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRWQEGN VFSCSVMHEALHNHYTQKSLSLSLGK;

**Anti-CD47 antibody Hu167-IgG4 AA** (prepared according to the method disclosed in patent application WO2018095428A1)

**Hu167-IgG4 AA heavy chain** (SEQ ID NO:87)

QVQLVQSGAEVKKPGASVKVSCKASGYTFTSYWMHWVRQAPGQGLEWMGNI DPSDSETHYNQKFKDRVTMTRDTSISTAYMELSRLRSDDTAVYYCARWGYLGR SAMDYWGQGTTVTVSSASTKGPSVFPLAPCSRSTSESTAALGCLVKDYFPEPVT VSWNSGALTSGVHTFPAVLQSSGLYSLSSVVTVPSSSLGTKTYTCNVDHKPSNT KVDKRVESKYGPPCPPCPAPEAAGGPSVFLFPPKPKDTLMISRTPEVTCVVVDVS QEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYP SDIAVEWESNGQPENNYKTTPPVLDSDGSFFLYSRLTVDKSRWQEGNVFSCSVM HEALHNHYTQKSLSLSLGK;

**Hu167-IgG4 AA light chain** (SEQ ID NO: 88)

DVQITQSPSSLSASVGDRVTITCRTSKSISKFLAWYQQKPGKAPKLLIYSGSTLQS GVPSRFSGSGSGTDFTLTISSLQPEDFATYYCQQHNEYPWTFGGGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC;

**S37-Fc** (SEQ ID NO: 110)

EEELQMIQPEKLLLVTVGETATLHCTVTSLLPVGPVLWFRGVGPGRELIYMSGR
GHFPRVTTVSDLTKRNNKDFSIRISSITPADVGTYYCVKFRKGSPEDVEFKSGPGT
EMALGAKPSESKYGPPCPPCPAPEFLGGPSVFLFPPKPKDTLMISRTPEVTCVVV
DVSQEDPEVQFNWYVDGVEVHNAKTKPREEQFNSTYRVVSVLTVLHQDWLNG
KEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFS
CSVMHEALHNHYTQKSLSLSLGK.

**Antibody h1831K**

The h1831 antibody was subjected to CDR mutation modification, and 36 mutants were obtained, and finally the N53K (position determined according to the Kabat numbering criteria) mutant h1831K was selected. The h1831 light chain LCDR2 was mutated from AAS**N**LES to AAS**K**LES, to obtain a new antibody h1831K.

h1831 light chain: (SEQ ID NO: 111)

DIVLTQSPASLAVSPGQRATITCRASESVSIHGTHLMHWYQQKPGQPPKLLIYAA
SKLESGVPARFSGSGSGTDFTLTINPVEAEDTANYYCQQSFEDPLTFGQGTKLEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC;

wherein LCDR1 was RASESVSIHGTHLMH (SEQ ID NO: 106), LCDR2 was AASKLES (SEQ ID NO: 112), LCDR3 was QQSFEDPLT (SEQ ID NO: 108). h1831K light chain variable region (SEQ ID NO: 113)

DIVLTQSPASLAVSPGQRATITCRASESVSIHGTHLMHWYQQKPGQPPKLLIYAA
SKLESGVPARFSGSGSGTDFTLTINPVEAEDTANYYCQQSFEDPLTFGQGTKLEIK;

h1831K heavy chain (same as h1831 heavy chain sequence, SEQ ID NO: 16)

QVQLVQSGAEVKKPGASVKVSCKASGYTFTSYWMHWVRQAPGQGLEWMGRI
TPSSGFAMYNEKFKNRVTMTRDTSTSTVYMELSSLRSEDTAVYYCARGGSSYD
YFDYWGQGTTVTVSSASTKGPSVFPLAPCSRSTSESTAALGCLVKDYFPEPVTV
SWNSGALTSGVHTFPAVLQSSGLYSLSSVVTVPSSSLGTKTYTCNVDHKPSNTK
VDKRVESKYGPPCPPCPAPEFLGGPSVFLFPPKPKDTLMISRTPEVTCVVVDVSQ
EDPEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSV
MHEALHNHYTQKSLSLSLG;

>h1831K-19-S37 heavy chain (same as h1831-19-S37 heavy chain sequence, SEQ ID NO: 109)

QVQLVQSGAEVKKPGASVKVSCKASGYTFTSYWMHWVRQAPGQGLEWMGRI TPSSGFAMYNEKFKNRVTMTRDTSTSTVYMELSSLRSEDTAVYYCARGGSSYD YFDYWGQGTTVTVSSASTKGPSVFPLAPCSRSTSESTAALGCLVKDYFPEPVTV SWNSGALTSGVHTFPAVLQSSGLYSLSSVVTVPSSSLGTKTYTCNVDHKPSNTK VDKRVESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKGFYPS

DIAVEWESNGQPENNYKTTPPVLDSDGSFFLYSRLTVDKSRWQEGNVFSCSVMH EALHNHYTQKSLSLSLGGGGGSGGGGSGGGGSGGGGEEELQMIQPEKLLLVTV GETATLHCTVTSLLPVGPVLWFRGVGPGRELIYMSGRGHFPRVTTVSDLTKRNN KDFSIRISSITPADVGTYYCVKFRKGSPEDVEFKSGPGTEMALGAKPS;

> h1831K-19-S37 light chain (same as h1831K light chain sequence, SEQ ID NO: 111)

DIVLTQSPASLAVSPGQRATITCRASESVSIHGTHLMHWYQQKPGQPPKLLIYAA SKLESGVPARFSGSGSGTDFTLTINPVEAEDTANYYCQQSFEDPLTFGQGTKLEIK RTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC;

S79-Fc (SEQ ID NO:114)

EEELQMIQPEKLLLVTVGETATLHCTVTSLWPVGPVLWFRGVGPGRELIYR TGTGRFPRVTTVSDLTKRNNMDFSIRISSITPADVGTYYCVKFRKGSPEDVE FKSGPGTEVALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S15-Fc (SEQ ID NO:115)

EEELQMIQPEKLLLVTVGETATLHCTVTSLLPVGPVLWFRGVGPGRELIYVS GRGHFPRVTTVSDLTKRENRDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S12-Fc (SEQ ID NO:116)

EEELQMIQPEKLLLVTVGETATLHCTVTSLLPVGPVLWFRGVGPGRELIYVS GRGHFPRVTTVSDLTKRENKDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S19-Fc (SEQ ID NO:117)

EEELQMIQPEKLLLVTVGETATLHCTVTSLLPVGPVLWFRGVGPGRELIYVS

GRGHFPRVTTVSDLTKRNNRDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S85-Fc (SEQ ID NO:118)

EEELQMIQPEKLLLVTVGETATLHCTVTSLLPVGPVLWFRGVGPGRELIYVS GRGHFPRVTTVSDLTKRNNKDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S38-Fc (SEQ ID NO:119)

EEELQMIQPEKLLLVTVGETATLHCTVTSLLPVGPVLWFRGVGPGRELIYFS GRGHFPRVTTVSDLTKRNNMDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S22-Fc (SEQ ID NO:120)

EEELQMIQPEKLLLVTVGETATLHCTVTSLLPVGPVLWFRGVGPGRELIYIS GRGHFPRVTTVSDLTKRNNMDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S29-Fc (SEQ ID NO:121)

EEELQMIQPEKLLLVTVGETATLHCTVTSLLPVGPVLWFRGVGPGRELIYLS GRGHFPRVTTVSDLTKRNNMDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ

EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S34-Fc (SEQ ID NO:122)

EEELQMIQPEKLLLVTVGETATLHCTVTSLLPVGPVLWFRGVGPGRELIYRS GRGHFPRVTTVSDLTKRNNMDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S41-Fc (SEQ ID NO:123

EEELQMIQPEKLLLVTVGETATLHCTVTSLLPVGPVLWFRGVGPGRELIYVS GRGHFPRVTTVSDLTKRNNMDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S42-Fc (SEQ ID NO:124)

EEELQMIQPEKLLLVTVGETATLHCTVTSLLPVGPVLWFRGVGPGRELIYMS GRGHFPRVTTVSDLTKRNNIDFSIRISSITPADVGTYYCVKFRKGSPEDVEFK SGPGTEMALGAKPSESKYGPPCPPCPAPEFLGGPSVFLFPPKPKDTLMISRT PEVTCVVVDVSQEDPEVQFNWYVDGVEVHNAKTKPREEQFNSTYRVVSV LTVLHQDWLNGKEYKCKVSNKGLPSSIEKTISKAKGQPREPQVYTLPPSQE EMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S43-Fc (SEQ ID NO:125)

EEELQMIQPEKLLLVTVGETATLHCTVTSLLPVGPVLWFRGVGPGRELIYMS GRGHFPRVTTVSDLTKRNNRDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S44-Fc (SEQ ID NO:126)

EEELQMIQPEKLLLVTVGETATLHCTVTSLLPVGPVLWFRGVGPGRELIYMS GRGHFPRVTTVSDLTKRNNVDFSIRISSITPADVGTYYCVKFRKGSPEDVEF KSGPGTEMALGAKPSESKYGPPCPPCPAPEFLGGPSVFLFPPKPKDTLMISR TPEVTCVVVDVSQEDPEVQFNWYVDGVEVHNAKTKPREEQFNSTYRVVS VLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFL YSRLTVDKSRWQEGNVFSCSVMHEALHNHYTQKSLSLSLGK;

S45-Fc (SEQ ID NO:127)

EEELQMIQPEKLLLVTVGETATLHCTVTSLLPVGPVLWFRGVGPGRELIYMSGR GHFPRVTTVSDLTKRDNMDFSIRISSITPADVGTYYCVKFRKGSPEDVEFKSGPG TEMALGAKPSESKYGPPCPPCPAPEFLGGPSVFLFPPKPKDTLMISRTPEVTCVV VDVSQEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRWQEGN VFSCSVMHEALHNHYTQKSLSLSLGK;

S46-Fc (SEQ ID NO:128)

EEELQMIQPEKLLLVTVGETATLHCTVTSLLPVGPVLWFRGVGPGRELIYMSGR
GHFPRVTTVSDLTKRENKDFSIRISSITPADVGTYYCVKFRKGSPEDVEFKSGPGT
EMALGAKPSESKYGPPCPPCPAPEFLGGPSVFLFPPKPKDTLMISRTPEVTCVVV
DVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNV
FSCSVMHEALHNHYTQKSLSLSLGK;

S47-Fc (SEQ ID NO:129)

EEELQMIQPEKLLLVTVGETATLHCTVTSLLPVGPVLWFRGVGPGRELIYMSGR
GHFPRVTTVSDLTKRENKDFSIRISSITPADVGTYYCVKFRKGSPEDVEFKSGPGT
EVALGAKPSESKYGPPCPPCPAPEFLGGPSVFLFPPKPKDTLMISRTPEVTCVVV
DVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNV
FSCSVMHEALHNHYTQKSLSLSLGK;

S48-Fc (SEQ ID NO:130)

EEELQMIQPEKLLLVTVGETATLHCTVTSLLPVGPVLWFRGVGPGRELIYRSGRG
HFPRVTTVSDLTKRNNKDFSIRISSITPADVGTYYCVKFRKGSPEDVEFKSGPGTE
MALGAKPSESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNG

KEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFS
CSVMHEALHNHYTQKSLSLSLGK;

S49-Fc (SEQ ID NO:131)

EEELQMIQPEKLLLVTVGETATLHCTVTSLLPVGPVLWFRGVGPGRELIYRSGRG
HFPRVTTVSDLTKRENKDFSIRISSITPADVGTYYCVKFRKGSPEDVEFKSGPGTE
MALGAKPSESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNG
KEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFS
CSVMHEALHNHYTQKSLSLSLGK.

The IgG4 control as a negative control was an antibody against a target related to neither PD-L1 nor CD47. IgG4-Fc and IgG1-Fc comprise only the Fc segment, respectively, and do not comprise any antigen-specific variable region segments.

**Test examples**

**Test example 1. ELISA experiment of PD-L1-CD47 bifunctional fusion protein binding to CD47-his protein**

The binding of the PD-L1-CD47 bifunctional fusion protein was detected by measuring the amount of the bifunctional fusion protein bound to the human CD47 or cyno CD47 immobilized on the ELISA plate. CD47-ECD-His (see Table 7) was diluted with PBS to 1 μg/ml and coated on a 96-well ELISA plate. After washing and blocking of the plate, bifunctional fusion protein samples of different concentrations were added, the plate was washed again, and then horseradish peroxidase-goat anti-human (H+L) antibody (Jackson, CAT#109-035-088) was added. The plate was washed again and tetramethyl benzidine solution was added for color development. Finally the stop solution was added, the OD450 was measured on a microplate reader and EC50 values were calculated. The results were shown in Table 8-1 and Table 8-2.

Table 7. Sources of CD47 of different germlines

| CD47 type | MW/kDa | Cat.No. | Lot.No. | Manufacturer |
|---|---|---|---|---|
| Human CD47 | 15.2 | 12283-H08H | N/A | S.B |
| Cyno CD47 | 15.8 | CD7-C52H1 | 2171b-76VF1-K9 | ACROBiosystems |

Table 8-1. Binding ELISA of PD-L1-CD47 bifunctional fusion protein to human CD47 and cyno CD47

| Antibody name | Human CD47-his binding EC50 (ng/ml) | Cyno CD47-his binding EC50 (ng/ml) |
|---|---|---|
| h1830-S37 | 53.79 | 51.55 |
| h1830-S85 | 71.19 | 66.72 |
| h1830-S19 | 67.35 | 63.43 |
| h1830-S12 | 51.11 | 78.94 |
| h1830-S15 | 64.09 | 72.26 |
| h1831-19-S58 | 46.95 | 59.99 |
| h1830-S58 | 40.79 | 61.79 |
| h1830-19-S79 | 90.53 | 43.53 |
| Hu167 IgG4AA | 70.32 | 31.73 |
| TTI-621 | 30.17 | 293.4 |
| hu5F9 | 37.4 | 30.33 |
| S58-Fc | 24.79 | 15.43 |
| IgG4 | No binding | No binding |

Table 8-2. Binding ELISA of PD-L1-CD47 bifunctional fusion protein to human CD47 and cyno CD47

| **Sample name** | **Binding ELISA (EC50, ng/ml)** | |
|---|---|---|
| | **Human CD47** | **Cyno CD47** |
| h1830-S37 | 40.74 | 30.57 |
| h1830-S85 | 84.66 | 64.08 |
| h1831K-19-S37 | 60.91 | 33.01 |
| S58-Fc | 25.55 | 11.96 |
| S37-Fc | 22.8 | 11.4 |
| IgG4 | No binding | No binding |

The results showed that each PD-L1-CD47 bifunctional fusion protein has a very strong affinity with free human CD47 protein, and also has a very strong cross-affinity with cyno CD47.

**Test example 2. ELISA experiment of PD-L1-CD47 bifunctional fusion protein binding to PD-L1-his protein**

The binding of the PD-L1-CD47 bifunctional fusion protein was detected by meauring the amount of the antibody bound with PD-L1 of different species immobilized on the ELISA plate. PD-L1-his antigen of different germlines (see Table 9) was diluted with PBS to 1 μg/ml and coated on a 96-well ELISA plate (Costar, CAT#3590). After washing and blocking of the plate, PD-L1-CD47 bifunctional fusion protein samples of different concentrations were added, the plate was washed again, and then horseradish peroxidase-goat anti-human (H+L) antibody (Jackson, CAT#109-035-088) was added. The plate was washed again and tetramethyl benzidine solution was added for color development. Finally the stop solution was added, the OD450 was measured on a microplate reader and EC50 values were calculated. The results were shown in Table 10.

Table 9. Sources of PD-L1 of different germlines

| PD-L1 type | MW/kDa | Cat.No. | Lot.No. | Manufacturer |
|---|---|---|---|---|
| hPD-L1-His | 26.8 | 10084-H08H | LC11SE1203 | S.B |
| Cyno PD-L1-His | 26.7 | 90251-C08H | LC10DE1308 | S.B |
| mPD-L1 | 26.3 | 50010-M08H | LC10NO0102 | S.B |

Table 10. Binding ELISA of PD-L1-CD47 bifunctional fusion protein to PD-L1 of different species

| Antibody name | Human PD-L1-his binding (OD450) EC50 (ng/ml) | Mouse PD-L1-his binding (OD450) EC50 (ng/ml) | Cyno PD-L1-his binding (OD450) EC50 (ng/ml) |
|---|---|---|---|
| h1830-S37 | 99.28 | 32 | 176.8 |
| h1830-S85 | 141.8 | 27.48 | 206.6 |
| h1830-S19 | 166.5 | 34.05 | 179.4 |
| h1830-S12 | 146.2 | 34.28 | 178.1 |
| h1830-S15 | 127.8 | 38.63 | 232.1 |
| h1831-19-S58 | 94.68 | No binding | 237.3 |
| h1831-19-S79 | 97.64 | No binding | 214.5 |
| h1830-S58 | 160.6 | 33.52 | 320.1 |
| h1830-19-S79 | 95.16 | 58.2 | 254.1 |
| HRP00052 | 163.1 | No binding | 195.5 |
| h1830 | 131.3 | 28.94 | 230.2 |
| h1831 | 129.2 | No binding | 221.7 |
| h1831K-19-S37 | 46 | No binding | 97.21 |
| IgG4 control | No binding | No binding | No binding |

The results showed that each PD-L1-CD47 bifunctional fusion protein has a very strong affinity with free human PD-L1 protein, and also a very strong cross-affinity with cyno PD-L1. PD-L1-CD47 bifunctional fusion protein comprising h1830 antibody also has a very strong cross-affinity with mouse PD-L1.

**Test example 3. Blocking effct of PD-L1-CD47 bifunctional fusion protein on the binding of PD-L1/PD1 and of PD-L1/B7.1**

PD-L1-Fc (prepared in-house) was diluted with PBS to 1 μg/ml, added to a 96-well plate at 100 μl/well, and

placed at 4°C for 16 h-20 h. The PBS buffer was removed from the 96-well plate, which was washed with PBST (pH 7.4 PBS comprising 0.05% tween20) buffer for once. PBST/1% milk was added at 120 μl/well and incubated at room temperature for 1 h for blocking. The blocking solution was removed and the plate was washed with PBST buffer for once. 90 μl of the PD-L1-CD47 bifunctional fusion protein to be tested diluted to appropriate concentrations with sample diluent (pH7.4 PBS comprising 5% BSA, 0.05% Tween20) was added, and pre-incubated at 4°C for 1 h. 10× concentration of biotin-labeled PD-1 (Beijing Sino Biological Inc., 10 μg/ml) or B7-1 (Beijing Sino Biological Inc., 10 μg/ml) was add at a volume of 10 μl/well. After shaking and mixing on a shaker, the plate was incubated at 37°C for 1 h. The reaction system was removed and the plate was washed with PBST for 6 times. 100 μl/well Streptavidin-Peroxidase Polymer 1:400 diluted with PBST buffer was added and incubated with shaking at room temperature for 50 min. The plate was washed with PBST for 6 times. 100 μl/well TMB was added and incubated at room temperature for 5-10 min. 100 μl/well 1 M $H_2SO_4$ was added to stop the reaction. OD450 was measured by using NOVOStar on a microplate reader and IC50 value was calculated. The results were shown in Table 11.

Table 11. Blocking ELISA of PD-L1-CD47 bifunctional fusion proteins

| | Protein blocking ELISA | |
|---|---|---|
| Antibody name | Blocking the binding of human PD-L1 -Fc to PD1 (OD450) IC50 (ng/ml) | Blocking the binding of human PD-L1 -Fc to B7-1 (OD450) IC50 (ng/ml) |
| h1830-S37 | 145.6 | 169.2 |
| h1830-S85 | 81.41 | 148.5 |
| h1830-S19 | 51.83 | 34.23 |
| h1830-S12 | 42.91 | 30.39 |
| h1830-S15 | 47.12 | 55.48 |
| h1831-19-S58 | 122.5 | 159.7 |
| h1831-19-S79 | 72.28 | 113.3 |
| h1830-S58 | 102.5 | 126.8 |
| h1830-19-S79 | 64.69 | 98.84 |
| HRP00052 | 32.04 | 30.71 |
| h1830 | 34.96 | 75.25 |
| h1831 | 26.58 | 49.39 |
| IgG4 control | No binding | No binding |

The test results showed that all bifunctional fusion proteins could also effectively block PD-L1/PD-1 and PD-L1/B7.1 pathways.

**Test Example 4. Experiment of PD-L1-CD47 bifunctional fusion proteins binding to human red blood cells**

Fresh healthy human blood was mixed with PBS in equal volume and centrifuged at 300 g for 5 min to obtain cell clusters. Red blood cells were obtained after washing with PBS for 3-5 times. Cells were resuspended in FACS buffer (PBS + 5%BSA) with the cell density adjusted to $2\times10^6$ cells/ml and seeded to a 96-well round bottom plate (3795#, corning) at 100μl/well. Then different concentrations of antibodies and bifunctional fusion proteins were added and incubated at 4°C for 1 hour. After washing twice with FACS buffer (PBS + 2%FBS), the secondary antibody (Alexa 488 goat anti-human IgG antibody (Invitrogen, CAT#A11013)) was added and incubated for 30 min on ice in the dark. Finally, the cells were resuspended after washing twice with FACS buffer. The plate was read in FACS Cantoll.

FACS test results showed that the control antibodies hu5F9 and Hu167 IgG4AA have strong binding ability with natural CD47 on the surface of human red blood cells. Among the bifunctional fusion proteins involved, except the bifunctional fusion proteins comprising S79, S34 and S49 which bind with CD47 on the surface of human red blood cells, other bifunctional fusion proteins have no binding ability with natural CD47 on the surface of human red blood cells, suggesting the safety advantages of the above bifunctional fusion proteins. The results were shown in Figure 2A, Figure 2B and Figure 2C (the experiments performed in Figure 2A, Figure 2B and Figure 2C involve three batches of experiments, with different donor cells).

**Test Example 5. Experiment of PD-L1-CD47 bifunctional fusion proteins binding to tumor cells**

Raji cells were cultured in RPMI medium (Hyclone, CAT#SH30809.01B) (comprising 10% fetal bovine serum). Raji cells at $1 \times 10^6$ cells/ml were blocked with 5% BSA, the bifunctional fusion protein samples were added to a concentration of 10 $\mu$g/ml. After washing twice, Alexa Fluor 488-goat anti-human (H+L) antibody (Invitrogen, CAT#A11013) was added. After washing twice, the fluorescence signal value was read by a flow cytometer.

The FACS test results showed that the PD-L1-CD47 bifunctional fusion proteins involved have a very strong binding ability with the natural CD47 on the surface of Raji cells, which is equivalent to the binding ability of the control antibody Hu5F9. The results were shown in Figure 3.

**Test Example 6. *In vitro* cell-mediated cell phagocytosis (ADCP) experiment of PD-L1-CD47 bifunctional fusion proteins**

PBMC was isolated from fresh human blood, and then CD14+ monocytes were sorted by using Human CD14 MicroBeads (130-050-201#, Miltenyi Biotec). These CD14+ monocytes were differentiated into macrophages by culturing in macrophage differentiation medium (1640 + 10% FBS + 50 ng/ml M-CSF) for 9 days. These monocyte-derived macrophages (MDM) became adhesive and had tentacles. On the day of the experiment, the macrophages were digested with trypsin for 5 min, scraped off gently with a scraper and spread on a 96-well round bottom plate (3795#). Human RBC cells (red blood cells) were labeled with 0.5 $\mu$M CFSE (BD, Catalog number 565082#) in a 37°C water bath for 13 min. After washing twice with PBS, the samples were added to macrophages at the ratio of 5 RBC cells per each macrophage, and PD-L1-CD47 bifunctional fusion proteins were added at various concentrations. The target cells were subjected to phagocytosis for 2.5 hours. After phagocytosis, cells were washed twice with PBS. Then human Fc blocker (564219#, BD) was added according to a certain ratio and placed at room temperature for 10 min to exclude non-specific binding. Subsequently, APC-labeled CD14 antibody (17-0149-42#, Ebioscience) was added. Cells were incubated on ice for 30 min. After the last twice washes, analysis was performed by flow cytometry. Phagocytosis was measured by selecting CFSE+ positive cells in the APC+ positive living cell gate, and then evaluating the percentage of CSFE+ positive cells (see Figure 4).

**Test Example 7. *In vitro* cell-mediated cell phagocytosis (ADCP) experiment of PD-L1-CD47 bifunctional fusion proteins**

PBMC was isolated from fresh human blood, and then CD14+ monocytes were sorted by using Human CD14 MicroBeads (130-050-201#, Miltenyi Biotec). These CD14+ monocytes were differentiated into macrophages by culturing in macrophage differentiation medium (1640 + 10% FBS + 50 ng/ml M-CSF) for 9 days. These monocyte-derived macrophages (MDM) became adhesive and had tentacles. On the day of the experiment, the macrophages were digested with trypsin for 5 min, scraped off gently with a scraper and spread on a 96-well round bottom plate (3795#). Molp-8 cells (Nanjing CoBioer) were labeled with 0.1 $\mu$M CFSE in a 37°C water bath for 13 min. After washing twice with PBS, samples were added to macrophages at the ratio of 5 Molp-8 tumor cells per each macrophage, and PD-L1-CD47 antibodies were added at various concentrations. The target cells were subjected to phagocytosis for 2.5 hours. After phagocytosis, cells were washed twice with PBS. Then human Fc blocker was added according to a certain ratio and placed at room temperature for 10 min to exclude non-specific binding. Subsequently, APC-labeled CD14 antibody was added. Cells were incubated on ice for 30 min. After the last twice washes, analysis was performed by flow cytometry. Phagocytosis was measured by selecting in the APC+ positive living cell gate, and then evaluating the percentage of CSFE+ positive cells (see Figure 5A and Figure 5B).

The results of Test Examples 6 and 7 were shown in Figure 4 and Figures 5A to Figure 5B, showing that the added bifunctional fusion proteins could effectively promote the phagocytosis on tumor cells. However, the bifunctional fusion proteins have no phagocytic effect on red blood cells, suggesting the potential advantages of the bifunctional fusion protein antibodies of the present disclosure in terms of safety. Meanwhile, the control antibody hu5F9 could effectively phagocytose red blood cells.

**Test Example 8. Red blood cell agglutination experiment of PD-L1-CD47 bifunctional fusion proteins**

Fresh healthy human blood was diluted 100 times with PBS (B320#, Shanghai BasalMedia Technologies Co., Ltd.). The diluted whole blood was plated onto a 96-well round bottom plate (3795#, corning) at 30 $\mu$l/well. Then antibodies or bifunctional fusion proteins with different concentration gradients were added in equal volumes. After mixing, the plate was placed at 37°C for 4-6 h. Sedimentation of red blood cells was observed by using a high-content microscope. Cells with no blood coagulation were clear red spots, and cells with blood coagulation appeared diffused.

Each sample was diluted from the first column (0.5 mg/ml) to the 11th column (1:3 dilution). The 12th column was PBS blank wells without antibody.

The results showed (see Figure 6) that under the same conditions, the bifunctional fusion proteins h1830-S37, h1830-S19, h1830-S12, h1830-S15, h1831-19-S58 and h1831-19-S79 did not cause red blood cell agglutination under the different concentrations tested, suggesting the advantages of the bifunctional fusion proteins of the present disclosure in terms of safety.

**Test Example 9. Affinity experiment of PD-L1-CD47 bifunctional fusion proteins detected by BIAcore**

The response signals of bifunctional fusion proteins for different antigens were detected in real time by Biacore T200 instrument by using a Protein A biosensor chip (Cat. # 29127556, GE) to affinity capture IgG, and different antigens (hCD47, cyno CD47, hPD-L1, cyno PD-L1 and mPD-L1, see Test Examples 3 and 4 for the sources) flowed through the surface of the chip, and the binding and dissociation curves were obtained. Once the dissociation of each experimental cycle was completed, the biosensor chip was washed and regenerated with 10 mM Glycine-HCl pH 1.5 buffer. The experimental buffer system was 1×HBS-EP buffer solution (Cat# BR-1001-88, GE). Once the experiment was completed, the data was fit with (1:1) Langmuir model by using the GE Biacore T200 Evaluation version 3.0 software to obtain the affinity values. The results were shown in Table 12-1 and Table 12-2. The results showed that the affinity of all modified SIRPγ peptide variants to human CD47 was dramatically improved, when compared with that of the wild-type SIRPγ peptide.

Table 12-1 Biacore affnity of antibodies to different antigens (KD (M))

| | Human CD47 | Cyno CD47 | Human PD-L1 | Cyno PD-L1 | Mouse PD-L1 |
|---|---|---|---|---|---|
| h1830-S37 | 1.72E-09 | 2.03E-09 | 3.87E-10 | 4.09E-10 | 7.09E-08 |
| h1830-S85 | 3.84E-09 | 4.63E-09 | 3.74E-10 | 3.97E-10 | 8.58E-08 |
| h1830-S19 | 5.60E-09 | 6.55E-09 | 3.99E-10 | 4.24E-10 | 6.86E-08 |
| h1830-S12 | 4.36E-09 | 5.11E-09 | 4.00E-10 | 4.29E-10 | 6.98E-08 |
| h1830-S15 | 4.11E-09 | 4.78E-09 | 3.80E-10 | 4.96E-10 | 7.88E-08 |
| h1831-19-S58 | 1.77E-09 | 1.94E-09 | 8.25E-11 | 8.91E-11 | No binding |
| h1831-19-S79 | 3.83E-09 | 4.63E-09 | 8.74E-11 | 1.21E-10 | No binding |
| h1830-S58 | 1.95E-09 | 2.18E-09 | 3.74E-10 | 3.98E-10 | 7.51E-08 |
| h1830-S79 | 4.06E-09 | 4.90E-09 | 4.25E-10 | 4.55E-10 | 7.75E-08 |
| Hu167 IgG4AA | 6.53E-10 | 1.09E-09 | - | - | - |
| TTI-621 | 4.15E-09 | 1.04E-08 | - | - | - |
| Hu5F9 | 5.05E-10 | 8.50E-10 | - | - | - |
| S58-Fc | 5.51E-10 | 9.74E-10 | - | - | - |
| HRP00052 | - | - | 1.44E-10 | 1.51E-10 | No binding |
| h1830 | - | - | 4.66E-10 | 4.98E-10 | 9.75E-08 |
| h1831 | - | - | 8.06E-11 | 9.19E-11 | No binding |
| h1831-19-S37 | 1.16E-09 | 1.39E-09 | 5.29E-11 | 4.71E-11 | No binding |
| M831K-19-S37 | 1.25E-09 | 1.48E-09 | 5.63E-11 | 4.93E-11 | No binding |
| S37-Fc | 5.24E-10 | 8.93E-10 | - | - | - |

Table 12-2 Biacore affnity of bifunctional fusion proteins to human CD47 (KD (M))

| Bifunctional fusion protein | Biacore (human CD47) |
|---|---|
| | KD (M) |
| h1830-S38 | 1.02E-08 |

(continued)

| Bifunctional fusion protein | Biacore (human CD47) |
|---|---|
| | KD (M) |
| h1830-S22 | 6.30E-09 |
| h1830-S29 | 4.59E-09 |
| h1830-S34 | 2.60E-09 |
| h1830-S41 | 5.05E-09 |
| h1830-S42 | 4.19E-09 |
| h1830-S43 | 2.13E-09 |
| h1830-S44 | 2.99E-09 |
| h1830-S45 | 4.85E-09 |
| h1830-S46 | 1.77E-09 |
| h1830-S47 | 1.94E-09 |
| h1830-S48 | 2.49E-09 |
| h1830-S49 | 3.09E-09 |

**Test Example 10. Effect of PD-L1-CD47 bifunctional fusion proteins on the cell secretion of IFNγ in PBMC-T lymphocyte activation experiment**

In order to study the effect of PD-L1-CD47 bifunctional fusion proteins on the function of human primary T lymphocytes, human peripheral blood mononuclear cells (PBMC) were collected and purified, stimulated with tuberculin (TB) for 5 days *in vitro,* and the secretion level of the cytokine IFNγ was detected. The experimental process is briefly described as follows:
PBMCs were obtained from fresh blood using Ficoll-Hypaque (17-5442-02, GE) by density gradient centrifugation (Stem Cell Technologies), cultured in RPMI 1640 (SH30809.01, GE) culture medium supplemented with 10% (v/v) FBS (10099-141, Gibco) at 37°C and 5% $CO_2$.

The freshly isolated and purified PBMCs were adjusted to a density of $2\times10^6$ cells/ml with RPMI 1640 culture medium. 40 μl tuberculin (97-8800, Synbiotics) was added to 20 mL cell suspension and cultured in a 37°C, 5% $CO_2$ incubator for 5 days. On the 5th day, the aforementioned cultured cells were collected by centrifugation, resuspended in fresh RPMI 1640 culture medium, adjusted to a density of $1.1\times10^6$ cells/ml, and seeded into a 96-well cell culture plate at 90 μl per well. At the same time, gradient diluted antibody samples diluted with PBS (B320, Shanghai BasalMedia Technologies Co., Ltd.) were added at 10 μl per well. The cell culture plate was placed in a 37°C, 5% $CO_2$ incubator and incubated for 3 days. The cell culture plate was taken out and the cell culture supernatant was collected by centrifugation (4000 rpm, 10 min). IFN-γ level was detected by using the ELISA method (human IFN-γ detection kit: EHC102g.96, Neobioscience). Instructions of the reagents were referred to for specific operations.

The results (see Figure 7A to Figure 7E) showed that among the tested molecules, all bifunctional fusion proteins could activate IFN-γ secretion, which was comparable to the control antibody HRP00052.

**Test Example 11. Effect of PD-L1-CD47 bifunctional fusion proteins in mouse colon cancer model MC38/H-11-hCD47**

In this experiment, B-hCD274/hCD47/hSIRPα mice were used and inoculated with artificially modified murine colon cancer MC38 cells: MC38/H-11-hCD47 (tranfected with human PD-L1 and human CD47, with murine CD47 and PDL1 knocked out) to establish the tumor-bearing mouse model, and the *in vivo* inhibition effect of different doses of PD-L1-CD47 bifunctional fusion proteins h1830-S85, SIRPγ protein S58-Fc and PD-L1 monoclonal antibody h1830 on the growth of murine colon cancer transplanted tumor was evaluated. B-hCD274/hCD47/hSIRPα mice were purchased from Biocytogen Experimental Animals, SPF grade; body weight: 22.0±3.0 g; gender: female.

MC38/H-11-hCD47 (#5-4) cells were inoculated subcutaneously into B-hCD274/hCD47/hSIRPα mice at an inoculum of $1\times10^6$ cells/100 μl/mouse. After establishing the tumor-bearing model, the tumor volume was measured and animals with too large and too small body weight and tumor size were excluded. Tumor-bearing mice were randomly divided into 5 groups (n=7) according to tumor volume: PBS control group, h1830-S85 high-dose experimental group, h1830-S85 low-dose experimental group, h1830 experimental group and S58-Fc experimental group. The date of grouping was set as D0.

1) Tumor growth was observed and recorded by using the method of measuring the tumor diameter. At the same time, the body weight of animals were observed and recorded.
2) The tumor diameter and animal body weight were measured twice a week.
3) On the 17th day after administration, the tumors of animals in the PBS control group were relatively large, thus following the principles of animal welfare, the animals in the PBS experimental group were sacrificed; on the 25th day after administration, the remaining animals in the experimental groups were sacrificed.
4) Tumor volume (TV) calculation formula: TV = 1/2 $\times$ a $\times$ $b^2$, wherein a and b represent the long diameter and short diameter of the measured tumor, respectively.
5) Relative tumor growth rate T/C% = (T-T0)/(C-C0) $\times$ 100%
6) Tumor growth inhibition rate TGI% = 1-T/C %

Statistical analysis of the experimental data was performed by using Excel and GraphPad. The animal body weight, tumor volume and tumor weight of each group were all presented as mean ± standard error (Mean ± SEM), and Graphpad Prism 6 software was used for plotting.

This experiment intended to evaluate the inhibitory effect of different doses of PD-L1-CD47 bifunctional fusion proteins on the tumor growth in B-hCD274/hCD47/hSIRPα mouse colon cancer transplanted tumor model. In this experiment, different antibodies or bifunctional fusion proteins were administrated at the same time when grouping.

As shown in the results of Figure 8 and Table 13, different doses of bifunctional fusion protein (h1830-S85) experimental group, PD-L1 monoclonal antibody (h1830) experimental group and SIRPγ protein S58-Fc experimental group all have smaller tumor volumes than the PBS control group; the tumor inhibitory effect of the PD-L1-CD47 bifunctional fusion protein high-dose experimental group is better than the same dose of PD-L1 monoclonal antibody experimental group and SIRPγ protein experimental group; there is a dose-dependent relationship among the experimental groups of different h1830-S85 doses.

During the experiment, there was no significant difference in body weight between the administration group and the control group, and the mice were tolerated to each administered antibody.

Table 13. Tumor inhibitory effect of antibodies or bifunctional fusion proteins on transplanted tumors in mice (TGI%)

| Day | h1830-S85 30mpk | h1830-S85 10mpk | h1830 25mpk | S58-Fc 13.2mpk |
|---|---|---|---|---|
| 4 | 18.43 | 36.60 | 42.98 | 29.45 |
| 7 | 45.80 | 39.39 | 35.93 | 26.04 |
| 11 | 61.48 | 49.07 | 45.22 | 45.40 |
| 14 | 62.23 | 50.67 | 39.72 | 49.63 |
| 18 | 63.22 | 57.19 | 44.48 | 54.25 |

**Test Example 12. Effect of PD-L1-CD47 bifunctional fusion protein in mouse MC38-hPD-L1-hCD47 colon cancer model**

MC38-hPD-L1-hCD47 cells (an MC38 cells transferred with human PD-L1 and human CD47, but with mouse CD47 knocked out) were inoculated into C57/BL-6 mice subcutaneously at $5.8\times10^5$ cells/100 μl/mouse. After establishing the tumor-bearing model, the tumor volume was measured and animals with too large and too small body weight and tumor size were excluded. Tumor-bearing mice were randomly divided into 5 groups (n=7) according to tumor volume: IgG4 control group, h1830-S58 experimental group, HRP00052 experimental group, h1830 experimental group and TTI-621 experimental group. The date of grouping was set as D0. After grouping, each agent was intraperitoneally administered three times a week for a total of 10 times and an administration cycle of 18 days. The tumor-bearing mice were no longer monitored, two days after withdrawal of administration. The tumor volume was measured twice a week, the weight was weighed, and the data was recorded. See the table below for grouping and administration. In this experiment, different antibodies were administrated at the same time when grouping. From the 14th day after administration, the dose of all the experimental groups was reduced to half; from the 25th day after administration, administration was stopped in all experimental groups.

Table 14. Experimental grouping and administration

| Grouping | Dose of agent | Route of administration | Administration cycle |
|---|---|---|---|
| IgG-PBS | 10 mpk | i.p. | q.o.d. |
| h1830-S58 | 12 mpk | i.p. | q.o.d |
| HRP00052 | 10 mpk | i.p. | q.o.d |
| h1830 | 10 mpk | i.p. | q.o.d |
| TTI-621 | 5 mpk | i.p. | q.o.d |
| Note: i.p means intraperitoneal injection, q.o.d means once every other day. | | | |

The animal body weight, tumor volume and tumor weight of each group were all presented as mean ± standard error (Mean ± SEM), and Graphpad Prism 6 and Excel software were used for plotting, and student t test was used for statistical analysis.

$$\text{Tumor volume (TV)} = 1/2 \times L_{long} \times L_{short}^2$$

$$\text{Tumor growth rate T/C\%} = (T\text{-}T0)/(C\text{-}C0) \times 100\%$$

$$\text{Tumor growth inhibition rate \%TGI} = 1\text{- T/C \%}$$

As shown in the results of Figure 9, the tumor volume in the PD-L1-CD47 bifunctional fusion protein h1830-S58 experimental group and PD-L1 monoclonal antibody (h1830) experimental group (which cross-reacts with mouse PD-L1) was smaller than that of the control group and TTI-621 experimental group, and the statistical difference compared with the control group was observed around one week after administration; the TTI-621 experimental group did not show tumor inhibitory effect in this experiment. In the h1830-S58 experimental group, 7 days after administration, the tumor inhibition rate reached 128.51%. By the end of the experiment, the tumor inhibition rate remained at a relatively high level.

After the experiment, the tumor-bearing mice were euthanized, and the tumor was collected and weighed. The results for tumor weight were similar to that of the tumor volume. During the experiment, there was no significant difference in body weight between the administration group and the control group, and the mice were tolerated to each administered antibody.

**Test Example 13. Effect of PD-L1-CD47 bifunctional fusion protein in mouse MC38-hPD-L1 colon cancer model**

MC38-hPD-L1 cells (an MC38 cells transferred with human PD-L1) were inoculated into C57/BL-6 mice subcutaneously at $3.5\times10^5$ cells/100 μl/mouse. After establishing the tumor-bearing model, the tumor volume was measured and animals with too large and too small body weight and tumor size were excluded. Tumor-bearing mice were randomly divided into 5 groups (n=7) according to tumor volume: IgG4 control group, h1830-19-S79 experimental group, h1830G1-19-S79 experimental group, SIRPα-CV experimental group and h1830 experimental group. The date of grouping was set as D0. After grouping, each agent was intraperitoneally administered three times a week for a total of 12 times and an administration cycle of 28 days. The tumor-bearing mice were no longer monitored, two days after withdrawal of administration. The tumor volume was measured twice a week, the weight was weighed, and the data was recorded. See the table below for grouping and administration.

Table 15. Experimental grouping and administration

| Grouping | Dose of agent | Route of administration | Administration cycle |
|---|---|---|---|
| IgG4 control | 10 mpk | i.p. | q.o.d. |
| h1830G1-19-S79 | 12 mpk | i.p. | q.o.d. |
| h1830-19-S79 | 12 mpk | i.p. | q.o.d |
| SIRPa-CV | 5 mpk | i.p. | q.o.d |
| h1830 | 10 mpk | i.p. | q.o.d |

The animal body weight, tumor volume and tumor weight of each group were all presented as mean ± standard error (Mean ± SEM), and Graphpad Prism 5 and Excel software were used for plotting, and student t test was used for statistical analysis.

$$\text{Tumor volume (TV)} = 1/2 \times L_{long} \times L_{short}^2$$

$$\text{Tumor growth rate T/C\%} = (T-T0)/(C-C0) \times 100\%$$

$$\text{Tumor growth inhibition rate \%TGI} = 1- T/C\ \%$$

This experiment intended to evaluate the inhibitory effect of different IgG forms of PD-L1-CD47 bifunctional fusion proteins on the tumor growth in C57/BL-6 mouse colon cancer transplanted tumor model. In this experiment, different antibodies were administrated at the same time when grouping. From the 14th day after administration, the dose in all the experimental groups was reduced to half; from the 25th day after administration, administration was stopped in all experimental groups.

As shown in the results of Figure 10, until the 25th day after administration, the tumor volume in all the bifunctional fusion protein administration group and PD-L1 monoclonal antibody h1830 administration group was smaller than that of the IgG4 control group and SIRPα-CV (TTI-621) experimental group, and there were statistical differences compared with the control group.

On the 25th day after administration, the control group and the SIRPα-CV (TTI-621) experimental group were euthanized due to the relatively large tumor size, while administration was stopped in the remaining experimental groups and the observation was continued. The results showed that the tumor volume in the PD-L1 monoclonal antibody h1830 experimental group showed a trend of rapid increase over time, and the tumor volume in the bifunctional fusion protein h1830-19-S79 and h1830G1-19-S79 experimental groups did not change significantly, and there was also no significant difference between these two different IgG forms of bispecific antibodies.

After the experiment, the tumor-bearing mice were euthanized, and the tumor was collected and weighed. The results for tumor weight were similar to that of the tumor volume. During the experiment, there was no significant difference in body weight between the administration group and the control group, and the mice were tolerated to each administered antibody.

**Test Example 14. Efficacy of PD-L1-CD47 bifunctional fusion proteins on MOLP-8 transplanted tumor nude mice**

Balb/c nude mice were inoculated with MOLP-8 cells ($5\times10^6$ +50% matrigel/mouse) subcutaneously at the right ribs for a total of 120 mice. After 10 d, the average tumor volume reached about 214.89 ± 6.75 $mm^3$. The tumor-bearing mice were randomly divided into 7 groups (n=8): PBS control group, h1830-S37 experimental group, h1830-S58 experimental group, h1831K-19-S37, h1830 experimental group, S37-Fc and Hu167 IgG4AA experimental group. The date of grouping was set as D0. After grouping, each agent was intraperitoneally administered twice a week for 3 consecutive weeks. The tumor volume was measured twice a week, the weight was weighed, and the data was recorded. The animal body weight, tumor volume and tumor weight of each group were all presented as mean ± standard error (Mean ± SEM), and Graphpad Prism 6 and Excel software were used for plotting, and student t test was used for statistical analysis.

$$\text{Calculation formula of tumor volume (V): } V = 1/2 \times L_{long} \times L_{short}^2$$

$$\text{Relative tumor volume (RTV)} = V_T/V_0$$

$$\text{Tumor growth inhibition rate (\%)} = (C_{RTV}-T_{RTV})/\ C_{RTV}(\%)$$

Wherein $V_0$ and $V_T$ were the tumor volume at the start of the experiment and at the end of the experiment, respectively. $C_{RTV}$ and $T_{RTV}$ are the relative tumor volumes of the blank control group (Blank) and the experimental group at the end of the experiment, respectively.

The results of this experiment showed (see Figure 11) that the mice were intraperitoneally injected once every other day for 10 consecutive administrations. Data until the 21st day of the experiment was used for statistics. The PD-L1-CD47 bifunctional fusion protein h1830-S37 (30 mpk) has a tumor inhibition rate of 34.98% ($P<0.05$); the bifunctional fusion protein h1831K-19-S37 (30 mpk) has a tumor inhibition rate of 54.18% ($P<0.01$); h1830 (25 mpk) did not have inhibitory effect on tumor growth.

During the administration, the animals in each group had normal body weights, indicating that the bifunctional fusion proteins have no obvious toxicity and side effects.

**Test Example 15. Blocking effect of PD-L1-CD47 bifunctional fusion proteins on the binding of CD47/SIRPα**

CD47-Fc was diluted with PBS to 1 μg/ml, added to a 96-well plate at 100 μl/well, and placed at 4°C for 16 h-20 h. The PBS buffer was removed from the 96-well plate, which was washed with PBST (pH 7.4 PBS comprising 0.05% tween20) buffer for once. PBST/1% milk was added at 120 μl/well and incubated at room temperature for 1 h for blocking. The blocking solution was removed and the plate was washed with PBST buffer for once. 90 μl of the PD-L1-CD47 bifunctional fusion protein to be tested diluted to appropriate concentrations with sample diluent (pH 7.4 PBS comprising 5% BSA, 0.05% Tween20) was added and pre-incubated at 4°C for 1 h. 10× concentration of biotin-labeled SIRPα-his (10 μg/ml) was added at a volume of 10 μl/well, shaken and mixed well on a shaker, and then incubated at 37°C for 1 h. The reaction system was removed and the plate was washed with PBST for 6 times. 100 μl/well Streptavidin-Peroxidase Polymer 1:400 diluted with PBST buffer was added and incubated with shaking at room temperature for 50 min. The plate was washed with PBST for 6 times. 100 μl/well TMB was added and incubated at room temperature for 5-10 min. 100 μl/well 1 M $H_2SO_4$ was added to stop the reaction. OD450 was measured by using NOVOStar on a microplate reader and IC50 value was calculated. The results were shown in Table 16.

Table 16. Blocking effect of the bispecific antibodies on the binding of CD47/SIRPα

| Sample | IC50 (ng/ml) |
|---|---|
| h1831K-19-S37 | 251.7 |
| S37-Fc | 566 |
| TTI-621 | 25985 |
| Hu5F9 | 263.1 |

The results showed that the bifunctional fusion proteins could effectively block the pathway of CD47 and SIRPα.

**Test Example 16. Efficacy of PD-L1-CD47 bifunctional fusion proteins on human breast cancer cell MDA-MB-231 transplanted tumor**

MDA-MB-231 cells (ATCC) were inoculated subcutaneously at the right rib of NOD/SCID mice at $3\times10^6$ cells/200 μl/mouse (comprising 50% Matrigel). When the average tumor volume of the tumor-bearing mice reached about 145 $mm^3$, mice were randomly divided into 4 groups: PBS, h1831K-19-S37-30mpk, h1831K-19-S37-10mpk, h1831K-25mpk (maintaining equimolar concentration with that of h1831K-19-S37 high dose) with 8 mice in each group. The grouping day was defined as Day0 of the experiment. On Day 0, the PBMCs of two volunteers stimulated with CD3 antibody for 3 days were mixed at a ratio of 1:1, and injected into the mouse tumor tissue at $5\times10^5$ cells/100 μl/mouse. Stimulation of the remaining PBMCs were stopped, and these PBMCs continued to be cultured for 1 week and then intraperitoneally injected into tumor-bearing mice at $5\times10^6$ cells/100 μl/mouse, which was regarded as the first round of injection. By the end of the experiment, a total of two rounds of PBMCs were injected. Starting from Day0, each antibody to be tested was injected intraperitoneally three times a week. The tumor volume and animal weight were monitored twice a week and the data was recorded. When the tumor volume exceeded 1000 $mm^3$ or ulcer was observecd in most tumors or the body weight was reduced by 20%, the tumor-bearing animals were euthanized as the experimental endpoint.

All data were graphed and statistically analyzed by using Excel and GraphPad Prism 5 software.

Calculation formula of tumor volume (V) is: $V=1/2\times a\times b^2$, wherein a and b represent length and width respectively.

Relative tumor proliferation rate T/C (%)=$(T-T_0)/(C-C_0)\times100$, wherein T and C are the tumor volume in the treatment group and the control group at the end of the experiment; $T_0$ and $C_0$ are the tumor volume at the beginning of the experiment.

$$\text{Tumor growth inhibition rate (TGI) (\%)} = 1 - \text{T/C (\%)}.$$

The experimental results showed that in the human breast cancer MDA-MB-231 mouse subcutaneously transplanted tumor model, the PDL1-CD47 bispecific antibody h1831K-19-S37 and the PDL1 monoclonal antibody h1831K both showed good tumor inhibition effects (p<0.001 vs PBS).

The PD-L1-CD47 bispecific antibody h1831K-19-S37 (30, 10 mg/kg) could significantly inhibit the growth of human breast cancer MDA-MB-231 mouse subcutaneously transplanted tumors, and there was a dose-dependency between high and low doses. From 3 days after administration till the end of the experiment (Day23), regardless of high-dose group or the low-dose group, the tumor inhibitory effect of h1831K-19-S37 was always better than that of the high-dose PD-L1 monoclonal antibody control h1831K (25mg/kg) (p<0.001), and there were also statistical differences between high and low doses (p<0.01) (Table 17).

At the endpoint of the experiment, the tumor-bearing mice were euthanized, the tumor was collected and the tumor weight was measured. The results showed that the *ex vivo* tumor weight was in line with the trend of tumor volume. All treatment groups were significantly better than the control group (p<0.001). Both the high and low dose groups of the bispecific antibody h1831K-19-S37 were better than the high dose PDL1 monoclonal antibody control h1831K (25 mg/kg, p<0.001), and there was a dose-dependent effect between the high and low doses of h1831K-19-S37.

The tumor-bearing mice were tolerated to the PDL1-CD47 bispecific antibody and the monoclonal antibody thereof. There was only a slight fluctuation in body weight during the whole administration process, and no obvious weight loss or other symptoms caused by the agent was observed.

Table 17. Inhibitory effect of bispecific antibodies on mice subcutaneous transplanted tumors

| Group | Tumor volume on Day0 | Tumor volume on Day23 | |
|---|---|---|---|
| | Mean±SEM ($mm^3$) | ($mm^3$) | TGI (%) |
| PBS | 145.8±8.9 | 809.7±41.9 | -- |
| h1831K-19-S37-30mpk | 143.7±10.0 | 73.2±16.1*** | 110.62 |
| h1831K-19-S37-10mpk | 143.8±8.5 | 163.2±15.4*** | 97.08 |
| h1831K-25mpk | 144.9±8.4 | 440.4±58.2*** | 55.49 |
| Note: Day0: time of the first administration. *** represents p<0.001 vs PBS, determined by student's T test. | | | |

## Claims

1. A bifunctional fusion protein comprising a SIRPγ peptide variant and an anti-human PD-L1 antibody, the SIRPγ peptide variant being linked to the polypeptide chain of the anti-human PD-L1 antibody directly or indirectly through a linker,
the SIRPγ peptide variant is a SIRPγ peptide variant with a substitution mutation at position N51 relative to the wild-type SIRPγ peptide as shown in SEQ ID NO: 20, preferably, the linker is selected from any one of the group consisiting of SEQ ID NO: 89-96, (GGGGS)n, (GGGES)n and (GKPGS)n, wherein n is an integer of 2 to 7.

2. The bifunctional fusion protein according to claim 1, wherein the carboxyl terminal of the SIRPγ peptide variant is linked to the amino terminal of the heavy chain variable region of the anti-human PD-L1 antibody,

    or the carboxyl terminal of the SIRPγ peptide variant is linked to the amino terminal of the light chain variable region of the anti-human PD-L1 antibody,
    or the carboxyl terminal of the heavy chain of the anti-human PD-L1 antibody is linked to the amino terminal of the SIRPγ peptide variant,
    or the carboxyl terminal of the light chain of the anti-human PD-L1 antibody is linked to the amino terminal of the SIRPγ peptide variant.

3. The bifunctional fusion protein according to claim 1 or 2, wherein the SIRPγ peptide variant further comprises amino acid substitution(s) at one or more positions selected from the group consisiting of K19, K53, N101, L31, Q52, E54, H56, N70, M72 and M112 relative to the wild-type SIRPγ peptide.

4. The bifunctional fusion protein according to any one of claims 1 to 3, wherein the SIRPγ peptide variant with a substitution mutation at position N51 does not substantially bind with CD47 on surface of red blood cells, preferably, the SIRPγ peptide variant with a substitution mutation at position N51 comprises N51F, N51I, N51L, N51M or N51V substitution mutation.

5. The bifunctional fusion protein according to any one of claims 1 to 3, wherein the SIRPγ peptide variant comprises N51R substitution mutation relative to the wild-type SIRPγ peptide as shown in SEQ ID NO: 20.

6. The bifunctional fusion protein according to any one of claims 1 to 5, wherein the SIRPγ peptide variant comprises K19E, K53G and N101D substitution mutations relative to the wild-type SIRPγ peptide as shown in SEQ ID NO: 20;

    preferably, the SIRPγ peptide variant comprises K19E, N51V, Q52S, K53G, E54R, M72K and N101D mutations relative to the wild-type SIRPγ peptide as shown in SEQ ID NO: 20; or
    the SIRPγ peptide variant comprises K19E, N51M, Q52S, K53G, E54R, M72K and N101D mutations relative to the wild-type SIRPγ peptide as shown in SEQ ID NO: 20.

7. The bifunctional fusion protein according to claim 6, wherein the SIRPγ peptide variant further comprises amino acid substitution(s) at one or more positions selected from the group consisiting of M6, V27, L30, V33, V36, L37, V42, E47, L66, T67, V92 and S98.

8. The bifunctional fusion protein according to claim 6, wherein the SIRPγ peptide variant is as shown in SEQ ID NO: 1.

9. The bifunctional fusion protein according to claim 6, wherein the SIRPγ peptide variant is as shown in SEQ ID NO: 2.

10. The bifunctional fusion protein according to claim 6, wherein the SIRPγ peptide variant is as shown in any one of the group consisiting of SEQ ID NO: 21, 22, 23, 24, 25, 26, 27, 28, 29, 30, 31, 32, 33, 34, 35, 36, 37, 38, 39 and 40.

11. The bifunctional fusion protein according to any one of claims 1 to 10, wherein the anti-human PD-L1 antibody is selected from the group consisiting of Avelumab, Atezolizumab, Durvalumab, JS-003, CS-1001, LY-3300054, KD-033, CK-301, CCX-4503, CX-072, KN-035, HRP00052, HRP00049, FAZ-053, GR-1405, KD-005, HLX-20, KL-A167, CBT-502, STI-A1014, REMD-290, BGB-A333, BCD-135 and MCLA-145.

**12.** The bifunctional fusion protein according to any one of claims 1 to 10, wherein the anti-human PD-L1 antibody comprises a heavy chain variable region and a light chain variable region, wherein:

the heavy chain variable region comprises HCDR1, HCDR2 and HCDR3 regions with the same sequence(s) as those in the heavy chain variable region as shown in SEQ ID NO: 6, and
the light chain variable region comprises LCDR1, LCDR2 and LCDR3 regions with the same sequence(s) as those in the light chain variable region as shown in SEQ ID NO: 7;
the heavy chain variable region comprises HCDR1, HCDR2 and HCDR3 regions with the same sequence(s) as those in the heavy chain variable region as shown in SEQ ID NO: 8, and
the light chain variable region comprises LCDR1, LCDR2 and LCDR3 regions with the same sequence(s) as those in the light chain variable region as shown in SEQ ID NO: 9; or
the heavy chain variable region comprises HCDR1, HCDR2 and HCDR3 regions with the same sequence(s) as those in the heavy chain variable region as shown in SEQ ID NO: 8, and
the light chain variable region comprises LCDR1, LCDR2 and LCDR3 regions with the same sequence(s) as those in the light chain variable region as shown in SEQ ID NO: 113;
preferably,
the heavy chain variable region comprises HCDR1, HCDR2 and HCDR3 regions as shown in SEQ ID NO: 97, 98 and 99, respectively, and
the light chain variable region comprises LCDR1, LCDR2 and LCDR3 regions as shown in SEQ ID NO: 100, 101 and 102, respectively; or
the heavy chain variable region comprises HCDR1, HCDR2 and HCDR3 regions as shown in SEQ ID NO: 103, 104 and 105, respectively, and
the light chain variable region comprises LCDR1, LCDR2 and LCDR3 regions as shown in SEQ ID NO: 106, 107 and 108, respectively; or
the heavy chain variable region comprises HCDR1, HCDR2 and HCDR3 regions as shown in SEQ ID NO: 103, 104 and 105, respectively, and
the light chain variable region comprises LCDR1, LCDR2 and LCDR3 regions as shown in SEQ ID NO: 106, 112 and 108, respectively.

**13.** The bifunctional fusion protein according to claim 12, wherein the anti-human PD-L1 antibody comprises a heavy chain variable region and a light chain variable region, wherein:

the heavy chain variable region is as shown in SEQ ID NO: 6, and
the light chain variable region is as shown in SEQ ID NO: 7; or
the heavy chain variable region is as shown in SEQ ID NO: 8, and
the light chain variable region is as shown in SEQ ID NO: 113; or
the heavy chain variable region is as shown in SEQ ID NO: 8, and
the light chain variable region is as shown in SEQ ID NO: 9.

**14.** The bifunctional fusion protein according to claim 12 or 13, wherein the anti-human PD-L1 antibody further comprises a heavy chain constant region and a light chain constant region, preferably, the heavy chain constant region is as shown in SEQ ID NO: 10 or 11, and the light chain constant region is as shown in SEQ ID NO: 12.

**15.** The bifunctional fusion protein according to claim 14, wherein the anti-human PD-L1 antibody comprises a heavy chain and a light chain, wherein the heavy chain is as shown in SEQ ID NO: 13 or 15, and the light chain is as shown in SEQ ID NO: 14; or
the heavy chain is as shown in SEQ ID NO: 16 or 18, and the light chain is as shown in SEQ ID NO: 17 or 111.

**16.** The bifunctional fusion protein according to claim 15, wherein the bifunctional fusion protein comprises a first polypeptide and a second polypeptide, wherein:

the first polypeptide is selected from the polypeptide as shown in any one of the group consisiting of SEQ ID NO: 41, 42, 43, 44, 45, 46, 47, 48, 49, 50, 51, 52, 53, 54, 55, 56, 57, 58, 59, 60, 61 and 62, and the second polypeptide is selected from the polypeptide as shown in SEQ ID NO: 14; or
the first polypeptide is selected from the polypeptide as shown in any one of the group consisiting of SEQ ID NO: 63, 64, 65, 66, 67, 68, 69, 70, 71, 72, 73, 74, 75, 76, 77, 78, 79, 80, 81, 82 and 109, and the second polypeptide is selected from the polypeptide as shown in SEQ ID NO: 17; or
the first polypeptide is selected from the polypeptide as shown in any one of the group consisiting of SEQ ID NO: 63, 64, 65, 66, 67, 68, 69, 70, 71, 72, 73, 74, 75, 76, 77, 78, 79, 80, 81, 82 and 109, and the second polypeptide is selected from the polypeptide as shown in SEQ ID NO: 111.

**17.** A SIRPγ peptide variant, wherein the SIRPγ peptide variant is a SIRPγ peptide variant with a substitution mutation at position N51 relative to the wild-type SIRPγ peptide as shown in SEQ ID NO: 20.

**18.** The SIRPγ peptide variant according to claim 17, wherein the SIRPγ peptide variant further comprises amino acid substitution(s) at one or more positions selected from the group consisiting of K19, K53, N101, L31, Q52, E54, H56, N70, M72 and M112 relative to the wild-type SIRPγ peptide.

**19.** The SIRPγ peptide variant according to claim 17 or 18, wherein the SIRPγ peptide variant with a substitution mutation at position N51 does not substantially bind with CD47 on surface of red blood cells, preferably, the SIRPγ peptide variant with a substitution mutation at position N51 comprises N51F, N51I, N51L, N51M or N51V substitution mutation.

**20.** The SIRPγ peptide variant according to claim 17 or 18, wherein the SIRPγ peptide variant comprises N51R substitution mutation relative to the wild-type SIRPγ peptide as shown in SEQ ID NO: 20.

**21.** The SIRPγ peptide variant according to any one of claims 17 to 20, wherein the SIRPγ peptide variant comprises K19E, K53G and N101D substitution mutations relative to the wild-type SIRPγ peptide as shown in SEQ ID NO: 20.

**22.** The SIRPγ peptide variant according to any one of claims 17 to 21, wherein the SIRPγ peptide variant further comprises amino acid substitution(s) at one or more positions selected from the group consisiting of M6, V27, L30, V33, V36, L37, V42, E47, L66, T67, V92 and S98.

**23.** The SIRPγ peptide variant according to claim 21, wherein the SIRPγ peptide variant is as shown in SEQ ID NO: 1.

**24.** The SIRPγ peptide variant according to claim 21, wherein the SIRPγ peptide variant is as shown in SEQ ID NO: 2.

**25.** The SIRPγ peptide variant according to claim 21, wherein the SIRPγ peptide variant is as shown in the group consisiting of SEQ ID NO: 21, 22, 23, 24, 25, 26, 27, 28, 29, 30, 31, 32, 33, 34, 35, 36, 37, 38, 39 and 40.

**26.** A fusion protein comprising a SIRPγ peptide variant and an antibody Fc fragment, the SIRPγ peptide variant being the SIRPγ peptide variant according to any one of claims 17 to 25, preferably, the antibody Fc fragment is a human antibody Fc fragment, more preferably, the antibody Fc fragment sequence is the same as the Fc fragment sequence in the heavy chain constant region as shown in SEQ ID NO: 10 or 11, most preferably, the amino acid sequence of the fusion protein is as shown in the group consisiting of SEQ ID NO: 86, 110, 114, 115, 116, 117, 118, 119, 120, 121, 122, 123, 124, 125, 126, 127, 128, 129, 130 and 131.

**27.** An anti-human PD-L1 antibody comprising a light chain variable region and a heavy chain variable region, the heavy chain variable region comprises HCDR1, HCDR2 and HCDR3 regions as shown in SEQ ID NO: 103, 104 and 105, respectively, and the light chain variable region comprises LCDR1, LCDR2 and LCDR3 regions as shown in SEQ ID NO: 106, 112 and 108, respectively.

**28.** The anti-human PD-L1 antibody according to claim 27, wherein the heavy chain variable region is shown in SEQ ID NO: 8, and the light chain variable region is shown in SEQ ID NO: 113.

**29.** The anti-human PD-L1 antibody according to claim 28, wherein the anti-human PD-L1 antibody is a full-length antibody, further comprising an antibody constant region, preferably, the heavy chain constant region of the antibody constant region is selected from the constant regions of human IgG1, IgG2, IgG3 and IgG4, the light chain constant region of the antibody constant region is selected from the constant regions of human antibody κ and λ chains, more preferably, the full-length antibody comprises the heavy chain constant region as shown in SEQ ID NO: 10 or 11 and the light chain constant region as shown in SEQ ID NO: 12.

**30.** The anti-human PD-L1 antibody according to claim 28, wherein the antibody comprises a heavy chain as shown in SEQ ID NO: 16 or 18, and a light chain as shown in SEQ ID NO: 111.

**31.** A pharmaceutical composition comprising a therapeutically effective amount of the bifunctional fusion protein according to any one of claims 1 to 16, or the SIRPγ peptide variant according to any one of claims 17 to 25, or the fusion protein according to claim 26, or the anti-human PD-L1 antibody according to any one of claims 27 to 30, and one or more pharmaceutically acceptable carriers, diluents, buffers or excipients.

**32.** An isolated nucleic acid molecule encoding the bifunctional fusion protein according to any one of claims 1 to 16, or the SIRPγ peptide variant according to any one of claims 17 to 25, or the fusion protein according to claim 26, or the anti-human PD-L1 antibody according to any one of claims 27 to 30.

**33.** A recombinant vector comprising the isolated nucleic acid molecule according to claim 32.

**34.** A method for eliminating immunosuppression-related diseases in a subject, which comprises administering to the subject a therapeutically effective amount of the bifunctional fusion protein of any one according to any one of claims 1 to 16, or the SIRPγ peptide variant according to any one of claims 17 to 25, or the fusion protein according to claim 26, or the anti-human PD-L1 antibody according to any one of claims 27 to 30, or the pharmaceutical composition according to claim 31, or the isolated nucleic acid molecule according to claim 32, preferably, the therapeutically effective amount is a unit dose of the composition comprising 0.1 to 3000 mg of the bifunctional fusion protein according to any one of claims 1 to 16, or the SIRPγ peptide variant according to any one of claims 17 to 25, or the fusion protein according to claim 26, or the anti-human PD-L1 antibody according to any one of claims 27 to 30.

**35.** The method for eliminating immunosuppression-related diseases in a subject according to claim 34, wherein the immunosuppression-related diseases include cancer, bacterial or viral infection, preferably, the cancer includes carcinoma, lymphoma, blastoma, sarcoma and leukemia or lymphoid malignancy, more preferably include squamous cell carcinoma, myeloma, small cell lung cancer, non-small cell lung cancer, head and neck squamous cell carcinoma, glioma, Hodgkin's lymphoma, non-Hodgkin's lymphoma, diffuse large B-cell lymphoma, follicular lymphoma, acute lymphoblastic leukemia, acute myelocytic leukemia, chronic lymphocytic leukemia, chronic myelocytic leukemia, primary mediastinal large B-cell lymphoma, mantle cell lymphoma, small lymphocytic lymphoma, T-cell/histiocyte-rich large B-cell lymphoma, multiple myeloma, myeloid cell leukemia-1 protein, myelodysplastic syndrome, gastrointestinal cancer, ovarian cancer, liver cancer, lymphoblastic leukemia, lymphocytic leukemia, colorectal cancer, endometrial cancer, prostate cancer, thyroid cancer, melanoma, chondrosarcoma, neuroblastoma, pancreatic cancer, glioblastoma multiforme, bone cancer, Ewing's sarcoma, cervical cancer, brain cancer, bladder cancer, breast cancer, colon cancer, hepatocellular carcinoma, clear cell renal cell carcinoma, head and neck cancer, pharyngolaryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myelodysplastic tumor, neuroendocrine tumor, Merkel cell carcinoma, testicular cancer and skin cancer.

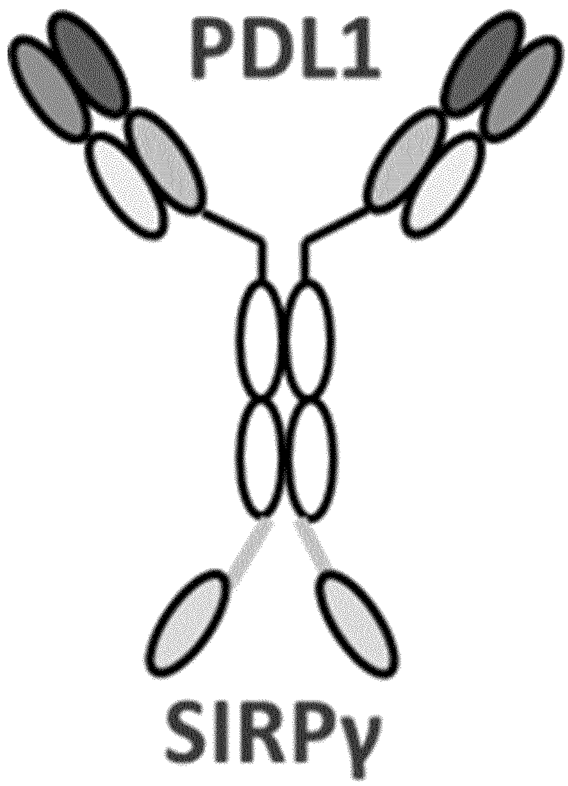


Figure 1

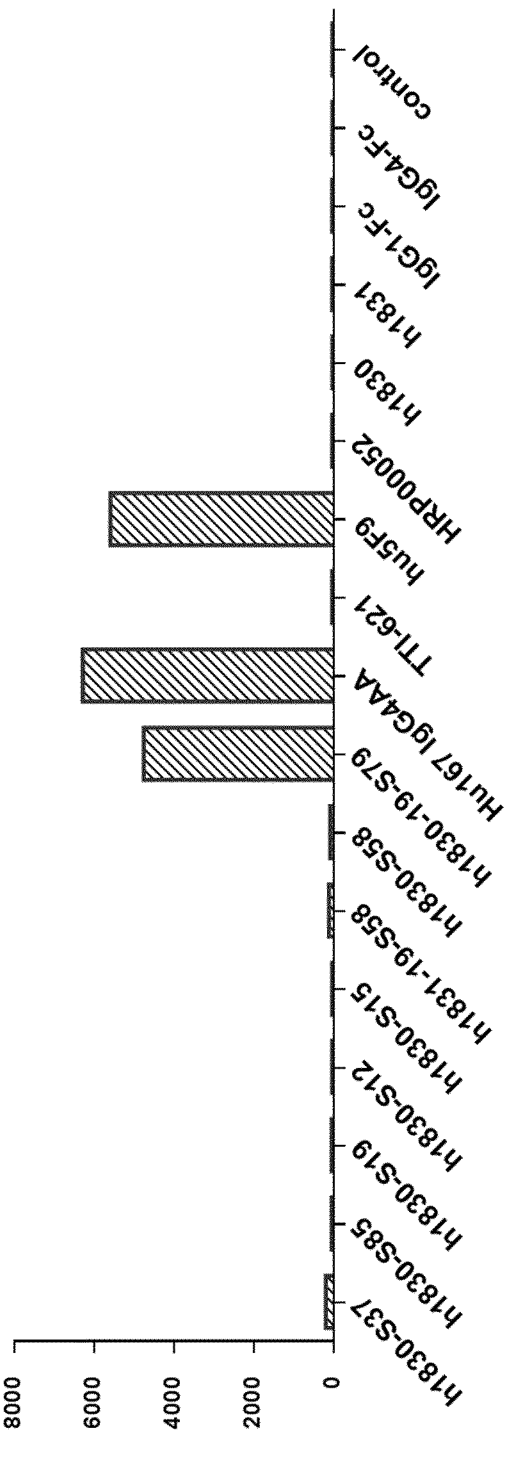


Figure 2A

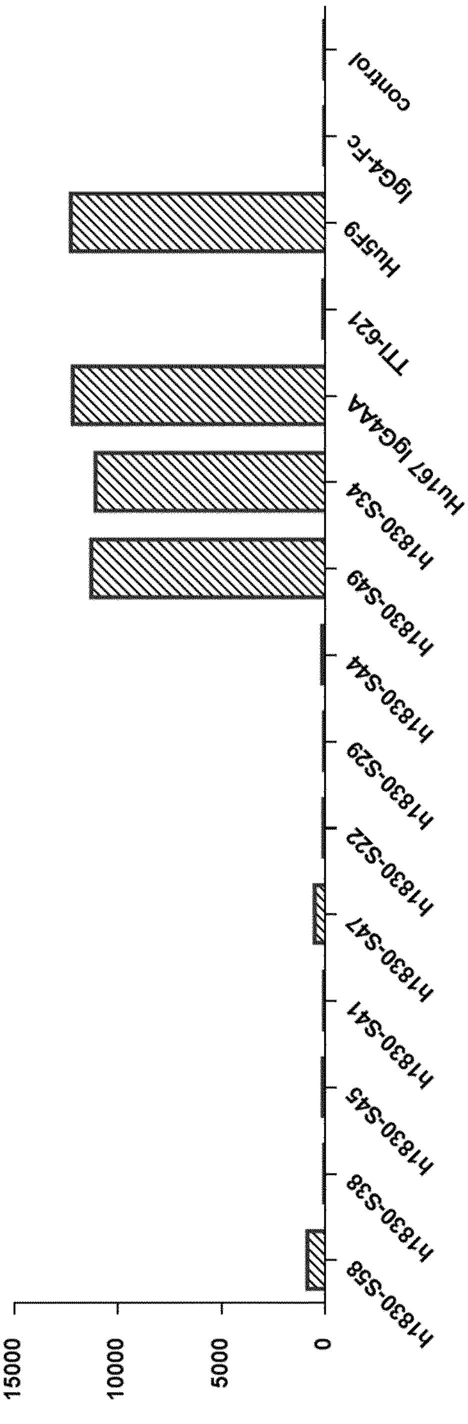


Figure 2B

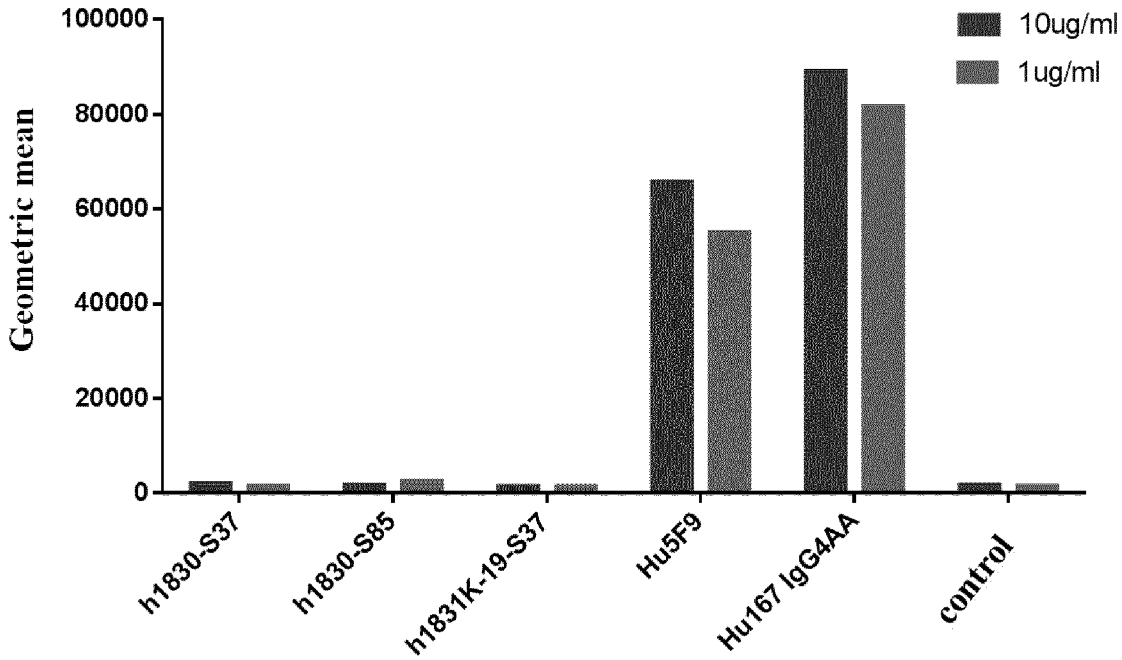


Figure 2C

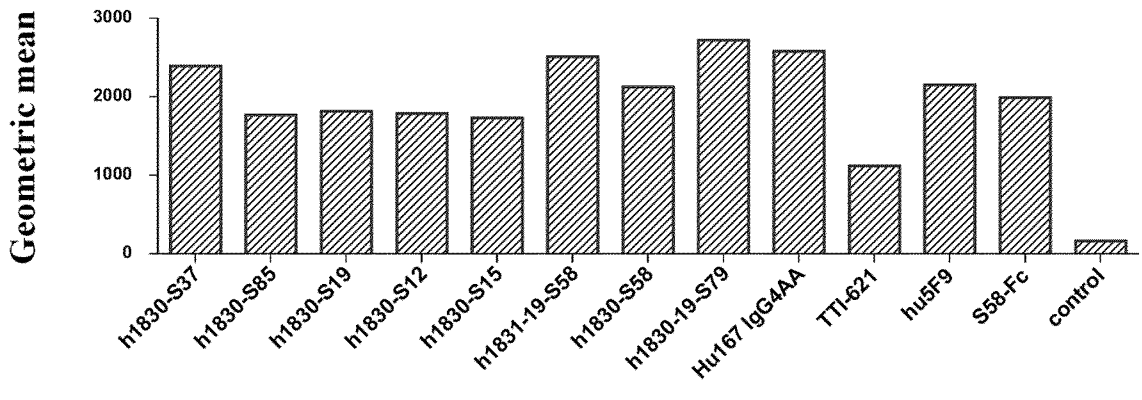


Figure 3

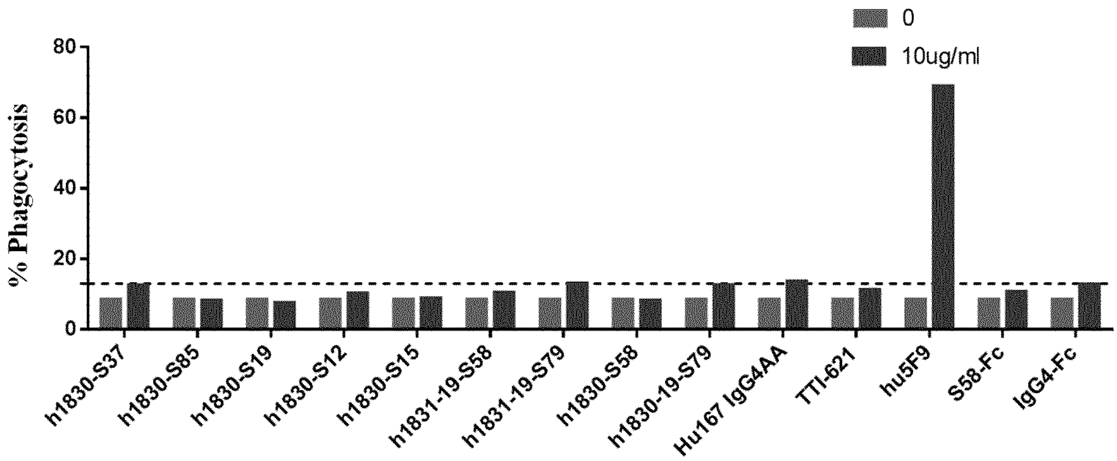


**Figure 4**

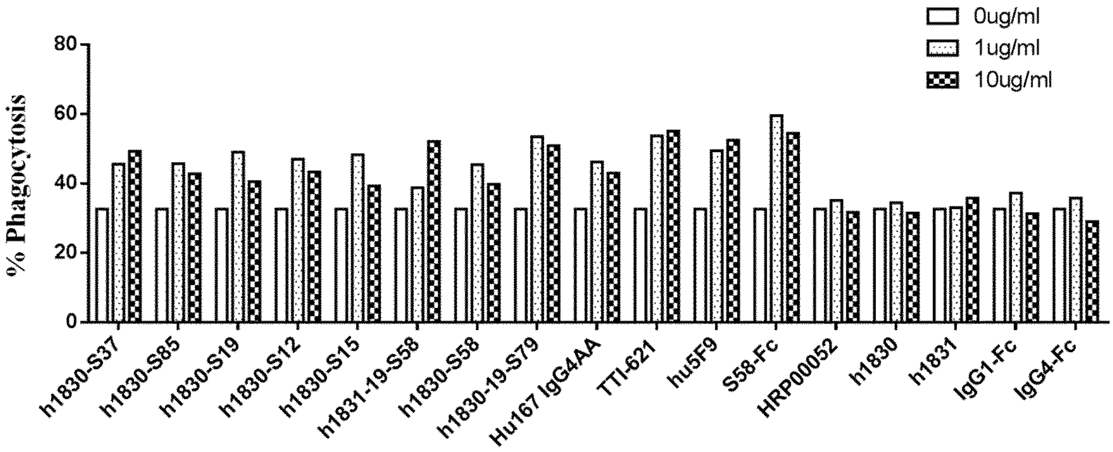


**Figure 5A**

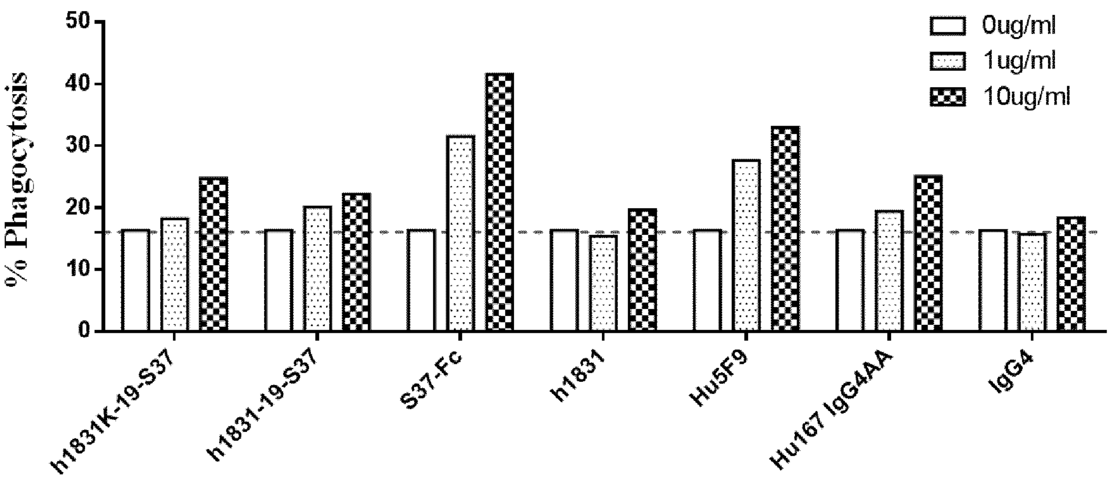


**Figure 5B**

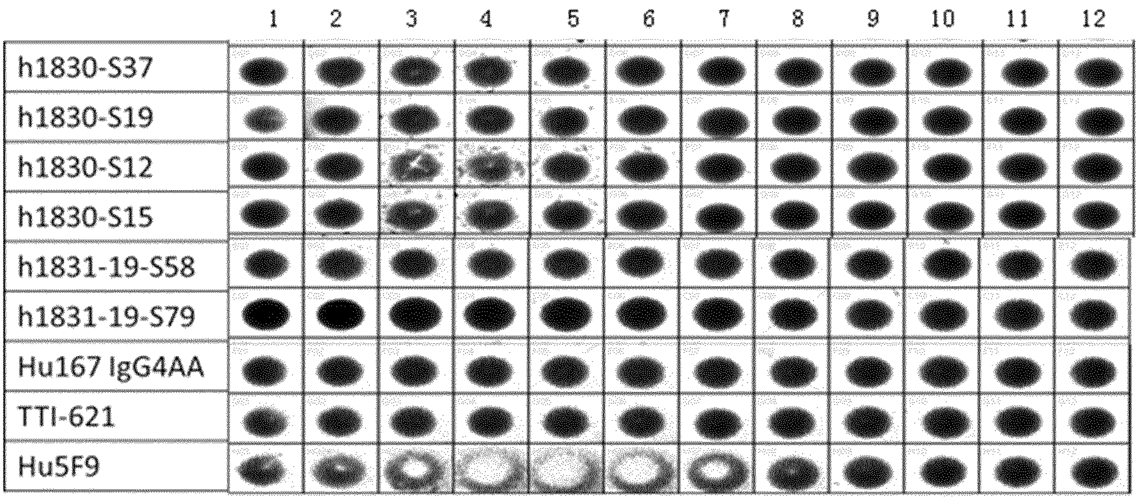


Figure 6

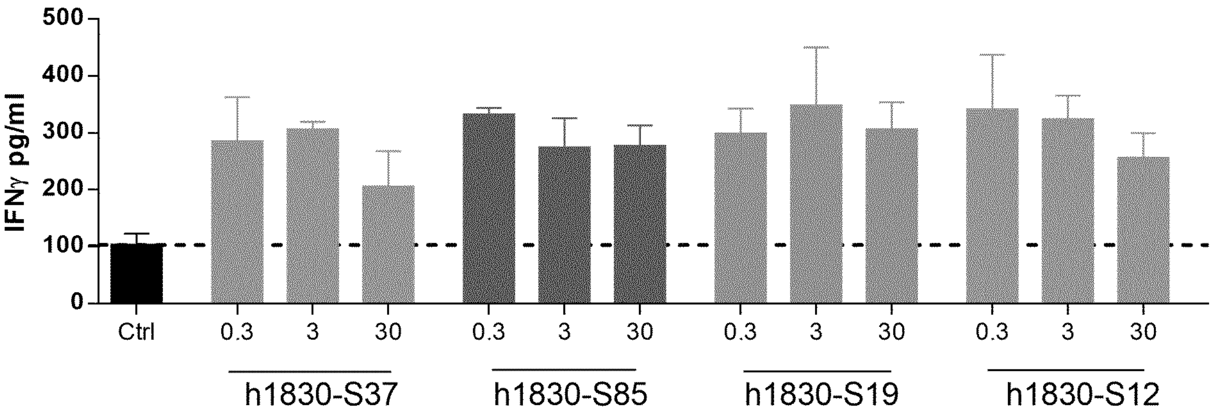


Figure 7A

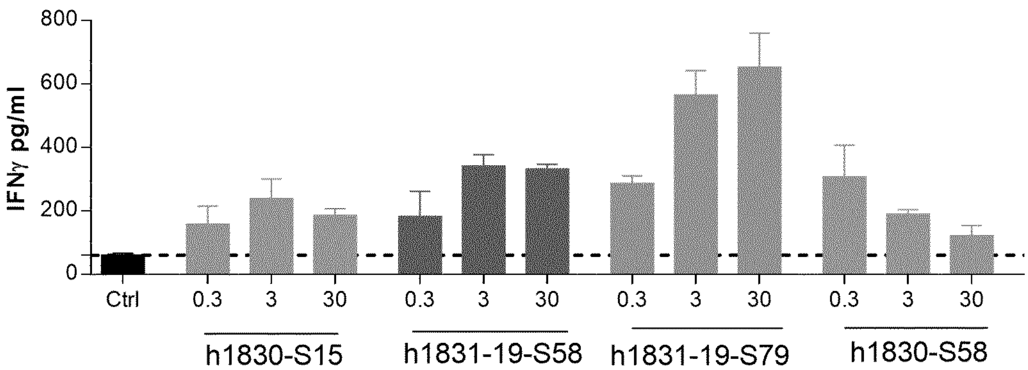


Figure 7B

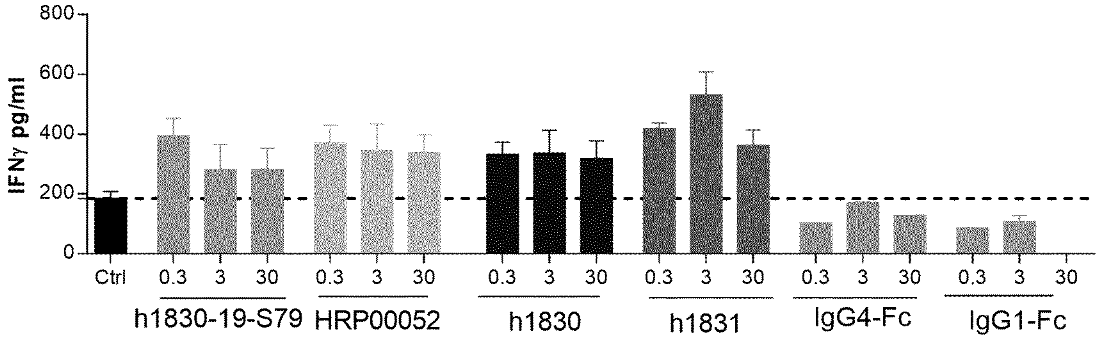


Figure 7C

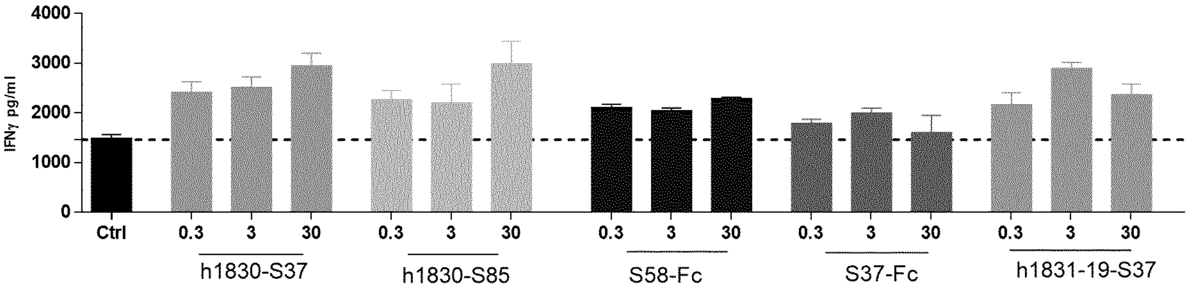


Figure 7D

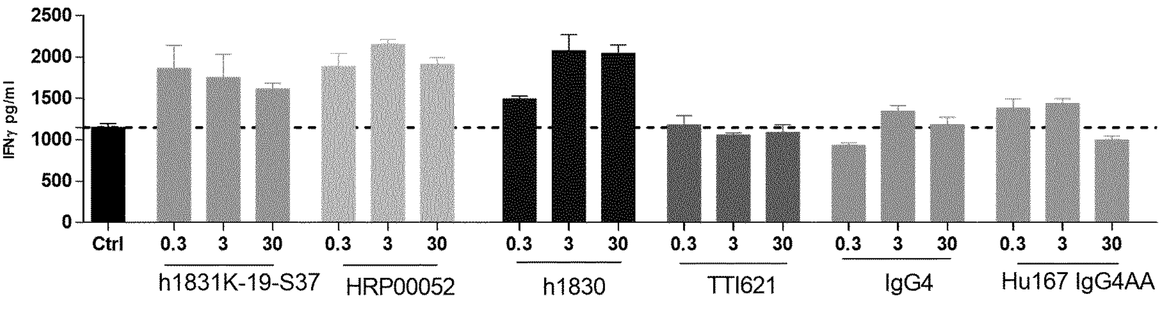


Figure 7E

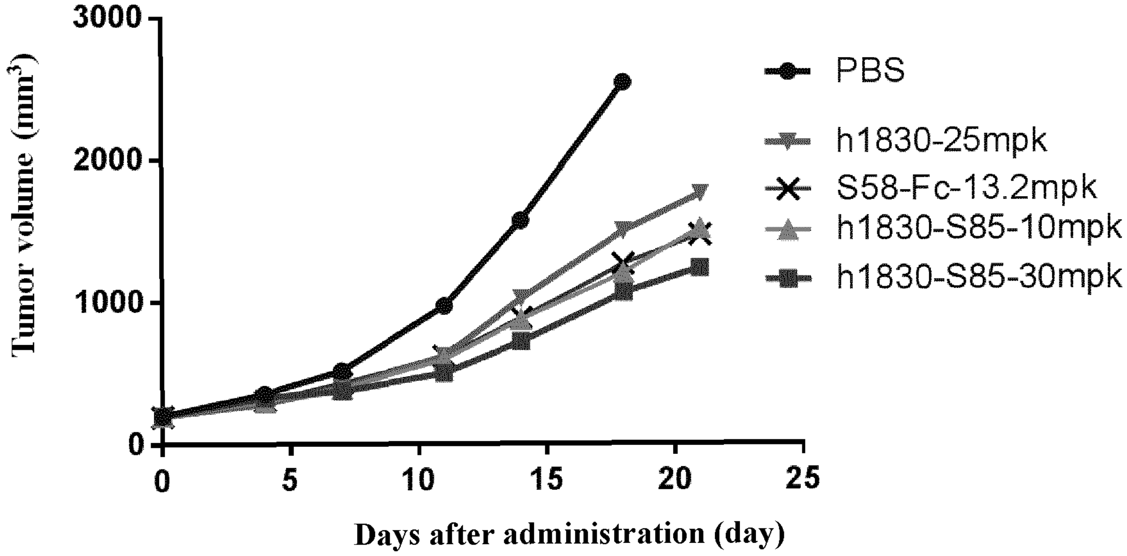


Figure 8

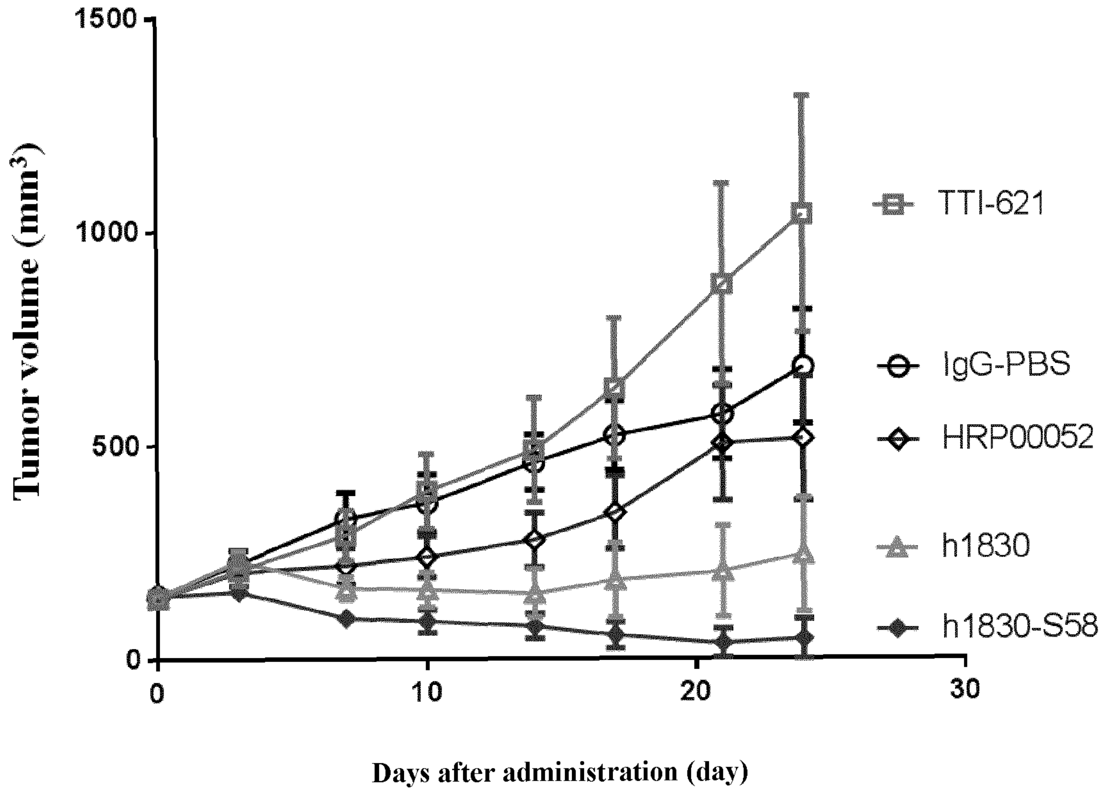


Figure 9

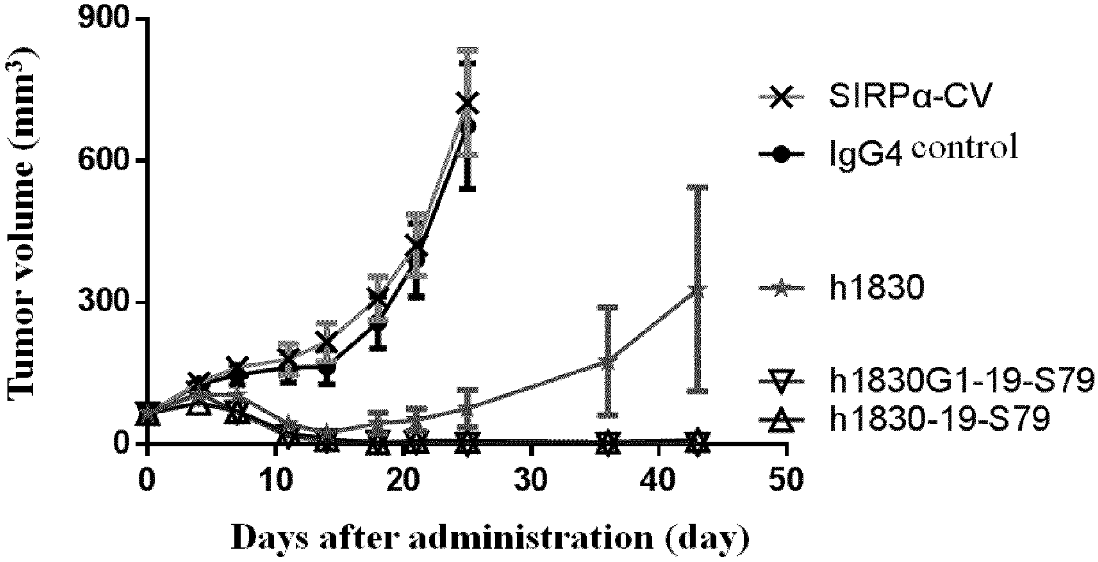


Figure 10

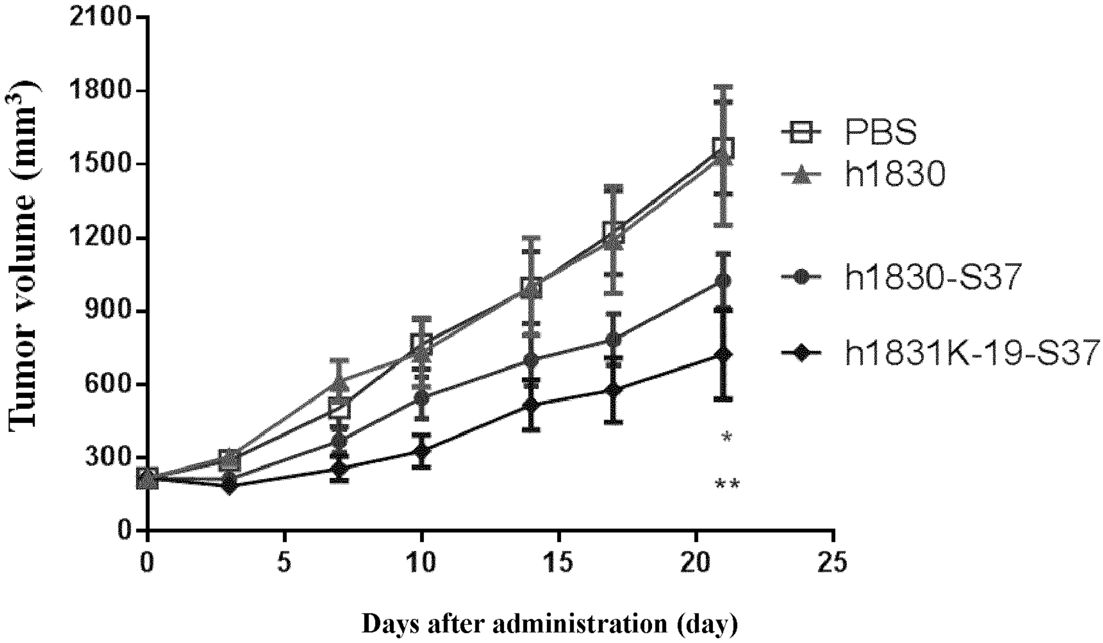


Figure 11

## INTERNATIONAL SEARCH REPORT

International application No.
**PCT/CN2020/077907**

### A. CLASSIFICATION OF SUBJECT MATTER

C07K 19/00(2006.01)i； C12N 15/62(2006.01)i； A61K 39/395(2006.01)i； A61P 35/00(2006.01)i

According to International Patent Classification (IPC) or to both national classification and IPC

### B. FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNMED; EPTXT; USTXT; WOTXT; CNABS; CNTXT; PUBMED; GenBank; STN: PD-L1, antibod+, SIRP, SIRPγ, SIRP-γ, mutat+, N51,fusion, variant, bifunctional, CD47, cancer, 抗体, 融合蛋白, 双功能, 突变, 变体, 癌症, 肿瘤, SEQ ID NO: 20-27, 103, 104, 105, 106, 112, 108

### C. DOCUMENTS CONSIDERED TO BE RELEVANT

| Category* | Citation of document, with indication, where appropriate, of the relevant passages | Relevant to claim No. |
|---|---|---|
| X | CN 107849143 A (AB INITIO BIOTHERAPEUTICS, INC.) 27 March 2018 (2018-03-27) description, paragraphs [0003], [0010], [0044]-[0049], [0053], [0056]-[0058] and [0105], embodiment 1, and claims 77, 81 and 82 | 1-11, 17-26, 31-35 |
| Y | CN 107849143 A (AB INITIO BIOTHERAPEUTICS, INC.) 27 March 2018 (2018-03-27) description, paragraphs [0003], [0010], [0044]-[0049], [0053], [0056]-[0058] and [0105], embodiment 1, and claims 77, 81 and 82 | 12-16 |
| X | WO 2017084495 A1 (JIANGSU HENGRUI MEDICINE CO., LTD. et al.) 26 May 2017 (2017-05-26) description, page 5, paragraph 1 and page 6, lines 25-29, embodiments 3, 4 and 7, and claims 20-25 | 27-35 |
| Y | WO 2017084495 A1 (JIANGSU HENGRUI MEDICINE CO., LTD. et al.) 26 May 2017 (2017-05-26) description, page 5, paragraph 1 and page 6, lines 25-29, embodiments 3, 4 and 7, and claims 20-25 | 12-16 |
| A | WO 2017019767 A1 (MYOSOTIS, LLC) 02 February 2017 (2017-02-02) entire document | 1-35 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 May 2020** | **28 May 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/077907**

**C. DOCUMENTS CONSIDERED TO BE RELEVANT**

| Category* | Citation of document, with indication, where appropriate, of the relevant passages | Relevant to claim No. |
|---|---|---|
| A | WO 2018012952 A1 (KOREA INSTITUTE OF SCIENCE AND TECHNOLOGY) 18 January 2018 (2018-01-18)<br>entire document | 1-35 |
| A | CN 107459578 A (TAIZHOU MAIBO TAIKE PHARMACEUTICAL CO., LTD.) 12 December 2017 (2017-12-12)<br>entire document | 1-35 |
| A | CN 106146670 A (IMMUNEONCO BIOPHARMACEUTICALS (SHANGHAI) CO., LTD.) 23 November 2016 (2016-11-23)<br>entire document | 1-35 |
| A | CN 108864290 A (SHANGHAI JMT BIO INC.) 23 November 2018 (2018-11-23)<br>entire document | 1-35 |
| A | NETTLESHIP, J. E. et al. "Crystal Structure of Signal Regulatory Protein Gamma (SIRPγ) in Complex with an Antibody Fab Fragment"<br>*BMC STRUCTURAL BIOLOGY,* Vol. 13, No. 13, 31 December 2013 (2013-12-31),<br>pp. 1-8 | 1-35 |

Form PCT/ISA/210 (second sheet) (January 2015)

**INTERNATIONAL SEARCH REPORT**

International application No.
**PCT/CN2020/077907**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/077907**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **34-35**
because they relate to subject matter not required to be searched by this Authority, namely:

[1] Claims 34 and 35 relate to a method for eliminating subjects' immunosuppression of related diseases, which are method for the treatment of diseases by therapy according to PCT rule 39.1 (iv). However, a search is still made base on its pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/077907**

| Patent document cited in search report | Publication date (day/month/year) | Patent family member(s) | Publication date (day/month/year) |
|---|---|---|---|
| CN 107849143 A | 27 March 2018 | US 2016340397 A1 | 24 November 2016 |
| | | US 9845345 B2 | 19 December 2017 |
| | | EP 3298043 A1 | 28 March 2018 |
| | | CA 2994935 A1 | 24 November 2016 |
| | | EP 3298043 A4 | 19 December 2018 |
| | | US 2018155405 A1 | 07 June 2018 |
| | | WO 2016187226 A1 | 24 November 2016 |
| | | JP 2018524282 A | 30 August 2018 |
| | | HK 1252870 A1 | 06 June 2019 |
| WO 2017084495 A1 | 26 May 2017 | EP 3378871 A4 | 07 August 2019 |
| | | CA 3004804 A1 | 26 May 2017 |
| | | RU 2018119165 A | 19 December 2019 |
| | | MX 2018005720 A | 09 November 2018 |
| | | AU 2016357901 A1 | 24 May 2018 |
| | | US 2018334504 A1 | 22 November 2018 |
| | | BR 112018009064 A8 | 26 February 2019 |
| | | CN 107614529 B | 12 November 2019 |
| | | TW 201718657 A | 01 June 2017 |
| | | CN 107614529 A | 19 January 2018 |
| | | CN 109608544 A | 12 April 2019 |
| | | EP 3378871 A1 | 26 September 2018 |
| | | BR 112018009064 A2 | 30 October 2018 |
| | | JP 2018536401 A | 13 December 2018 |
| | | RU 2018119165 A3 | 14 February 2020 |
| | | KR 20180071376 A | 27 June 2018 |
| WO 2017019767 A1 | 02 February 2017 | None | |
| WO 2018012952 A1 | 18 January 2018 | KR 20180008353 A | 24 January 2018 |
| | | EP 3501509 A1 | 26 June 2019 |
| | | CA 3040440 A1 | 18 January 2018 |
| | | US 2019216947 A1 | 18 July 2019 |
| | | CN 109803642 A | 24 May 2019 |
| | | KR 101953617 B1 | 23 May 2019 |
| CN 107459578 A | 12 December 2017 | None | |
| CN 106146670 A | 23 November 2016 | JP 2018512850 A | 24 May 2018 |
| | | EP 3287470 A1 | 28 February 2018 |
| | | EP 3287470 A4 | 31 October 2018 |
| | | US 2018141986 A1 | 24 May 2018 |
| | | WO 2016169261 A1 | 27 October 2016 |
| | | CN 106146670 B | 15 January 2019 |
| CN 108864290 A | 23 November 2018 | EP 3623388 A1 | 18 March 2020 |
| | | AU 2018264321 A1 | 12 December 2019 |
| | | WO 2018205936 A1 | 15 November 2018 |
| | | KR 20200003845 A | 10 January 2020 |
| | | TW 201843181 A | 16 December 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016065329 A
- WO 2016109415 A
- WO 2014087248 A
- WO 2014093678 A
- CN 107849143 A
- CN 108350048
- CN 106535914
- WO 2016023001 A
- CN 107459578 A
- CN 2017110167989
- CN 201711016798
- US 5641870 A
- WO 2017084495 A1
- CN 2019070982 W
- US 09017675 B
- WO 2014094122 A1
- WO 2018095428 A1


### Non-patent literature cited in the description


- **PICCIO et al.** *Blood,* 2005, vol. 105, 2421-2427
- **STEFANISAKIS et al.** *Blood,* 2008, vol. 112, 1280-1289
- *J. biol. chem,* 1968, vol. 243, 3558
- **KABAT, E. A. et al.** Sequences of Proteins of Immunological Interest. 1991
- **BIRD et al.** *Science,* 1988, vol. 242, 423-426
- **HUSTON et al.** *Proc. Natl. Acad. Sci USA,* 1988, vol. 85, 5879-5883
- **ZAPATA et al.** *Protein Eng.,* 1995, vol. 8 (10), 1057-1062
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol Immunother.,* 2001, vol. 50, 51-59
- *Protein Engineering,* 1994, vol. 7, 697
- **KABAT et al.** Sequences of Proteins of Immunological Interest. Public Health Service, National Institutes of Health, 1991
- **KABAT et al.** Sequences of Proteins of Immunological Interest. National Institutes of Health, 1991
- **AL-LAZIKANI et al.** *J. Molec. Biol.,* 1997, vol. 273, 927-948
- **AL-LAZIKANI et al.** *JMB,* 1997, vol. 273, 927-948
- **LEFRANC M. P.** *Immunologist,* 1999, vol. 7, 132-136
- **LEFRANC, M. P. et al.** *Dev. Comp. Immunol.,* 2003, vol. 27, 55-77
- Epitope Mapping Protocols in Methods in Molecular Biology. 1996, vol. 66
- **WATSON et al.** Molecular Biology of the Gene. The Benjamin/Cummings Pub. Co, 1987, 224
- Immunotherapeutic Adjuvants to Anticancer Antibodies. *Science,* 05 July 2013, vol. 341 (6141), 88-91